# EUROPEAN PATENT APPLICATION

(11) **EP 2 289 544 A2**
(43) Date of publication of application: **02.03.2011**
(21) Application number: 10010864.6
(22) Date of filing: 19.11.2007
(51) Int. Cl.: A61K 39/09, C07K 14/315, G01N 33/569

(54) **Peptides protective against S. pneumoniae and compositions, methods and uses relating thereto**

(30) Priority: 20.11.2006 EP 06024006
(62) Divisional of application: 07847204.0
(71) Applicant: Intercell AG, 1030 Vienna (AT)
(72) Inventor: The designation of the inventor has not yet been filed
(74) Representative: Bösl, Raphael Konrad

(57) **Abstract**

The present invention relates to a protective peptide or a functionally active variant of the protective peptide; a composition comprising at least two proteins selected from the group consisting of i) a first type of protective peptide or functionally active variant thereof, ii) a second type of protective peptide or functionally active variant thereof and iii) a supportive peptide or a functionally active variant thereof; one or more nucleic acid(s) encoding the protective peptide or functionally active variant thereof or the at least two proteins comprised in the composition; a pharmaceutical composition comprising the protective peptide or functionally active variant thereof, the composition, or the nucleic acid(s); a method of producing an antibody using the protective peptide or functionally active variant thereof or the composition; the use of the protective peptide or functionally active variant thereof and/or the composition and/or the nucleic acid(s) for the manufacture of a medicament for the immunization or treatment of a subject; a method of diagnosing a *S. pneumoniae* infect ion using the protective peptide or a functionally active variant thereof, the composition or a primer and/or probe specific for the nucleic acid(s); a method for identifying a ligand capable of binding to a protective peptide or variant thereof; and the use of a protective peptide or variant thereof for the isolation and/or purification and/or identification of an interaction partner of the peptide.

SEQUENCE LISTING

<110>  Intercell AG

<120>  Peptides protective against S. pneumoniae and compositions,
       methods and uses relating thereto

<130>  I66456PCEPT1

<150>  EP06024006.6
<151>  2006-11-20

<160>  143

<170>  PatentIn version 3.4

<210>  1
<211>  251
<212>  PRT
<213>  Artificial

<220>
<223>  Construct  1: SP2216-1

<400>  1

Glu Thr Thr Asp Asp Lys Ile Ala Ala Gln Asp Asn Lys Ile Ser Asn
1               5                   10                  15

Leu Thr Ala Gln Gln Gln Glu Ala Gln Lys Gln Val Asp Gln Ile Gln
            20                  25                  30

Glu Gln Val Ser Ala Ile Gln Ala Glu Gln Ser Asn Leu Gln Ala Glu
        35                  40                  45

Asn Asp Arg Leu Gln Ala Glu Ser Lys Lys Leu Glu Gly Glu Ile Thr
    50                  55                  60

Glu Leu Ser Lys Asn Ile Val Ser Arg Asn Gln Ser Leu Glu Lys Gln
65                  70                  75                  80

Ala Arg Ser Ala Gln Thr Asn Gly Ala Val Thr Ser Tyr Ile Asn Thr
                85                  90                  95

Ile Val Asn Ser Lys Ser Ile Thr Glu Ala Ile Ser Arg Val Ala Ala
            100                 105                 110

Met Ser Glu Ile Val Ser Ala Asn Asn Lys Met Leu Glu Gln Gln Lys
        115                 120                 125

Ala Asp Lys Lys Ala Ile Ser Glu Lys Gln Val Ala Asn Asn Asp Ala
    130                 135                 140

Ile Asn Thr Val Ile Ala Asn Gln Gln Lys Leu Ala Asp Asp Ala Gln
145                 150                 155                 160

Ala Leu Thr Thr Lys Gln Ala Glu Leu Lys Ala Ala Glu Leu Ser Leu
                165                 170                 175

Ala Ala Glu Lys Ala Thr Ala Glu Gly Glu Lys Ala Ser Leu Leu Glu
            180                 185                 190

Gln Lys Ala Ala Ala Glu Ala Glu Ala Arg Ala Ala Ala Val Ala Glu
        195                 200                 205

Ala Ala Tyr Lys Glu Lys Arg Ala Ser Gln Gln Gln Ser Val Leu Ala
    210                 215                 220

Ser Ala Asn Thr Asn Leu Thr Ala Gln Val Gln Ala Val Ser Glu Ser
225                 230                 235                 240

Ala Ala Ala Pro Val Arg Ala Lys Val Arg Pro
                245                 250

<210> 2
<211> 315
<212> PRT
<213> Artificial

<220>
<223> Construct 2: SP1732-3

<400> 2

Tyr Leu Ile Leu Leu Ala Ser Leu Val Leu Val Ala Ala Ser Leu Ile
1               5                   10                  15

Trp Ile Leu Ser Arg Thr Pro Ala Thr Ile Ala Ile Pro Asp Val Ala
            20                  25                  30

Gly Gln Thr Val Ala Glu Ala Lys Ala Thr Leu Lys Lys Ala Asn Phe
        35                  40                  45

Glu Ile Gly Glu Glu Lys Thr Glu Ala Ser Glu Lys Val Glu Glu Gly
    50                  55                  60

Arg Ile Ile Arg Thr Asp Pro Gly Ala Gly Thr Gly Arg Lys Glu Gly
65                  70                  75                  80

Thr Lys Ile Asn Leu Val Val Ser Ser Gly Lys Gln Ser Phe Gln Ile
                85                  90                  95

Ser Asn Tyr Val Gly Arg Lys Ser Ser Asp Val Ile Ala Glu Leu Lys
            100                 105                 110

Glu Lys Lys Val Pro Asp Asn Leu Ile Lys Ile Glu Glu Glu Glu Ser
        115                 120                 125

Asn Glu Ser Glu Ala Gly Thr Val Leu Lys Gln Ser Leu Pro Glu Gly
    130                 135                 140

Thr Thr Tyr Asp Leu Ser Lys Ala Thr Gln Ile Val Leu Thr Val Ala
145                 150                 155                 160

Lys Lys Ala Thr Thr Ile Gln Leu Gly Asn Tyr Ile Gly Arg Asn Ser
                165                 170                 175

Thr Glu Val Ile Ser Glu Leu Lys Gln Lys Lys Val Pro Glu Asn Leu
            180                 185                 190

Ile Lys Ile Glu Glu Glu Glu Ser Ser Glu Ser Glu Pro Gly Thr Ile
        195                 200                 205

Met Lys Gln Ser Pro Gly Ala Gly Thr Thr Tyr Asp Val Ser Lys Pro
    210                 215                 220

Thr Gln Ile Val Leu Thr Val Ala Lys Lys Val Thr Ser Val Ala Met
225                 230                 235                 240

Pro Ser Tyr Ile Gly Ser Ser Leu Glu Phe Thr Lys Asn Asn Leu Ile
                245                 250                 255

Gln Ile Val Gly Ile Lys Glu Ala Asn Ile Glu Val Val Glu Val Thr
            260                 265                 270

Thr Ala Pro Ala Gly Ser Val Glu Gly Met Val Val Glu Gln Ser Pro
        275                 280                 285

Arg Ala Gly Glu Lys Val Asp Leu Asn Lys Thr Arg Val Lys Ile Ser
    290                 295                 300

Ile Tyr Lys Pro Lys Thr Thr Ser Ala Thr Pro
305                 310                 315

<210>  3
<211>  289
<212>  PRT
<213>  Artificial

<220>
<223>  Construct  3: SP1650 (PsaA)

<400>  3

Ala Ser Gly Lys Lys Asp Thr Thr Ser Gly Gln Lys Leu Lys Val Val
1               5                   10                  15

Ala Thr Asn Ser Ile Ile Ala Asp Ile Thr Lys Asn Ile Ala Gly Asp
            20                  25                  30

Lys Ile Asp Leu His Ser Ile Val Pro Ile Gly Gln Asp Pro His Glu
        35                  40                  45

Tyr Glu Pro Leu Pro Glu Asp Val Lys Lys Thr Ser Glu Ala Asp Leu
    50                  55                  60

Ile Phe Tyr Asn Gly Ile Asn Leu Glu Thr Gly Gly Asn Ala Trp Phe
65                  70                  75                  80

Thr Lys Leu Val Glu Asn Ala Lys Lys Thr Glu Asn Lys Asp Tyr Phe
                85                  90                  95

Ala Val Ser Asp Gly Val Asp Val Ile Tyr Leu Glu Gly Gln Asn Glu
            100                 105                 110

Lys Gly Lys Glu Asp Pro His Ala Trp Leu Asn Leu Glu Asn Gly Ile
        115                 120                 125

Ile Phe Ala Lys Asn Ile Ala Lys Gln Leu Ser Ala Lys Asp Pro Asn
    130                 135                 140

Asn Lys Glu Phe Tyr Glu Lys Asn Leu Lys Glu Tyr Thr Asp Lys Leu
145                 150                 155                 160

Asp Lys Leu Asp Lys Glu Ser Lys Asp Lys Phe Asn Lys Ile Pro Ala
                165                 170                 175

Glu Lys Lys Leu Ile Val Thr Ser Glu Gly Ala Phe Lys Tyr Phe Ser
            180                 185                 190

Lys Ala Tyr Gly Val Pro Ser Ala Tyr Ile Trp Glu Ile Asn Thr Glu
        195                 200                 205

Glu Glu Gly Thr Pro Glu Gln Ile Lys Thr Leu Val Glu Lys Leu Arg
    210                 215                 220

Gln Thr Lys Val Pro Ser Leu Phe Val Glu Ser Ser Val Asp Asp Arg
225                 230                 235                 240

Pro Met Lys Thr Val Ser Gln Asp Thr Asn Ile Pro Ile Tyr Ala Gln
                245                 250                 255

Ile Phe Thr Asp Ser Ile Ala Glu Gln Gly Lys Glu Gly Asp Ser Tyr
            260                 265                 270

Tyr Ser Met Met Lys Tyr Asn Leu Asp Lys Ile Ala Glu Gly Leu Ala
        275                 280                 285

Lys


<210>  4
<211>  572
<212>  PRT
<213>  Artificial

<220>

<223>  Construct  4 & 5: SP2216-1 + SP1732-3

<400>  4

Glu Thr Thr Asp Asp Lys Ile Ala Ala Gln Asp Asn Lys Ile Ser Asn
1               5                   10                  15

Leu Thr Ala Gln Gln Gln Glu Ala Gln Lys Gln Val Asp Gln Ile Gln
            20                  25                  30

Glu Gln Val Ser Ala Ile Gln Ala Glu Gln Ser Asn Leu Gln Ala Glu
        35                  40                  45

Asn Asp Arg Leu Gln Ala Glu Ser Lys Lys Leu Glu Gly Glu Ile Thr
    50                  55                  60

Glu Leu Ser Lys Asn Ile Val Ser Arg Asn Gln Ser Leu Glu Lys Gln
65                  70                  75                  80

Ala Arg Ser Ala Gln Thr Asn Gly Ala Val Thr Ser Tyr Ile Asn Thr
                85                  90                  95

Ile Val Asn Ser Lys Ser Ile Thr Glu Ala Ile Ser Arg Val Ala Ala
            100                 105                 110

Met Ser Glu Ile Val Ser Ala Asn Asn Lys Met Leu Glu Gln Gln Lys
        115                 120                 125

Ala Asp Lys Lys Ala Ile Ser Glu Lys Gln Val Ala Asn Asn Asp Ala
    130                 135                 140

Ile Asn Thr Val Ile Ala Asn Gln Gln Lys Leu Ala Asp Asp Ala Gln
145                 150                 155                 160

Ala Leu Thr Thr Lys Gln Ala Glu Leu Lys Ala Ala Glu Leu Ser Leu
                165                 170                 175

Ala Ala Glu Lys Ala Thr Ala Glu Gly Glu Lys Ala Ser Leu Leu Glu
            180                 185                 190

Gln Lys Ala Ala Ala Glu Ala Glu Ala Arg Ala Ala Ala Val Ala Glu
        195                 200                 205

Ala Ala Tyr Lys Glu Lys Arg Ala Ser Gln Gln Gln Ser Val Leu Ala
    210                 215                 220

Ser Ala Asn Thr Asn Leu Thr Ala Gln Val Gln Ala Val Ser Glu Ser
225                 230                 235                 240

Ala Ala Ala Pro Val Arg Ala Lys Val Arg Pro Gly Pro Val Asp Pro
                245                 250                 255

Gly Tyr Leu Ile Leu Leu Ala Ser Leu Val Leu Val Ala Ala Ser Leu
            260                 265                 270

Ile Trp Ile Leu Ser Arg Thr Pro Ala Thr Ile Ala Ile Pro Asp Val
        275                 280                 285

Ala Gly Gln Thr Val Ala Glu Ala Lys Ala Thr Leu Lys Lys Ala Asn
    290                 295                 300

Phe Glu Ile Gly Glu Glu Lys Thr Glu Ala Ser Glu Lys Val Glu Glu
305                 310                 315                 320

Gly Arg Ile Ile Arg Thr Asp Pro Gly Ala Gly Thr Gly Arg Lys Glu
                325                 330                 335

Gly Thr Lys Ile Asn Leu Val Val Ser Ser Gly Lys Gln Ser Phe Gln
            340                 345                 350

Ile Ser Asn Tyr Val Gly Arg Lys Ser Ser Asp Val Ile Ala Glu Leu
        355                 360                 365

Lys Glu Lys Lys Val Pro Asp Asn Leu Ile Lys Ile Glu Glu Glu Glu
    370                 375                 380

Ser Asn Glu Ser Glu Ala Gly Thr Val Leu Lys Gln Ser Leu Pro Glu
385                 390                 395                 400

Gly Thr Thr Tyr Asp Leu Ser Lys Ala Thr Gln Ile Val Leu Thr Val
                405                 410                 415

Ala Lys Lys Ala Thr Thr Ile Gln Leu Gly Asn Tyr Ile Gly Arg Asn
            420                 425                 430

Ser Thr Glu Val Ile Ser Glu Leu Lys Gln Lys Lys Val Pro Glu Asn
        435                 440                 445

Leu Ile Lys Ile Glu Glu Glu Glu Ser Ser Glu Ser Glu Pro Gly Thr
    450                 455                 460

Ile Met Lys Gln Ser Pro Gly Ala Gly Thr Thr Tyr Asp Val Ser Lys
465                 470                 475                 480

Pro Thr Gln Ile Val Leu Thr Val Ala Lys Lys Val Thr Ser Val Ala
                485                 490                 495

Met Pro Ser Tyr Ile Gly Ser Ser Leu Glu Phe Thr Lys Asn Asn Leu
            500                 505                 510

Ile Gln Ile Val Gly Ile Lys Glu Ala Asn Ile Glu Val Val Glu Val
        515                 520                 525

Thr Thr Ala Pro Ala Gly Ser Val Glu Gly Met Val Val Glu Gln Ser
    530                 535                 540

Pro Arg Ala Gly Glu Lys Val Asp Leu Asn Lys Thr Arg Val Lys Ile
545                 550                 555                 560

Ser Ile Tyr Lys Pro Lys Thr Thr Ser Ala Thr Pro
                565                 570

<210>  5
<211>  572
<212>  PRT
<213>  Artificial

<220>
<223>  Construct  6 & 7: SP1732-3 + SP2216-1

<400>  5

Tyr Leu Ile Leu Leu Ala Ser Leu Val Leu Val Ala Ala Ser Leu Ile
1               5                   10                  15

Trp Ile Leu Ser Arg Thr Pro Ala Thr Ile Ala Ile Pro Asp Val Ala
            20                  25                  30

Gly Gln Thr Val Ala Glu Ala Lys Ala Thr Leu Lys Lys Ala Asn Phe
        35                  40                  45

Glu Ile Gly Glu Glu Lys Thr Glu Ala Ser Glu Lys Val Glu Glu Gly
    50                  55                  60

Arg Ile Ile Arg Thr Asp Pro Gly Ala Gly Thr Gly Arg Lys Glu Gly
65                  70                  75                  80

Thr Lys Ile Asn Leu Val Val Ser Ser Gly Lys Gln Ser Phe Gln Ile
                85                  90                  95

Ser Asn Tyr Val Gly Arg Lys Ser Ser Asp Val Ile Ala Glu Leu Lys
            100                 105                 110

Glu Lys Lys Val Pro Asp Asn Leu Ile Lys Ile Glu Glu Glu Glu Ser
        115                 120                 125

Asn Glu Ser Glu Ala Gly Thr Val Leu Lys Gln Ser Leu Pro Glu Gly
    130                 135                 140

Thr Thr Tyr Asp Leu Ser Lys Ala Thr Gln Ile Val Leu Thr Val Ala
145                 150                 155                 160

Lys Lys Ala Thr Thr Ile Gln Leu Gly Asn Tyr Ile Gly Arg Asn Ser
                165                 170                 175

Thr Glu Val Ile Ser Glu Leu Lys Gln Lys Lys Val Pro Glu Asn Leu
            180                 185                 190

Ile Lys Ile Glu Glu Glu Glu Ser Ser Glu Ser Glu Pro Gly Thr Ile
        195                 200                 205

Met Lys Gln Ser Pro Gly Ala Gly Thr Thr Tyr Asp Val Ser Lys Pro
    210                 215                 220

Thr Gln Ile Val Leu Thr Val Ala Lys Lys Val Thr Ser Val Ala Met
225                 230                 235                 240

Pro Ser Tyr Ile Gly Ser Ser Leu Glu Phe Thr Lys Asn Asn Leu Ile
                245                 250                 255

Gln Ile Val Gly Ile Lys Glu Ala Asn Ile Glu Val Val Glu Val Thr
            260                 265                 270

Thr Ala Pro Ala Gly Ser Val Glu Gly Met Val Val Glu Gln Ser Pro
        275                 280                 285

Arg Ala Gly Glu Lys Val Asp Leu Asn Lys Thr Arg Val Lys Ile Ser
    290                 295                 300

Ile Tyr Lys Pro Lys Thr Thr Ser Ala Thr Pro Gly Pro Val Asp Pro
305                 310                 315                 320

Gly Glu Thr Thr Asp Asp Lys Ile Ala Ala Gln Asp Asn Lys Ile Ser
                325                 330                 335

Asn Leu Thr Ala Gln Gln Gln Glu Ala Gln Lys Gln Val Asp Gln Ile
            340                 345                 350

Gln Glu Gln Val Ser Ala Ile Gln Ala Glu Gln Ser Asn Leu Gln Ala
        355                 360                 365

Glu Asn Asp Arg Leu Gln Ala Glu Ser Lys Lys Leu Glu Gly Glu Ile
    370                 375                 380

Thr Glu Leu Ser Lys Asn Ile Val Ser Arg Asn Gln Ser Leu Glu Lys
385                 390                 395                 400

Gln Ala Arg Ser Ala Gln Thr Asn Gly Ala Val Thr Ser Tyr Ile Asn
                405                 410                 415

Thr Ile Val Asn Ser Lys Ser Ile Thr Glu Ala Ile Ser Arg Val Ala
            420                 425                 430

Ala Met Ser Glu Ile Val Ser Ala Asn Asn Lys Met Leu Glu Gln Gln
        435                 440                 445

Lys Ala Asp Lys Lys Ala Ile Ser Glu Lys Gln Val Ala Asn Asn Asp
    450                 455                 460

Ala Ile Asn Thr Val Ile Ala Asn Gln Gln Lys Leu Ala Asp Asp Ala
465                 470                 475                 480

Gln Ala Leu Thr Thr Lys Gln Ala Glu Leu Lys Ala Ala Glu Leu Ser
                485                 490                 495

Leu Ala Ala Glu Lys Ala Thr Ala Glu Gly Glu Lys Ala Ser Leu Leu
            500                 505                 510

Glu Gln Lys Ala Ala Ala Glu Ala Glu Ala Arg Ala Ala Ala Val Ala
        515                 520                 525

Glu Ala Ala Tyr Lys Glu Lys Arg Ala Ser Gln Gln Gln Ser Val Leu
    530                 535                 540

Ala Ser Ala Asn Thr Asn Leu Thr Ala Gln Val Gln Ala Val Ser Glu
545                 550                 555                 560

Ser Ala Ala Ala Pro Val Arg Ala Lys Val Arg Pro
                565                 570

<210>   6
<211>   336
<212>   PRT
<213>   Artificial

<220>
<223>   Construct  8 & 9: SP2216-1 + SP1732-PASTA

<400>   6

Glu Thr Thr Asp Asp Lys Ile Ala Ala Gln Asp Asn Lys Ile Ser Asn
1               5                   10                  15

Leu Thr Ala Gln Gln Gln Glu Ala Gln Lys Gln Val Asp Gln Ile Gln
            20                  25                  30

Glu Gln Val Ser Ala Ile Gln Ala Glu Gln Ser Asn Leu Gln Ala Glu
        35                  40                  45

Asn Asp Arg Leu Gln Ala Glu Ser Lys Lys Leu Glu Gly Glu Ile Thr
    50                  55                  60

Glu Leu Ser Lys Asn Ile Val Ser Arg Asn Gln Ser Leu Glu Lys Gln
65                  70                  75                  80

Ala Arg Ser Ala Gln Thr Asn Gly Ala Val Thr Ser Tyr Ile Asn Thr
                85                  90                  95

Ile Val Asn Ser Lys Ser Ile Thr Glu Ala Ile Ser Arg Val Ala Ala
            100                 105                 110

Met Ser Glu Ile Val Ser Ala Asn Asn Lys Met Leu Glu Gln Gln Lys
        115                 120                 125

Ala Asp Lys Lys Ala Ile Ser Glu Lys Gln Val Ala Asn Asn Asp Ala
    130                 135                 140

Ile Asn Thr Val Ile Ala Asn Gln Gln Lys Leu Ala Asp Asp Ala Gln
145                 150                 155                 160

Ala Leu Thr Thr Lys Gln Ala Glu Leu Lys Ala Ala Glu Leu Ser Leu
                165                 170                 175

Ala Ala Glu Lys Ala Thr Ala Glu Gly Glu Lys Ala Ser Leu Leu Glu
            180                 185                 190

Gln Lys Ala Ala Ala Glu Ala Glu Ala Arg Ala Ala Ala Val Ala Glu
        195                 200                 205

Ala Ala Tyr Lys Glu Lys Arg Ala Ser Gln Gln Gln Ser Val Leu Ala
    210                 215                 220

Ser Ala Asn Thr Asn Leu Thr Ala Gln Val Gln Ala Val Ser Glu Ser
225                 230                 235                 240

Ala Ala Ala Pro Val Arg Ala Lys Val Arg Pro Gly Gly Pro Gly Val
                245                 250                 255

Thr Ser Val Ala Met Pro Ser Tyr Ile Gly Ser Ser Leu Glu Phe Thr
            260                 265                 270

Lys Asn Asn Leu Ile Gln Ile Val Gly Ile Lys Glu Ala Asn Ile Glu
        275                 280                 285

Val Val Glu Val Thr Thr Ala Pro Ala Gly Ser Val Glu Gly Met Val
    290                 295                 300

Val Glu Gln Ser Pro Arg Ala Gly Glu Lys Val Asp Leu Asn Lys Thr
305                 310                 315                 320

Arg Val Lys Ile Ser Ile Tyr Lys Pro Lys Thr Thr Ser Ala Thr Pro
                325                 330                 335

<210>  7
<211>  336
<212>  PRT
<213>  Artificial

<220>
<223>  Construct  10 & 11: SP1732- PASTA + SP2216-1

<400>  7

Val Thr Ser Val Ala Met Pro Ser Tyr Ile Gly Ser Ser Leu Glu Phe
1               5                   10                  15

Thr Lys Asn Asn Leu Ile Gln Ile Val Gly Ile Lys Glu Ala Asn Ile
            20                  25                  30

Glu Val Val Glu Val Thr Thr Ala Pro Ala Gly Ser Val Glu Gly Met
        35                  40                  45

Val Val Glu Gln Ser Pro Arg Ala Gly Glu Lys Val Asp Leu Asn Lys
    50                  55                  60

Thr Arg Val Lys Ile Ser Ile Tyr Lys Pro Lys Thr Thr Ser Ala Thr
65                  70                  75                  80

Pro Gly Gly Pro Gly Glu Thr Thr Asp Asp Lys Ile Ala Ala Gln Asp
                85                  90                  95

Asn Lys Ile Ser Asn Leu Thr Ala Gln Gln Gln Glu Ala Gln Lys Gln
            100                 105                 110

Val Asp Gln Ile Gln Glu Gln Val Ser Ala Ile Gln Ala Glu Gln Ser
        115                 120                 125

Asn Leu Gln Ala Glu Asn Asp Arg Leu Gln Ala Glu Ser Lys Lys Leu
    130                 135                 140

Glu Gly Glu Ile Thr Glu Leu Ser Lys Asn Ile Val Ser Arg Asn Gln
145                 150                 155                 160

Ser Leu Glu Lys Gln Ala Arg Ser Ala Gln Thr Asn Gly Ala Val Thr
                165                 170                 175

Ser Tyr Ile Asn Thr Ile Val Asn Ser Lys Ser Ile Thr Glu Ala Ile
            180                 185                 190

Ser Arg Val Ala Ala Met Ser Glu Ile Val Ser Ala Asn Asn Lys Met
        195                 200                 205

Leu Glu Gln Gln Lys Ala Asp Lys Lys Ala Ile Ser Glu Lys Gln Val
    210                 215                 220

Ala Asn Asn Asp Ala Ile Asn Thr Val Ile Ala Asn Gln Gln Lys Leu
225                 230                 235                 240

Ala Asp Asp Ala Gln Ala Leu Thr Thr Lys Gln Ala Glu Leu Lys Ala
                245                 250                 255

Ala Glu Leu Ser Leu Ala Ala Glu Lys Ala Thr Ala Glu Gly Glu Lys
            260                 265                 270

Ala Ser Leu Leu Glu Gln Lys Ala Ala Ala Glu Ala Glu Ala Arg Ala
        275                 280                 285

Ala Ala Val Ala Glu Ala Ala Tyr Lys Glu Lys Arg Ala Ser Gln Gln
    290                 295                 300

Gln Ser Val Leu Ala Ser Ala Asn Thr Asn Leu Thr Ala Gln Val Gln
305                 310                 315                 320

Ala Val Ser Glu Ser Ala Ala Ala Pro Val Arg Ala Lys Val Arg Pro
                325                 330                 335

<210>  8
<211>  753
<212>  DNA
<213>  Artificial

<220>
<223>  Construct  1: SP2216-1

<400>  8
gaaacgactg atgacaaaat tgctgctcaa gataataaaa ttagtaactt aacagcacaa      60

caacaagaag cccaaaaaca agttgaccaa attcaggagc aagtatcagc tattcaagct     120

gagcagtcta acttgcaagc tgaaaatgat agattacaag cagaatctaa gaaactcgag     180

ggtgagatta cagaactttc taaaaacatt gtttctcgta accaatcgtt ggaaaaacaa     240

gctcgtagtg ctcaaacaaa tggagccgta actagctata tcaataccat tgtaaactca     300

aaatcaatta cagaagctat ttcacgtgtt gctgcaatga gtgaaatcgt atctgcaaac     360

aacaaaatgt tagaacaaca aaaggcagat aaaaaagcta tttctgaaaa acaagtagca     420

aataatgatg ctatcaatac tgtaattgct aatcaacaaa aattggctga tgatgctcaa     480

gcattgacta cgaaacaggc agaactaaaa gctgctgaat taagtcttgc tgctgagaaa     540

gcgacagctg aaggggaaaa agcaagtcta ttagagcaaa aagcagcagc tgaggcagag     600

gctcgtgcag ctgcggtagc agaagcagct tataaagaaa aacgagctag ccaacaacaa     660

tcagtacttg cttcagcaaa cactaactta acagctcaag tgcaagcagt atctgaatct     720

gcagcagcac ctgtccgtgc aaaagttcgt cca                                  753

<210>  9
<211>  945
<212>  DNA
<213>  Artificial

<220>
<223>  Construct  2: SP1732-3

<400>  9
tacctgattt tgttggccag ccttgtattg gtggcagctt ctcttatttg gatactatcc      60

agaactcctg caaccattgc cattccagat gtggcaggtc agacagttgc agaggccaag     120

gcaacgctca aaaaagccaa ttttgagatt ggtgaggaga agacagaggc tagtgaaaag     180

gtggaagaag ggcggattat ccgtacagat cctggcgctg gaactggtcg aaaagaagga     240

acgaaaatca atttggttgt ctcatcaggc aaacaatcct tccaaattag taattatgtc     300

ggccggaaat cttctgatgt tatcgcggaa ttaaaagaga aaaaagttcc agataatttg 360
attaaaattg aggaagaaga gtcgaatgag agtgaggctg gaacggtcct gaagcaaagt 420
ctaccagaag gtacgaccta tgacttgagc aaggcaactc aaattgtttt gacagtagct 480
aaaaaagcta cgacgattca attagggaac tatattggac ggaactctac agaagtaatc 540
tcagaactca agcagaagaa ggttcctgag aatttgatta agatagagga agaagagtcc 600
agcgaaagcg aaccaggaac gattatgaaa caaagtccag gtgccggaac gacttatgat 660
gtgagtaaac ctactcaaat tgtcttgaca gtagctaaaa aagttacaag tgttgccatg 720
ccgagttaca ttggttccag cttggagttt actaagaaca atttgattca aattgttggg 780
attaaggaag ctaatataga agttgtagaa gtgacgacag cgcctgcagg tagtgtagaa 840
ggcatggttg ttgaacaaag tcctagagca ggtgaaaagg tagacctaaa taagactaga 900
gtcaagattt caatctacaa acctaaaaca acttcagcta ctcct 945

<210> 10
<211> 867
<212> DNA
<213> Artificial

<220>
<223> Construct 3: SP1650 (PsaA)

<400> 10
gctagcggaa aaaaagatac aacttctggt caaaaactaa aagttgttgc tacaaactca 60
atcatcgctg atattactaa aaatattgct ggtgacaaaa ttgaccttca tagtatcgtt 120
ccgattgggc aagacccaca cgaatacgaa ccacttcctg aagacgttaa gaaaacttct 180
gaggctgatt tgatttctta taacggtatc aaccttgaaa caggtggcaa tgcttggttt 240
acaaaattgg tagaaaatgc caagaaaact gaaaacaaag actacttcgc agtcagcgac 300
ggcgttgatg ttatctacct tgaaggtcaa aatgaaaaag gaaaagaaga cccacacgct 360
tggcttaacc ttgaaaacgg tattattttt gctaaaaata tcgccaaaca attgagcgcc 420
aaagacccta acaataaaga attctatgaa aaaaatctca aagaatatac tgataagtta 480
gacaaacttg ataaagaaag taaggataaa tttaataaga tccctgctga aaagaaactc 540
attgtaacca gcgaaggagc attcaaatac ttctctaaag cctatggtgt cccaagtgcc 600
tacatctggg aaatcaatac tgaagaagaa ggaactcctg aacaaatcaa gaccttggtt 660
gaaaaacttc gccaaacaaa agttccatca ctctttgtag aatcaagtgt ggatgaccgt 720
ccaatgaaaa ctgtttctca agacacaaac atcccaatct acgcacaaat ctttactgac 780
tctatcgcag aacaaggtaa agaaggcgac agctactaca gcatgatgaa atacaacctt 840
gacaagattg ctgaaggatt ggcaaaa 867

<210> 11
<211> 1716
<212> DNA
<213> Artificial

<220>
<223>  Construct  4 & 5: SP2216-1 + SP1732-3

<400>  11
gaaacgactg atgacaaaat tgctgctcaa gataataaaa ttagtaactt aacagcacaa      60

caacaagaag cccaaaaaca agttgaccaa attcaggagc aagtatcagc tattcaagct     120

gagcagtcta acttgcaagc tgaaaatgat agattacaag cagaatctaa gaaactcgag     180

ggtgagatta cagaactttc taaaaacatt gtttctcgta accaatcgtt ggaaaaacaa     240

gctcgtagtg ctcaaacaaa tggagccgta actagctata tcaataccat tgtaaactca     300

aaatcaatta cagaagctat ttcacgtgtt gctgcaatga gtgaaatcgt atctgcaaac     360

aacaaaatgt tagaacaaca aaaggcagat aaaaaagcta tttctgaaaa acaagtagca     420

aataatgatg ctatcaatac tgtaattgct aatcaacaaa aattggctga tgatgctcaa     480

gcattgacta cgaaacaggc agaactaaaa gctgctgaat taagtcttgc tgctgagaaa     540

gcgacagctg aaggggaaaa agcaagtcta ttagagcaaa aagcagcagc tgaggcagag     600

gctcgtgcag ctgcggtagc agaagcagct tataaagaaa aacgagctag ccaacaacaa     660

tcagtacttg cttcagcaaa cactaactta acagctcaag tgcaagcagt atctgaatct     720

gcagcagcac ctgtccgtgc aaaagttcgt ccaggaccag tcgaccctgg ttacctgatt     780

ttgttggcca gccttgtatt ggtggcagct tctcttattt ggatactatc cagaactcct     840

gcaaccattg ccattccaga tgtggcaggt cagacagttg cagaggccaa ggcaacgctc     900

aaaaaagcca attttgagat tggtgaggag aagacagagg ctagtgaaaa ggtggaagaa     960

gggcggatta tccgtacaga tcctggcgct ggaactggtc gaaaagaagg aacgaaaatc    1020

aatttggttg tctcatcagg caaacaatcc ttccaaatta gtaattatgt cggccggaaa    1080

tcttctgatg ttatcgcgga attaaaagag aaaaaagttc cagataattt gattaaaatt    1140

gaggaagaag agtcgaatga gagtgaggct ggaacggtcc tgaagcaaag tctaccagaa    1200

ggtacgacct atgacttgag caaggcaact caaattgttt tgacagtagc taaaaaagct    1260

acgacgattc aattagggaa ctatattgga cggaactcta cagaagtaat ctcagaactc    1320

aagcagaaga aggttcctga gaatttgatt aagatagagg aagaagagtc cagcgaaagc    1380

gaaccaggaa cgattatgaa acaaagtcca ggtgccggaa cgacttatga tgtgagtaaa    1440

cctactcaaa ttgtcttgac agtagctaaa aaagttacaa gtgttgccat gccgagttac    1500

attggttcca gcttggagtt tactaagaac aatttgattc aaattgttgg gattaaggaa    1560

gctaatatag aagttgtaga agtgacgaca gcgcctgcag gtagtgtaga aggcatggtt    1620

gttgaacaaa gtcctagagc aggtgaaaag gtagacctaa ataagactag agtcaagatt    1680

tcaatctaca aacctaaaac aacttcagct actcct                              1716

<210>  12
<211>  1716
<212>  DNA
<213>  Artificial

<220>
<223> Construct 6 & 7: SP1732-3 + SP2216-1

<400> 12
tacctgattt tgttggccag ccttgtattg gtggcagctt ctcttatttg gatactatcc 60

agaactcctg caaccattgc cattccagat gtggcaggtc agacagttgc agaggccaag 120

gcaacgctca aaaaagccaa ttttgagatt ggtgaggaga agacagaggc tagtgaaaag 180

gtggaagaag ggcggattat ccgtacagat cctggcgctg gaactggtcg aaaagaagga 240

acgaaaatca atttggttgt ctcatcaggc aaacaatcct tccaaattag taattatgtc 300

ggccggaaat cttctgatgt tatcgcggaa ttaaaagaga aaaaagttcc agataatttg 360

attaaaattg aggaagaaga gtcgaatgag agtgaggctg gaacggtcct gaagcaaagt 420

ctaccagaag gtacgaccta tgacttgagc aaggcaactc aaattgtttt gacagtagct 480

aaaaaagcta cgacgattca attagggaac tatattggac ggaactctac agaagtaatc 540

tcagaactca agcagaagaa ggttcctgag aatttgatta agatagagga agaagagtcc 600

agcgaaagcg aaccaggaac gattatgaaa caaagtccag gtgccggaac gacttatgat 660

gtgagtaaac ctactcaaat tgtcttgaca gtagctaaaa aagttacaag tgttgccatg 720

ccgagttaca ttggttccag cttggagttt actaagaaca atttgattca aattgttggg 780

attaaggaag ctaatataga agttgtagaa gtgacgacag cgcctgcagg tagtgtagaa 840

ggcatggttg ttgaacaaag tcctagagca ggtgaaaagg tagacctaaa taagactaga 900

gtcaagattt caatctacaa acctaaaaca acttcagcta ctcctggacc agtcgaccct 960

ggtgaaacga ctgatgacaa aattgctgct caagataata aaattagtaa cttaacagca 1020

caacaacaag aagcccaaaa acaagttgac caaattcagg agcaagtatc agctattcaa 1080

gctgagcagt ctaacttgca agctgaaaat gatagattac aagcagaatc taagaaactc 1140

gagggtgaga ttacagaact ttctaaaaac attgtttctc gtaaccaatc gttggaaaaa 1200

caagctcgta gtgctcaaac aaatggagcc gtaactagct atatcaatac cattgtaaac 1260

tcaaaatcaa ttacagaagc tatttcacgt gttgctgcaa tgagtgaaat cgtatctgca 1320

aacaacaaaa tgttagaaca acaaaaggca gataaaaaag ctatttctga aaaacaagta 1380

gcaaataatg atgctatcaa tactgtaatt gctaatcaac aaaaattggc tgatgatgct 1440

caagcattga ctacgaaaca ggcagaacta aaagctgctg aattaagtct tgctgctgag 1500

aaagcgacag ctgaagggga aaaagcaagt ctattagagc aaaaagcagc agctgaggca 1560

gaggctcgtg cagctgcggt agcagaagca gcttataaag aaaaacgagc tagccaacaa 1620

caatcagtac ttgcttcagc aaacactaac ttaacagctc aagtgcaagc agtatctgaa 1680

tctgcagcag cacctgtccg tgcaaaagtt cgtcca 1716

<210> 13
<211> 1008
<212> DNA
<213> Artificial

<220>
<223>  Construct  8 & 9: SP2216-1 + SP1732-PASTA

<400>  13
gaaacgactg atgacaaaat tgctgctcaa gataataaaa ttagtaactt aacagcacaa      60

caacaagaag cccaaaaaca agttgaccaa attcaggagc aagtatcagc tattcaagct     120

gagcagtcta acttgcaagc tgaaaatgat agattacaag cagaatctaa gaaactcgag     180

ggtgagatta cagaactttc taaaaacatt gtttctcgta accaatcgtt ggaaaaacaa     240

gctcgtagtg ctcaaacaaa tggagccgta actagctata tcaataccat tgtaaactca     300

aaatcaatta cagaagctat ttcacgtgtt gctgcaatga gtgaaatcgt atctgcaaac     360

aacaaaatgt tagaacaaca aaaggcagat aaaaaagcta tttctgaaaa acaagtagca     420

aataatgatg ctatcaatac tgtaattgct aatcaacaaa aattggctga tgatgctcaa     480

gcattgacta cgaaacaggc agaactaaaa gctgctgaat taagtcttgc tgctgagaaa     540

gcgacagctg aaggggaaaa agcaagtcta ttagagcaaa aagcagcagc tgaggcagag     600

gctcgtgcag ctgcggtagc agaagcagct tataaagaaa aacgagctag ccaacaacaa     660

tcagtacttg cttcagcaaa cactaactta acagctcaag tgcaagcagt atctgaatct     720

gcagcagcac ctgtccgtgc aaaagttcgt ccaggcggac ccggggttac aagtgttgcc     780

atgccgagtt acattggttc cagcttggag tttactaaga acaatttgat tcaaattgtt     840

gggattaagg aagctaatat agaagttgta gaagtgacga cagcgcctgc aggtagtgta     900

gaaggcatgg ttgttgaaca aagtcctaga gcaggtgaaa aggtagacct aaataagact     960

agagtcaaga tttcaatcta caaacctaaa acaacttcag ctactcct                 1008

<210>  14
<211>  1008
<212>  DNA
<213>  Artificial

<220>
<223>  Construct  10 & 11: SP1732- PASTA + SP2216-1

<400>  14
gttacaagtg ttgccatgcc gagttacatt ggttccagct tggagtttac taagaacaat      60

ttgattcaaa ttgttgggat taaggaagct aatatagaag ttgtagaagt gacgacagcg     120

cctgcaggta gtgtagaagg catggttgtt gaacaaagtc ctagagcagg tgaaaaggta     180

gacctaaata agactagagt caagatttca atctacaaac ctaaaacaac ttcagctact     240

cctggcggac ccggggaaac gactgatgac aaaattgctg ctcaagataa taaaattagt     300

aacttaacag cacaacaaca agaagcccaa aaacaagttg accaaattca ggagcaagta     360

tcagctattc aagctgagca gtctaacttg caagctgaaa atgatagatt acaagcagaa     420

tctaagaaac tcgagggtga gattacagaa ctttctaaaa acattgtttc tcgtaaccaa     480

tcgttggaaa aacaagctcg tagtgctcaa acaaatggag ccgtaactag ctatatcaat     540

accattgtaa actcaaaatc aattacagaa gctatttcac gtgttgctgc aatgagtgaa     600

atcgtatctg caaacaacaa aatgttagaa caacaaaagg cagataaaaa agctatttct 660

gaaaaacaag tagcaaataa tgatgctatc aatactgtaa ttgctaatca acaaaaattg 720

gctgatgatg ctcaagcatt gactacgaaa caggcagaac taaaagctgc tgaattaagt 780

cttgctgctg agaaagcgac agctgaaggg gaaaaagcaa gtctattaga gcaaaaagca 840

gcagctgagg cagaggctcg tgcagctgcg gtagcagaag cagcttataa agaaaaacga 900

gctagccaac aacaatcagt acttgcttca gcaaacacta acttaacagc tcaagtgcaa 960

gcagtatctg aatctgcagc agcacctgtc cgtgcaaaag ttcgtcca 1008

<210> 15
<211> 251
<212> PRT
<213> artificial

<220>
<223> Consensus SP2216-1

<400> 15

Glu Thr Thr Asp Asp Lys Ile Ala Ala Gln Asp Asn Lys Ile Ser Asn
1               5                   10                  15

Leu Thr Ala Gln Gln Gln Glu Ala Gln Lys Gln Val Asp Gln Ile Gln
            20                  25                  30

Glu Gln Val Ser Ala Ile Gln Ala Glu Gln Ser Asn Leu Gln Ala Glu
        35                  40                  45

Asn Asp Arg Leu Gln Ala Glu Ser Lys Lys Leu Glu Gly Glu Ile Thr
    50                  55                  60

Glu Leu Ser Lys Asn Ile Val Ser Arg Asn Gln Ser Leu Glu Lys Gln
65                  70                  75                  80

Ala Arg Ser Ala Gln Thr Asn Gly Ala Val Thr Ser Tyr Ile Asn Thr
                85                  90                  95

Ile Val Asn Ser Lys Ser Ile Thr Glu Ala Ile Ser Arg Val Ala Ala
            100                 105                 110

Met Ser Glu Ile Val Ser Ala Asn Asn Lys Met Leu Glu Gln Gln Lys
        115                 120                 125

Ala Asp Lys Lys Ala Ile Ser Glu Lys Gln Val Ala Asn Asn Asp Ala
    130                 135                 140

Ile Asn Thr Val Ile Ala Asn Gln Gln Lys Leu Ala Asp Asp Ala Gln
145                 150                 155                 160

Ala Leu Thr Thr Lys Gln Ala Glu Leu Lys Ala Ala Glu Leu Ser Leu
                165                 170                 175

Ala Ala Glu Lys Ala Thr Ala Glu Gly Glu Lys Ala Ser Leu Leu Glu
            180                     185                     190

Gln Lys Ala Ala Ala Glu Ala Glu Ala Arg Ala Ala Ala Val Ala Glu
        195                     200                     205

Ala Ala Tyr Lys Glu Lys Arg Ala Ser Gln Gln Gln Ser Val Leu Ala
    210                     215                     220

Ser Ala Asn Thr Asn Leu Thr Ala Gln Val Gln Ala Val Ser Glu Ser
225                     230                     235                     240

Ala Ala Ala Pro Val Arg Ala Lys Val Arg Pro
                245                     250

<210>  16
<211>  315
<212>  PRT
<213>  Artificial

<220>
<223>  Consensus SP1732-3


<220>
<221>  MISC_FEATURE
<222>  (156)..(156)
<223>  Xaa = Val or Ile

<220>
<221>  MISC_FEATURE
<222>  (279)..(279)
<223>  Xaa = Ala or Val

<400>  16

Tyr Leu Ile Leu Leu Ala Ser Leu Val Leu Val Ala Ala Ser Leu Ile
1               5                   10                  15

Trp Ile Leu Ser Arg Thr Pro Ala Thr Ile Ala Ile Pro Asp Val Ala
            20                  25                  30

Gly Gln Thr Val Ala Glu Ala Lys Ala Thr Leu Lys Lys Ala Asn Phe
        35                  40                  45

Glu Ile Gly Glu Glu Lys Thr Glu Ala Ser Glu Lys Val Glu Glu Gly
    50                  55                  60

Arg Ile Ile Arg Thr Asp Pro Gly Ala Gly Thr Gly Arg Lys Glu Gly
65                  70                  75                  80

Thr Lys Ile Asn Leu Val Val Ser Ser Gly Lys Gln Ser Phe Gln Ile
                85                  90                  95

Ser Asn Tyr Val Gly Arg Lys Ser Ser Asp Val Ile Ala Glu Leu Lys

            100                 105                 110

Glu Lys Lys Val Pro Asp Asn Leu Ile Lys Ile Glu Glu Glu Glu Ser
        115                 120                 125

Asn Glu Ser Glu Ala Gly Thr Val Leu Lys Gln Ser Leu Pro Glu Gly
    130                 135                 140

Thr Thr Tyr Asp Leu Ser Lys Ala Thr Gln Ile Xaa Leu Thr Val Ala
145                 150                 155                 160

Lys Lys Ala Thr Thr Ile Gln Leu Gly Asn Tyr Ile Gly Arg Asn Ser
                165                 170                 175

Thr Glu Val Ile Ser Glu Leu Lys Gln Lys Lys Val Pro Glu Asn Leu
            180                 185                 190

Ile Lys Ile Glu Glu Glu Glu Ser Ser Glu Ser Glu Pro Gly Thr Ile
        195                 200                 205

Met Lys Gln Ser Pro Gly Ala Gly Thr Thr Tyr Asp Val Ser Lys Pro
    210                 215                 220

Thr Gln Ile Val Leu Thr Val Ala Lys Lys Val Thr Ser Val Ala Met
225                 230                 235                 240

Pro Ser Tyr Ile Gly Ser Ser Leu Glu Phe Thr Lys Asn Asn Leu Ile
                245                 250                 255

Gln Ile Val Gly Ile Lys Glu Ala Asn Ile Glu Val Val Glu Val Thr
            260                 265                 270

Thr Ala Pro Ala Gly Ser Xaa Glu Gly Met Val Val Glu Gln Ser Pro
        275                 280                 285

Arg Ala Gly Glu Lys Val Asp Leu Asn Lys Thr Arg Val Lys Ile Ser
    290                 295                 300

Ile Tyr Lys Pro Lys Thr Thr Ser Ala Thr Pro
305                 310                 315

<210> 17
<211> 289
<212> PRT
<213> Artificial

<220>
<223> Consensus PsaA

<220>
<221> MISC_FEATURE
<222> (7)..(7)
<223> Xaa = Thr or Ala

<220>
<221>  MISC_FEATURE
<222>  (8)..(8)
<223>  Xaa = Thr or Ala

<220>
<221>  MISC_FEATURE
<222>  (10)..(10)
<223>  Xaa = Gly or Ser

<220>
<221>  MISC_FEATURE
<222>  (63)..(63)
<223>  Xaa = Asp or Asn

<220>
<221>  MISC_FEATURE
<222>  (100)..(100)
<223>  Xaa = Asp or Glu

<220>
<221>  MISC_FEATURE
<222>  (110)..(110)
<223>  Xaa = Gln or Lys

<220>
<221>  MISC_FEATURE
<222>  (173)..(173)
<223>  Xaa = Lys or Asn

<220>
<221>  MISC_FEATURE
<222>  (265)..(265)
<223>  Xaa = Gln or Glu

<220>
<221>  MISC_FEATURE
<222>  (285)..(285)
<223>  Xaa = Glu or Gly

<400>  17

Ala Ser Gly Lys Lys Asp Xaa Xaa Ser Xaa Gln Lys Leu Lys Val Val
1               5                   10                  15

Ala Thr Asn Ser Ile Ile Ala Asp Ile Thr Lys Asn Ile Ala Gly Asp
            20                  25                  30

Lys Ile Asp Leu His Ser Ile Val Pro Ile Gly Gln Asp Pro His Glu
        35                  40                  45

Tyr Glu Pro Leu Pro Glu Asp Val Lys Lys Thr Ser Glu Ala Xaa Leu
    50                  55                  60

Ile Phe Tyr Asn Gly Ile Asn Leu Glu Thr Gly Gly Asn Ala Trp Phe
65                  70                  75                  80

Thr Lys Leu Val Glu Asn Ala Lys Lys Thr Glu Asn Lys Asp Tyr Phe
                85                  90                  95

Ala Val Ser Xaa Gly Val Asp Val Ile Tyr Leu Glu Gly Xaa Asn Glu

            100                 105                 110

Lys Gly Lys Glu Asp Pro His Ala Trp Leu Asn Leu Glu Asn Gly Ile
        115                 120                 125

Ile Phe Ala Lys Asn Ile Ala Lys Gln Leu Ser Ala Lys Asp Pro Asn
    130                 135                 140

Asn Lys Glu Phe Tyr Glu Lys Asn Leu Lys Glu Tyr Thr Asp Lys Leu
145                 150                 155                 160

Asp Lys Leu Asp Lys Glu Ser Lys Asp Lys Phe Asn Xaa Ile Pro Ala
                165                 170                 175

Glu Lys Lys Leu Ile Val Thr Ser Glu Gly Ala Phe Lys Tyr Phe Ser
            180                 185                 190

Lys Ala Tyr Gly Val Pro Ser Ala Tyr Ile Trp Glu Ile Asn Thr Glu
        195                 200                 205

Glu Glu Gly Thr Pro Glu Gln Ile Lys Thr Leu Val Glu Lys Leu Arg
    210                 215                 220

Gln Thr Lys Val Pro Ser Leu Phe Val Glu Ser Ser Val Asp Asp Arg
225                 230                 235                 240

Pro Met Lys Thr Val Ser Gln Asp Thr Asn Ile Pro Ile Tyr Ala Gln
                245                 250                 255

Ile Phe Thr Asp Ser Ile Ala Glu Xaa Gly Lys Glu Gly Asp Ser Tyr
            260                 265                 270

Tyr Ser Met Met Lys Tyr Asn Leu Asp Lys Ile Ala Xaa Gly Leu Ala
        275                 280                 285

Lys

<210> 18
<211> 392
<212> PRT
<213> Streptococcus pneumoniae CDC 3 (serotype 3)

<400> 18

Met Lys Lys Lys Ile Leu Ala Ser Leu Leu Leu Ser Thr Val Met Val
1               5                   10                  15

Ser Gln Val Ala Val Leu Thr Thr Ala His Ala Glu Thr Thr Asp Asp
            20                  25                  30

Lys Ile Ala Ala Gln Asp Asn Lys Ile Ser Asn Leu Thr Ala Gln Gln
        35                  40                  45

Gln Glu Ala Gln Lys Gln Val Asp Gln Ile Gln Glu Gln Val Ser Ala
    50                  55                  60

Ile Gln Ala Glu Gln Ser Asn Leu Gln Ala Glu Asn Asp Arg Leu Gln
65                  70                  75                  80

Ala Glu Ser Lys Lys Leu Glu Gly Glu Ile Thr Glu Leu Ser Lys Asn
                85                  90                  95

Ile Val Ser Arg Asn Gln Ser Leu Glu Lys Gln Ala Arg Ser Ala Gln
            100                 105                 110

Thr Asn Gly Ala Val Thr Ser Tyr Ile Asn Thr Ile Val Asn Ser Lys
        115                 120                 125

Ser Ile Thr Glu Ala Ile Ser Arg Val Ala Ala Met Ser Glu Ile Val
    130                 135                 140

Ser Ala Asn Asn Lys Met Leu Glu Gln Gln Lys Ala Asp Lys Lys Ala
145                 150                 155                 160

Ile Ser Glu Lys Gln Val Ala Asn Asn Asn Ala Ile Asn Thr Val Ile
                165                 170                 175

Ala Asn Gln Gln Lys Leu Ala Asp Asp Ala Gln Ala Leu Thr Thr Lys
            180                 185                 190

Gln Ala Glu Leu Lys Ala Ala Glu Leu Ser Leu Ala Ala Glu Lys Ala
        195                 200                 205

Thr Ala Glu Gly Glu Lys Ala Ser Leu Leu Glu Gln Lys Ala Ala Ala
    210                 215                 220

Glu Ala Glu Ala Arg Ala Ala Ala Val Ala Glu Ala Ala Tyr Lys Glu
225                 230                 235                 240

Lys Arg Ala Ser Gln Gln Gln Ser Val Leu Ala Ser Ala Asn Thr Asn
                245                 250                 255

Leu Thr Ala Gln Val Gln Ala Val Ser Glu Ser Ala Ala Ala Pro Val
            260                 265                 270

Arg Ala Lys Val Arg Pro Thr Tyr Ser Thr Asn Ala Ser Ser Tyr Pro
        275                 280                 285

Ile Gly Glu Cys Thr Trp Gly Val Lys Thr Leu Ala Pro Trp Ala Gly
    290                 295                 300

Asp Tyr Trp Gly Asn Gly Ala Gln Trp Ala Thr Ser Ala Ala Ala Ala
305                 310                 315                 320

Gly Phe Arg Thr Gly Ser Thr Pro Gln Val Gly Ala Ile Ala Cys Trp
                325                 330                 335

Asn Asp Gly Gly Tyr Gly His Val Ala Val Val Thr Ala Val Glu Ser
            340                 345                 350

Thr Thr Arg Ile Gln Val Ser Glu Ser Asn Tyr Ala Gly Asn Arg Thr
        355                 360                 365

Ile Gly Asn His Arg Gly Trp Phe Asn Pro Thr Thr Thr Ser Glu Gly
    370                 375                 380

Phe Val Thr Tyr Ile Tyr Ala Asp
385                 390

<210>  19
<211>  392
<212>  PRT
<213>  Streptococcus pneumoniae CDC 11 (serotype 7F)

<400>  19

Met Lys Lys Lys Ile Leu Ala Ser Leu Leu Leu Ser Thr Val Met Val
1               5                   10                  15

Ser Gln Val Ala Val Leu Thr Thr Ala His Ala Glu Thr Thr Asp Asp
            20                  25                  30

Lys Ile Ala Ala Gln Asp Asn Lys Ile Ser Asn Leu Thr Ala Gln Gln
        35                  40                  45

Gln Glu Ala Gln Lys Gln Val Asp Gln Ile Gln Glu Gln Val Ser Ala
    50                  55                  60

Ile Gln Ala Glu Gln Ser Asn Leu Gln Ala Glu Asn Asp Arg Leu Gln
65                  70                  75                  80

Ala Glu Ser Lys Lys Leu Glu Gly Glu Ile Thr Glu Leu Ser Lys Asn
                85                  90                  95

Ile Val Ser Arg Asn Gln Ser Leu Glu Lys Gln Ala Arg Ser Ala Gln
            100                 105                 110

Thr Asn Gly Ala Val Thr Ser Tyr Ile Asn Thr Ile Val Asn Ser Lys
        115                 120                 125

Ser Ile Thr Glu Ala Ile Ser Arg Val Ala Ala Met Ser Glu Ile Val
    130                 135                 140

Ser Ala Asn Asn Lys Met Leu Glu Gln Gln Lys Ala Asp Lys Lys Ala
145                 150                 155                 160

Ile Ser Glu Lys Gln Val Ala Asn Asn Asp Ala Ile Asn Thr Val Ile
                165                 170                 175

Ala Asn Gln Gln Lys Leu Ala Asp Asp Ala Gln Ala Leu Thr Thr Lys
            180                 185                 190

Gln Ala Glu Leu Lys Ala Ala Glu Leu Ser Leu Ala Ala Glu Lys Ala
        195                 200                 205

Thr Ala Glu Gly Glu Lys Ala Ser Leu Leu Glu Gln Lys Ala Ala Ala
    210                 215                 220

Glu Ala Glu Ala Arg Ala Ala Ala Val Ala Glu Ala Ala Tyr Lys Glu
225                 230                 235                 240

Lys Arg Ala Ser Gln Gln Gln Ser Val Leu Ala Ser Ala Asn Thr Asn
                245                 250                 255

Leu Thr Ala Gln Val Gln Ala Val Tyr Glu Ser Ala Ala Ala Pro Val
            260                 265                 270

Arg Ala Lys Val Arg Pro Thr Tyr Ser Thr Asn Ala Ser Ser Tyr Pro
        275                 280                 285

Ile Gly Glu Cys Thr Trp Gly Val Lys Thr Leu Ala Pro Trp Ala Gly
    290                 295                 300

Asp Tyr Trp Gly Asn Gly Ala Gln Trp Ala Thr Ser Ala Ala Ala Ala
305                 310                 315                 320

Gly Phe Arg Thr Gly Ser Thr Pro Gln Val Gly Ala Ile Ala Cys Trp
                325                 330                 335

Asn Asp Gly Gly Tyr Gly His Val Ala Val Val Thr Ala Val Glu Ser
            340                 345                 350

Thr Thr Arg Ile Gln Val Ser Glu Ser Asn Tyr Ala Gly Asn Arg Thr
        355                 360                 365

Ile Gly Asn His Arg Gly Trp Phe Asn Pro Thr Thr Thr Ser Glu Gly
    370                 375                 380

Phe Val Thr Tyr Ile Tyr Ala Asp
385                 390

<210>  20
<211>  392
<212>  PRT
<213>  Streptococcus pneumoniae CDC 24 (serotype 11D)

<400>  20

Met Lys Lys Lys Ile Leu Ala Ser Leu Leu Leu Ser Thr Val Met Val
1               5                   10                  15

Ser Gln Val Ala Val Leu Thr Thr Ala His Ala Glu Thr Thr Asp Asp
            20                  25                  30

Lys Ile Ala Ala Gln Asp Asn Lys Ile Ser Asn Leu Thr Ala Gln Gln
        35                  40                  45

Gln Glu Ala Gln Lys Gln Val Asp Gln Ile Gln Glu Gln Val Ser Ala
    50                  55                  60

Ile Gln Ala Glu Gln Ser Asn Leu Gln Ala Glu Asn Asp Arg Leu Gln
65                  70                  75                  80

Ala Glu Ser Lys Lys Leu Glu Ser Glu Ile Thr Glu Leu Ser Lys Asn
                85                  90                  95

Ile Val Ser Arg Asn Gln Ser Leu Glu Lys Gln Ala Arg Ser Ala Gln
            100                 105                 110

Thr Asn Gly Ala Val Thr Ser Tyr Ile Asn Thr Ile Val Asn Ser Lys
        115                 120                 125

Ser Ile Thr Glu Ala Ile Ser Arg Val Ala Ala Met Ser Glu Ile Val
    130                 135                 140

Ser Ala Asn Asn Lys Met Leu Glu Gln Gln Lys Ala Asp Lys Lys Ala
145                 150                 155                 160

Ile Ser Glu Lys Gln Val Ala Asn Asn Asp Ala Ile Asn Thr Val Ile
                165                 170                 175

Ala Asn Gln Gln Lys Leu Ala Asp Asp Ala Gln Ala Leu Thr Thr Lys
            180                 185                 190

Gln Ala Glu Leu Lys Ala Ala Glu Leu Ser Leu Ala Ala Glu Lys Ala
        195                 200                 205

Thr Ala Glu Gly Glu Lys Ala Ser Leu Leu Glu Gln Lys Ala Ala Ala
    210                 215                 220

Glu Ala Glu Ala Arg Ala Ala Ala Val Ala Glu Ala Ala Tyr Lys Glu
225                 230                 235                 240

Lys Arg Ala Ser Gln Gln Gln Ser Val Leu Ala Ser Ala Asn Thr Asn
                245                 250                 255

Leu Thr Ala Gln Val Gln Ala Val Ser Glu Ser Ala Ala Ala Pro Val
            260                 265                 270

Arg Ala Lys Val Arg Pro Thr Tyr Ser Thr Asn Ala Ser Ser Tyr Pro
        275                     280                     285

Ile Gly Glu Cys Thr Trp Gly Val Lys Thr Leu Ala Pro Trp Ala Gly
    290                     295                     300

Asp Tyr Trp Gly Asn Gly Ala Gln Trp Ala Thr Ser Ala Ala Ala Ala
305                     310                     315                     320

Gly Phe Arg Thr Gly Ser Thr Pro Gln Val Gly Ala Ile Ala Cys Trp
                325                     330                     335

Asn Asp Gly Gly Tyr Gly His Val Ala Val Val Thr Ala Val Glu Ser
            340                     345                     350

Thr Thr Arg Ile Gln Val Ser Glu Ser Asn Tyr Ala Gly Asn Arg Thr
        355                     360                     365

Ile Gly Asn His Arg Gly Trp Phe Asn Pro Thr Thr Thr Ser Glu Gly
    370                     375                     380

Phe Val Thr Tyr Ile Tyr Ala Asp
385                     390

<210>  21
<211>  392
<212>  PRT
<213>  Streptococcus pneumoniae CDC 25 (serotype 11F)

<400>  21

Met Lys Lys Lys Ile Leu Ala Ser Leu Leu Leu Ser Thr Val Met Val
1               5                   10                  15

Ser Gln Val Ala Val Leu Thr Thr Ala His Ala Glu Thr Thr Asp Asp
            20                  25                  30

Lys Ile Ala Ala Gln Asp Asn Lys Ile Ser Asn Leu Thr Ala Gln Gln
        35                  40                  45

Gln Glu Ala Gln Lys Gln Val Asp Gln Ile Gln Glu Gln Val Ser Ala
    50                  55                  60

Ile Gln Ala Glu Gln Ser Asn Leu Gln Ala Glu Asn Asp Arg Leu Gln
65                  70                  75                  80

Ala Glu Ser Lys Lys Leu Glu Gly Glu Ile Thr Glu Leu Ser Lys Asn
                85                  90                  95

Ile Val Ser Arg Asn Gln Ser Leu Glu Lys Gln Ala Arg Ser Ala Gln
            100                 105                 110

Thr Asn Gly Ala Val Thr Ser Tyr Ile Asn Thr Ile Val Asn Ser Lys

        115                 120                 125

Ser Ile Thr Glu Val Ile Ser Arg Val Ala Ala Met Ser Glu Ile Val
    130                 135                 140

Ser Ala Asn Asn Lys Met Leu Glu Gln Gln Lys Ala Asp Lys Lys Ala
145                 150                 155                 160

Ile Ser Glu Lys Gln Val Ala Asn Asn Asp Ala Ile Asn Thr Val Ile
                165                 170                 175

Ala Asn Gln Gln Lys Leu Ala Asp Asp Ala Gln Ala Leu Thr Thr Lys
            180                 185                 190

Gln Ala Glu Leu Lys Ala Ala Glu Leu Ser Leu Ala Ala Glu Lys Ala
        195                 200                 205

Thr Ala Glu Gly Glu Lys Ala Ser Leu Leu Glu Gln Lys Ala Ala Ala
    210                 215                 220

Glu Ala Glu Ala Arg Ala Ala Ala Val Ala Glu Ala Ala Tyr Lys Glu
225                 230                 235                 240

Lys Arg Ala Ser Gln Gln Gln Ser Val Leu Ala Ser Ala Asn Thr Asn
                245                 250                 255

Leu Thr Ala Gln Val Gln Ala Val Ser Glu Ser Ala Ala Ala Pro Val
            260                 265                 270

Arg Ala Lys Val Arg Pro Thr Tyr Ser Thr Asn Ala Ser Ser Tyr Pro
        275                 280                 285

Ile Gly Glu Cys Thr Trp Gly Val Lys Thr Leu Ala Pro Trp Ala Gly
    290                 295                 300

Asp Tyr Trp Gly Asn Gly Ala Gln Trp Ala Thr Ser Ala Ala Ala Ala
305                 310                 315                 320

Gly Phe Arg Thr Gly Ser Thr Pro Gln Val Gly Ala Ile Ala Cys Trp
                325                 330                 335

Asn Asp Gly Gly Tyr Gly His Val Ala Val Val Thr Ala Val Glu Ser
            340                 345                 350

Thr Thr Arg Ile Gln Val Ser Glu Ser Asn Tyr Ala Gly Asn Arg Thr
        355                 360                 365

Ile Gly Asn His Arg Gly Trp Phe Asn Pro Thr Thr Thr Ser Glu Gly
    370                 375                 380

Phe Val Thr Tyr Ile Tyr Ala Asp

385                 390

<210>  22
<211>  392
<212>  PRT
<213>  Streptococcus pneumoniae CDC 27 (serotype 12B)

<400>  22

Met Lys Lys Lys Ile Leu Ala Ser Leu Leu Leu Ser Thr Val Met Val
1               5                   10                  15

Ser Gln Val Ala Val Leu Thr Thr Ala His Ala Glu Thr Thr Asp Asp
            20                  25                  30

Lys Ile Ala Ala Gln Asp Asn Lys Ile Ser Asn Leu Thr Ala Gln Gln
        35                  40                  45

Gln Glu Ala Gln Lys Gln Val Asp Gln Ile Gln Glu Gln Val Ser Ala
    50                  55                  60

Ile Gln Ala Glu Gln Ser Asn Leu Gln Ala Glu Asn Asp Arg Leu Gln
65                  70                  75                  80

Ala Glu Ser Lys Lys Leu Glu Ser Glu Ile Thr Glu Leu Ser Lys Asn
                85                  90                  95

Ile Val Ser Arg Asn Gln Ser Leu Glu Lys Gln Ala Arg Ser Ala Gln
            100                 105                 110

Thr Asn Gly Ala Val Thr Ser Tyr Ile Asn Thr Ile Val Asn Ser Lys
        115                 120                 125

Ser Ile Thr Glu Ala Ile Ser Arg Val Ala Ala Met Ser Glu Ile Val
    130                 135                 140

Ser Ala Asn Asn Lys Met Leu Glu Gln Gln Lys Ala Asp Lys Lys Ala
145                 150                 155                 160

Ile Ser Glu Lys Gln Val Ala Asn Asn Asp Ala Ile Asn Thr Val Ile
                165                 170                 175

Ala Asn Gln Gln Lys Leu Ala Asp Asp Ala Gln Ala Leu Thr Thr Lys
            180                 185                 190

Gln Ala Glu Leu Lys Ala Ala Glu Leu Ser Leu Ala Ala Glu Lys Ala
        195                 200                 205

Thr Ala Glu Gly Glu Lys Ala Ser Leu Leu Glu Gln Lys Ala Ala Ala
    210                 215                 220

Glu Ala Glu Ala Arg Ala Ala Ala Val Ala Glu Ala Ala Tyr Lys Glu
225                 230                 235                 240

Lys Arg Ala Ser Gln Gln Gln Ser Val Leu Ala Ser Ala Asn Thr Asn
                245                 250                 255

Leu Thr Ala Gln Val Gln Ala Val Ser Glu Ser Ala Ala Ala Pro Val
            260                 265                 270

Arg Ala Lys Val Arg Pro Thr Tyr Ser Thr Asn Ala Ser Ser Tyr Pro
        275                 280                 285

Ile Gly Glu Cys Thr Trp Gly Val Lys Thr Leu Ala Pro Trp Ala Gly
    290                 295                 300

Asp Tyr Trp Gly Asn Gly Ala Gln Trp Ala Thr Ser Ala Ala Ala Ala
305                 310                 315                 320

Gly Phe Arg Thr Gly Ser Thr Pro Gln Val Gly Ala Ile Ala Cys Trp
                325                 330                 335

Asn Asp Gly Gly Tyr Gly His Val Ala Val Val Thr Ala Val Glu Ser
            340                 345                 350

Thr Thr Arg Ile Gln Val Ser Glu Ser Asn Tyr Ala Gly Asn Arg Thr
        355                 360                 365

Ile Gly Asn His Arg Gly Trp Phe Asn Pro Thr Thr Thr Ser Glu Gly
    370                 375                 380

Phe Val Thr Tyr Ile Tyr Ala Asp
385                 390

<210>  23
<211>  392
<212>  PRT
<213>  Streptococcus pneumoniae CDC 49 (serotype 22A)

<400>  23

Met Lys Lys Lys Ile Leu Ala Ser Leu Leu Leu Ser Thr Val Met Val
1               5                   10                  15

Ser Gln Val Ala Val Leu Thr Thr Ala His Ala Glu Thr Thr Asp Asp
            20                  25                  30

Lys Ile Ala Ala Gln Asp Asn Lys Ile Ser Asn Leu Thr Ala Gln Gln
        35                  40                  45

Gln Glu Ala Gln Lys Gln Val Asp Gln Ile Gln Glu Gln Val Ser Ala
    50                  55                  60

Ile Gln Ala Glu Gln Ser Asn Leu Gln Ala Glu Asn Asp Arg Leu Gln
65                  70                  75                  80

Ala Glu Ser Lys Lys Leu Glu Gly Glu Ile Thr Glu Leu Ser Lys Asn
                85                  90                  95

Ile Val Ser Arg Asn Gln Ser Leu Glu Lys Gln Ala Arg Ser Ala Gln
            100                 105                 110

Thr Asn Gly Ala Val Thr Ser Tyr Ile Asn Thr Ile Val Asn Ser Lys
        115                 120                 125

Ser Ile Thr Glu Ala Ile Ser Arg Val Ala Ala Met Ser Glu Ile Val
    130                 135                 140

Ser Ala Asn Asn Lys Met Leu Glu Gln Gln Lys Ala Asp Lys Lys Ala
145                 150                 155                 160

Ile Ser Glu Lys Gln Val Ala Asn Asn Asp Ala Ile Asn Thr Val Ile
                165                 170                 175

Ala Asn Gln Gln Lys Leu Ala Asp Asp Ala Gln Ala Leu Thr Thr Lys
            180                 185                 190

Gln Ala Glu Leu Lys Ala Ala Glu Leu Ser Leu Ala Ala Glu Arg Ala
        195                 200                 205

Thr Ala Glu Gly Glu Lys Ala Ser Leu Leu Glu Gln Lys Ala Ala Ala
    210                 215                 220

Glu Ala Glu Ala Arg Ala Ala Ala Val Ala Glu Ala Ala Tyr Lys Glu
225                 230                 235                 240

Lys Arg Ala Ser Gln Gln Gln Ser Val Leu Ala Ser Ala Asn Thr Asn
                245                 250                 255

Leu Thr Ala Gln Val Gln Ala Val Ser Glu Ser Ala Ala Ala Pro Val
            260                 265                 270

Arg Ala Lys Val Arg Pro Thr Tyr Ser Thr Asn Ala Ser Ser Tyr Pro
        275                 280                 285

Ile Gly Glu Cys Thr Trp Gly Val Lys Thr Leu Ala Pro Trp Ala Gly
    290                 295                 300

Asp Tyr Trp Gly Asn Gly Ala Gln Trp Ala Thr Ser Ala Ala Ala Ala
305                 310                 315                 320

Gly Phe Arg Thr Gly Ser Thr Pro Gln Val Gly Ala Ile Ala Cys Trp
                325                 330                 335

Asn Asp Gly Gly Tyr Gly His Val Ala Val Val Thr Ala Val Glu Ser
            340                 345                 350

Thr Thr Arg Ile Gln Val Ser Glu Ser Asn Tyr Ala Gly Asn Arg Thr
        355                 360                 365

Ile Gly Asn His Arg Gly Trp Phe Asn Pro Thr Thr Thr Ser Glu Gly
    370                 375                 380

Phe Val Thr Tyr Ile Tyr Ala Asp
385                 390

<210>  24
<211>  392
<212>  PRT
<213>  Streptococcus pneumoniae CDC 56 (serotype 24F)

<400>  24

Met Lys Lys Lys Ile Leu Ala Ser Leu Leu Leu Ser Thr Val Met Val
1               5                   10                  15

Ser Gln Val Ala Val Leu Thr Thr Ala His Ala Glu Thr Thr Asp Asp
            20                  25                  30

Lys Ile Ala Ala Gln Asp Asn Lys Ile Ser Asn Leu Thr Ala Gln Gln
        35                  40                  45

Gln Glu Ala Gln Lys Gln Val Asp Gln Ile Gln Glu Gln Val Ser Ala
    50                  55                  60

Ile Gln Ala Glu Gln Ser Asn Leu Gln Ala Glu Asn Asp Arg Leu Gln
65                  70                  75                  80

Ala Glu Ser Lys Lys Leu Glu Gly Glu Ile Thr Glu Leu Ser Lys Asn
                85                  90                  95

Ile Val Ser Arg Asn Gln Ser Leu Glu Lys Gln Ala Arg Ser Ala Gln
            100                 105                 110

Thr Asn Gly Ala Val Thr Ser Tyr Ile Asn Thr Ile Val Asn Ser Lys
        115                 120                 125

Ser Ile Thr Glu Ala Ile Ser Arg Val Ala Ala Met Ser Glu Ile Val
    130                 135                 140

Ser Ala Asn Asn Lys Met Leu Glu Gln Gln Lys Ala Asp Lys Lys Ala
145                 150                 155                 160

Ile Ser Glu Lys Gln Val Ala Asn Asn Asp Ala Ile Asn Ile Val Ile
                165                 170                 175

Ala Asn Gln Gln Lys Leu Ala Asp Asp Ala Gln Ala Leu Thr Thr Lys
            180                 185                 190

Gln Ala Glu Leu Lys Ala Ala Glu Leu Ser Leu Ala Ala Glu Lys Ala
        195                 200                 205

Thr Ala Glu Gly Glu Lys Ala Ser Leu Leu Glu Gln Lys Ala Ala Ala
    210                 215                 220

Glu Ala Glu Ala Arg Ala Ala Ala Val Ala Glu Ala Ala Tyr Lys Glu
225                 230                 235                 240

Lys Arg Ala Ser Gln Gln Gln Ser Val Leu Ala Ser Ala Asn Thr Asn
                245                 250                 255

Leu Thr Ala Gln Val Gln Ala Val Ser Glu Ser Ala Ala Ala Pro Val
            260                 265                 270

Arg Ala Lys Val Arg Pro Thr Tyr Ser Thr Asn Ala Ser Ser Tyr Pro
        275                 280                 285

Ile Gly Glu Cys Thr Trp Gly Val Lys Thr Leu Ala Pro Trp Ala Gly
    290                 295                 300

Asp Tyr Trp Gly Asn Gly Ala Gln Trp Ala Thr Ser Ala Ala Ala Ala
305                 310                 315                 320

Gly Phe Arg Thr Gly Ser Thr Pro Gln Val Gly Ala Ile Ala Cys Trp
                325                 330                 335

Asn Asp Gly Gly Tyr Gly His Val Ala Val Val Thr Ala Val Glu Ser
            340                 345                 350

Thr Thr Arg Ile Gln Val Ser Glu Ser Asn Tyr Ala Gly Asn Arg Thr
        355                 360                 365

Ile Gly Asn His Arg Gly Trp Phe Asn Pro Thr Thr Thr Ser Glu Gly
    370                 375                 380

Phe Val Thr Tyr Ile Tyr Ala Asp
385                 390

<210>  25
<211>  392
<212>  PRT
<213>  Streptococcus pneumoniae CDC 61 (serotype 28F)

<400>  25

Met Lys Lys Lys Ile Leu Ala Ser Leu Leu Leu Ser Thr Val Met Val
1               5                   10                  15

Ser Gln Val Ala Val Leu Thr Thr Ala His Ala Glu Thr Thr Asp Asp
            20                  25                  30

Lys Ile Ala Ala Gln Asp Asn Lys Ile Ser Asn Leu Thr Ala Gln Gln

        35                  40                  45

Gln Glu Ala Gln Lys Gln Val Asp Gln Ile Gln Glu Gln Val Ser Ala
    50                  55                  60

Ile Gln Ala Glu Gln Ser Asn Leu Gln Ala Glu Asn Asp Arg Leu Gln
65                  70                  75                  80

Ala Glu Ser Lys Lys Leu Glu Gly Glu Ile Thr Glu Leu Ser Lys Asn
                85                  90                  95

Ile Val Ser Arg Asn Gln Ser Leu Glu Lys Gln Ala Arg Ser Ala Gln
            100                 105                 110

Thr Asn Gly Ala Val Thr Ser Tyr Ile Asn Thr Ile Val Asn Ser Lys
        115                 120                 125

Ser Ile Thr Glu Ala Ile Ser Arg Val Ala Ala Met Ser Glu Ile Val
    130                 135                 140

Ser Ala Asn Asn Lys Met Leu Glu Gln Gln Lys Ala Asp Lys Lys Ala
145                 150                 155                 160

Ile Ser Glu Lys Gln Val Ala Asn Asn Asp Ala Ile Asn Thr Val Ile
                165                 170                 175

Ala Asn Gln Gln Lys Leu Ala Asp Asp Ala Gln Ala Leu Thr Thr Lys
            180                 185                 190

Gln Ala Glu Leu Lys Ala Ala Glu Leu Ser Leu Ala Ala Glu Lys Ala
        195                 200                 205

Thr Ala Glu Gly Glu Lys Ala Ser Leu Leu Glu Gln Lys Ala Ala Ala
    210                 215                 220

Glu Ala Glu Ala Arg Ala Ala Ala Val Ala Glu Ala Ala Tyr Lys Glu
225                 230                 235                 240

Lys Arg Ala Ser Gln Gln Gln Ser Val Leu Ala Ser Ala Asn Thr Asn
                245                 250                 255

Leu Thr Ala Gln Val Gln Ala Val Ser Glu Ser Ala Ala Ala Pro Val
            260                 265                 270

Arg Ala Lys Val Arg Pro Thr Tyr Ser Thr Asn Ala Ser Ser Tyr Pro
        275                 280                 285

Ile Gly Glu Cys Thr Trp Gly Val Lys Thr Leu Ala Pro Trp Ala Glu
    290                 295                 300

Asp Tyr Trp Gly Asn Gly Ala Gln Trp Ala Thr Ser Ala Ala Ala Ala

305                 310                 315                 320

Gly Phe Arg Thr Gly Ser Thr Pro Gln Val Gly Ala Ile Ala Cys Trp
                325                 330                 335

Asn Asp Gly Gly Tyr Gly His Val Ala Val Val Thr Ala Val Glu Ser
            340                 345                 350

Thr Thr Arg Ile Gln Val Ser Glu Ser Asn Tyr Ala Gly Asn Arg Thr
        355                 360                 365

Ile Gly Asn His Arg Gly Trp Phe Asn Pro Thr Thr Thr Ser Glu Gly
    370                 375                 380

Phe Val Thr Tyr Ile Tyr Ala Asp
385                 390

<210>  26
<211>  392
<212>  PRT
<213>  Streptococcus pneumoniae CDC 97 (serotype 19A)

<400>  26

Met Lys Lys Lys Ile Leu Ala Ser Leu Leu Leu Ser Thr Val Met Val
1               5                   10                  15

Ser Gln Val Ala Val Leu Thr Thr Ala His Ala Glu Thr Thr Asp Asp
            20                  25                  30

Lys Ile Ala Ala Gln Asp Asn Lys Ile Ser Asn Leu Thr Ala Gln Gln
        35                  40                  45

Gln Glu Ala Gln Lys Gln Val Asp Gln Ile Gln Glu Gln Val Ser Ala
    50                  55                  60

Ile Gln Ala Glu Gln Ser Asn Leu Gln Ala Glu Asn Asp Arg Leu Gln
65                  70                  75                  80

Ala Glu Ser Lys Lys Leu Glu Gly Glu Ile Thr Glu Leu Ser Lys Asn
                85                  90                  95

Ile Val Ser Arg Asn Gln Ser Leu Glu Lys Gln Ala Arg Ser Ala Gln
            100                 105                 110

Thr Asn Gly Ala Val Thr Ser Tyr Ile Asn Thr Ile Val Asn Ser Lys
        115                 120                 125

Ser Ile Thr Glu Ala Ile Ser Arg Val Ala Ala Met Ser Glu Ile Val
    130                 135                 140

Ser Ala Asn Asn Lys Met Leu Glu Gln Gln Lys Ala Asp Lys Lys Ala
145                 150                 155                 160

Ile Ser Glu Lys Gln Val Ala Asn Asn Asp Ala Ile Asn Thr Val Ile
                165                 170                 175

Ala Asn Gln Gln Lys Leu Ala Asp Asp Ala Gln Ala Leu Thr Thr Lys
            180                 185                 190

Gln Ala Glu Leu Lys Ala Ala Glu Leu Ser Leu Ala Ala Glu Lys Ala
        195                 200                 205

Thr Ala Glu Gly Glu Lys Ala Ser Leu Leu Glu Gln Lys Ala Ala Ala
    210                 215                 220

Glu Ala Glu Ala Arg Ala Ala Ala Val Ala Glu Ala Ala Tyr Lys Glu
225                 230                 235                 240

Lys Arg Ala Ser Gln Gln Gln Ser Val Leu Ala Ser Ala Asn Thr Asn
                245                 250                 255

Leu Thr Ala Gln Val Gln Ala Val Ser Glu Ser Ala Ala Ala Pro Val
            260                 265                 270

Arg Ala Lys Val Arg Pro Thr Tyr Ser Thr Asn Ala Ser Ser Tyr Pro
        275                 280                 285

Ile Gly Glu Cys Thr Trp Gly Val Lys Thr Leu Ala Pro Trp Ala Gly
    290                 295                 300

Asp Tyr Trp Gly Asn Gly Gly Gln Trp Ala Thr Ser Ala Ala Ala Ala
305                 310                 315                 320

Gly Phe Arg Thr Gly Ser Thr Pro Gln Val Gly Ala Ile Ala Cys Trp
                325                 330                 335

Asn Asp Gly Gly Tyr Gly His Val Ala Val Val Thr Ala Val Glu Ser
            340                 345                 350

Thr Thr Arg Ile Gln Val Ser Glu Ser Asn Tyr Ala Gly Asn Arg Thr
        355                 360                 365

Ile Gly Asn His Arg Gly Trp Phe Asn Pro Thr Thr Thr Ser Glu Gly
    370                 375                 380

Phe Val Thr Tyr Ile Tyr Ala Asp
385                 390

<210>  27
<211>  391
<212>  PRT
<213>  Streptococcus pneumoniae CDC 103 (serotype 19A)

<400>  27

Met Lys Lys Lys Ile Leu Ala Ser Leu Leu Leu Ser Thr Val Met Val
1               5                   10                  15

Ser Gln Val Ala Val Leu Thr Thr Ala His Ala Glu Thr Thr Asp Asp
            20                  25                  30

Lys Ile Ala Ala Gln Asp Asn Lys Ile Ser Asn Leu Thr Ala Gln Gln
        35                  40                  45

Gln Glu Ala Gln Lys Gln Val Asp Gln Ile Gln Glu Gln Val Ser Ala
    50                  55                  60

Ile Gln Ala Glu Gln Ser Asn Leu Gln Ala Glu Asn Asp Arg Leu Gln
65                  70                  75                  80

Ala Glu Ser Lys Lys Leu Glu Gly Glu Ile Thr Glu Leu Ser Lys Asn
                85                  90                  95

Ile Val Ser Arg Asn Gln Ser Leu Glu Lys Gln Ala Arg Ser Ala Gln
            100                 105                 110

Thr Asn Gly Ala Val Thr Ser Tyr Ile Asn Thr Ile Val Asn Ser Lys
        115                 120                 125

Ser Ile Thr Glu Ala Ile Ser Arg Val Ala Ala Met Ser Glu Ile Val
    130                 135                 140

Ser Ala Asn Asn Lys Met Leu Glu Gln Gln Lys Ala Asp Lys Lys Ala
145                 150                 155                 160

Ile Ser Glu Lys Gln Val Ala Asn Asn Asp Ala Ile Asn Thr Val Ile
                165                 170                 175

Ala Asn Gln Gln Lys Leu Ala Asp Asp Ala Gln Ala Leu Thr Thr Lys
            180                 185                 190

Gln Ala Glu Leu Lys Ala Ala Glu Ser Leu Ala Ala Glu Lys Ala Thr
        195                 200                 205

Ala Glu Gly Glu Lys Ala Ser Leu Leu Glu Gln Lys Ala Ala Ala Glu
    210                 215                 220

Ala Glu Ala Arg Ala Ala Ala Ala Ala Glu Ala Ala Tyr Lys Glu Lys
225                 230                 235                 240

Gln Ala Ser Gln Gln Gln Ser Val Leu Ala Ser Gly Asn Thr Asn Leu
                245                 250                 255

Thr Ala Gln Val Gln Ala Val Ser Glu Ser Ala Val Ala Pro Val Gln
            260                 265                 270

Ala Lys Val Arg Pro Thr Tyr Asn Thr Asp Ala Ser Thr Tyr Pro Ile
        275                 280                 285

Gly Glu Cys Thr Trp Gly Val Lys Thr Leu Ala Pro Trp Ala Gly Asp
    290                 295                 300

Tyr Trp Gly Asn Gly Ala Gln Trp Ala Thr Ser Ala Ala Ala Ala Gly
305                 310                 315                 320

Phe Arg Thr Gly Ser Thr Pro Gln Val Gly Ala Ile Ala Cys Trp Asn
                325                 330                 335

Asp Gly Gly Tyr Gly His Val Ala Val Val Thr Ala Val Glu Ser Thr
            340                 345                 350

Thr Arg Ile Gln Val Ser Glu Ser Asn Tyr Ala Gly Asn Arg Thr Ile
        355                 360                 365

Gly Asn His Arg Gly Trp Phe Asn Pro Thr Thr Thr Ser Glu Gly Phe
    370                 375                 380

Val Thr Tyr Ile Tyr Ala Asp
385                 390

<210>  28
<211>  392
<212>  PRT
<213>  Streptococcus pneumoniae CDC 106 (serotype 23F)

<400>  28

Met Lys Lys Lys Ile Leu Ala Ser Leu Leu Leu Ser Thr Val Met Val
1               5                   10                  15

Ser Gln Val Ala Val Leu Thr Thr Ala His Ala Glu Thr Thr Asp Asp
            20                  25                  30

Lys Ile Ala Ala Gln Asp Asn Lys Ile Ser Asn Leu Thr Ala Gln Gln
        35                  40                  45

Gln Glu Ala Gln Lys Gln Val Asp Gln Ile Gln Glu Gln Val Ser Ala
    50                  55                  60

Ile Gln Ala Glu Gln Ser Asn Leu Gln Ala Glu Asn Asp Arg Leu Gln
65                  70                  75                  80

Ala Glu Ser Lys Lys Leu Glu Gly Glu Ile Thr Glu Leu Ser Lys Asn
                85                  90                  95

Ile Val Ser Arg Asn Gln Ser Leu Glu Lys Gln Ala Arg Ser Ala Gln
            100                 105                 110

Thr Asn Gly Ala Val Thr Ser Tyr Ile Asn Thr Ile Val Asn Ser Lys
        115                 120                 125

Ser Ile Thr Glu Ala Ile Ser Arg Val Ala Ala Met Ser Glu Ile Val
    130                 135                 140

Ser Ala Asn Asn Lys Met Leu Glu Gln Gln Lys Ala Asp Lys Lys Ala
145                 150                 155                 160

Ile Ser Glu Lys Gln Val Ala Asn Asn Asp Ala Ile Asn Thr Val Ile
                165                 170                 175

Ala Asn Gln Gln Lys Leu Ala Asp Asp Ala Gln Ala Leu Thr Thr Lys
            180                 185                 190

Gln Ala Glu Leu Lys Ala Ala Glu Leu Ser Leu Ala Ala Glu Lys Ala
        195                 200                 205

Thr Ala Glu Gly Glu Lys Ala Ser Leu Leu Glu Gln Lys Ala Ala Ala
    210                 215                 220

Glu Ala Glu Ala Arg Ala Ala Ala Val Ala Glu Ala Ala Tyr Lys Glu
225                 230                 235                 240

Lys Arg Ala Ser Gln Gln Gln Ser Val Leu Ala Ser Ala Asn Thr Asn
                245                 250                 255

Leu Thr Ala Gln Val Gln Ala Val Ser Glu Ser Ala Ala Ala Pro Val
            260                 265                 270

Arg Ala Lys Val Arg Pro Thr Tyr Ser Thr Asn Ala Ser Ser Tyr Pro
        275                 280                 285

Ile Gly Glu Cys Thr Trp Gly Val Lys Thr Leu Ala Pro Trp Ala Gly
    290                 295                 300

Asp Tyr Trp Gly Asn Gly Ala Gln Trp Ala Thr Ser Ala Ala Ala Ala
305                 310                 315                 320

Gly Phe Arg Thr Gly Ser Thr Pro Gln Val Gly Ala Ile Ala Cys Trp
                325                 330                 335

Asn Asp Gly Gly Tyr Gly His Val Ala Val Val Thr Ala Val Glu Ser
            340                 345                 350

Thr Thr Arg Ile Gln Val Ser Glu Ser Asn Tyr Ser Gly Asn Arg Thr
        355                 360                 365

Ile Gly Asn His Arg Gly Trp Phe Asn Pro Thr Thr Thr Ser Glu Gly
    370                 375                 380

Phe Val Thr Tyr Ile Tyr Ala Asp
385                 390

<210>  29
<211>  392
<212>  PRT
<213>  Streptococcus pneumoniae CDC 112 (serotype 6B)

<400>  29

Met Lys Lys Lys Ile Leu Ala Ser Leu Leu Leu Ser Thr Val Met Val
1               5                   10                  15

Ser Gln Val Ala Val Leu Thr Thr Ala His Ala Glu Thr Thr Asp Asp
            20                  25                  30

Lys Ile Ala Ala Gln Asp Asn Lys Ile Ser Asn Leu Thr Ala Gln Gln
        35                  40                  45

Gln Glu Ala Gln Lys Gln Val Asp Gln Ile Gln Glu Gln Val Ser Ala
    50                  55                  60

Ile Gln Ala Glu Gln Ser Asn Leu Gln Ala Glu Asn Asp Arg Leu Gln
65                  70                  75                  80

Ala Glu Ser Lys Lys Leu Glu Gly Glu Ile Thr Glu Leu Ser Lys Asn
                85                  90                  95

Ile Val Ser Arg Asn Gln Ser Leu Glu Lys Gln Ala Arg Ser Ala Gln
            100                 105                 110

Thr Asn Gly Ala Val Thr Ser Tyr Ile Asn Thr Ile Val Asn Ser Lys
        115                 120                 125

Ser Ile Thr Glu Ala Ile Ser Arg Val Ala Ala Met Ser Glu Ile Val
    130                 135                 140

Ser Ala Asn Asn Lys Met Leu Glu Gln Gln Lys Ala Asp Lys Lys Ala
145                 150                 155                 160

Ile Ser Glu Lys Gln Val Ala Asn Asn Asp Ala Ile Asn Thr Val Ile
                165                 170                 175

Ala Asn Gln Gln Lys Leu Ala Asp Asp Ala Gln Ala Leu Thr Thr Lys
            180                 185                 190

Gln Ala Glu Leu Lys Ala Ala Glu Leu Ser Leu Ala Ala Glu Lys Ala
        195                 200                 205

Thr Ala Glu Gly Glu Lys Ala Ser Leu Leu Glu Gln Lys Ala Ala Ala
    210                 215                 220

Glu Ala Glu Ala Arg Ala Ala Ala Val Ala Glu Ala Ala Tyr Lys Glu

225                 230                 235                 240

Lys Arg Ala Ser Gln Gln Gln Ser Val Leu Ala Ser Ala Asn Thr Asn
                245                 250                 255

Leu Thr Ala Gln Val Gln Ala Val Tyr Glu Ser Ala Ala Ala Pro Val
            260                 265                 270

Arg Ala Lys Val Arg Pro Thr Tyr Ser Thr Asn Ala Ser Ser Tyr Pro
        275                 280                 285

Ile Gly Glu Cys Thr Trp Gly Val Lys Thr Leu Ala Pro Trp Ala Gly
    290                 295                 300

Asp Tyr Trp Gly Asn Gly Ala Gln Trp Ala Thr Ser Ala Ala Ala Ala
305                 310                 315                 320

Gly Phe Arg Thr Gly Ser Thr Pro Gln Val Gly Ala Ile Ala Cys Trp
                325                 330                 335

Asn Asp Gly Gly Tyr Gly His Val Ala Val Val Thr Ala Val Glu Ser
            340                 345                 350

Thr Thr Arg Ile Gln Val Ser Glu Ser Asn Tyr Ala Gly Asn Arg Thr
        355                 360                 365

Ile Gly Asn His Arg Gly Trp Phe Asn Pro Thr Thr Thr Ser Glu Gly
    370                 375                 380

Phe Val Thr Tyr Ile Tyr Ala Asp
385                 390

<210>   30
<211>   392
<212>   PRT
<213>   Streptococcus pneumoniae PJ-1280_10A (serotype 10A)

<400>   30

Met Lys Lys Lys Ile Leu Ala Ser Leu Leu Leu Ser Thr Val Met Val
1               5                   10                  15

Ser Gln Val Ala Val Leu Thr Thr Ala His Ala Glu Thr Thr Asp Asp
            20                  25                  30

Lys Ile Ala Ala Gln Asp Asn Lys Ile Ser Asn Leu Thr Ala Gln Gln
        35                  40                  45

Gln Glu Ala Gln Lys Gln Val Asp Gln Ile Gln Glu Gln Val Ser Ala
    50                  55                  60

Ile Gln Ala Glu Gln Ser Asn Leu Gln Ala Glu Asn Asp Arg Leu Gln
65                  70                  75                  80

Ala Glu Ser Lys Lys Leu Glu Gly Glu Ile Thr Glu Leu Ser Lys Asn
                85                  90                  95

Ile Val Ser Arg Asn Gln Ser Leu Glu Lys Gln Ala Arg Ser Ala Gln
            100                 105                 110

Thr Asn Gly Ala Val Thr Ser Tyr Ile Asn Thr Ile Val Asn Ser Lys
        115                 120                 125

Ser Ile Thr Glu Ala Ile Ser Arg Val Ala Ala Met Ser Glu Ile Val
    130                 135                 140

Ser Ala Asn Asn Lys Met Leu Glu Gln Gln Lys Ala Asp Lys Lys Ala
145                 150                 155                 160

Ile Ser Glu Lys Gln Val Ala Asn Asn Asp Ala Ile Asn Thr Val Ile
                165                 170                 175

Ala Asn Gln Gln Lys Leu Ala Asp Asp Ala Gln Ala Leu Thr Thr Lys
            180                 185                 190

Gln Ala Glu Leu Lys Ala Ala Glu Leu Ser Leu Ala Ala Glu Lys Ala
        195                 200                 205

Thr Ala Glu Gly Glu Lys Ala Ser Leu Leu Glu Gln Lys Ala Ala Ala
    210                 215                 220

Glu Ala Glu Ala Arg Ala Ala Ala Val Ala Glu Ala Ala Tyr Lys Glu
225                 230                 235                 240

Lys Arg Ser Ser Gln Gln Gln Ser Val Leu Ala Ser Ala Asn Thr Asn
                245                 250                 255

Leu Thr Ala Gln Val Gln Ala Val Ser Glu Ser Ala Ala Ala Pro Val
            260                 265                 270

Arg Ala Lys Val Arg Pro Thr Tyr Ser Thr Asn Ala Ser Ser Tyr Pro
        275                 280                 285

Ile Gly Glu Cys Thr Trp Gly Val Lys Thr Leu Ala Pro Trp Ala Gly
    290                 295                 300

Asp Tyr Trp Gly Asn Gly Ala Gln Trp Ala Thr Ser Ala Ala Ala Ala
305                 310                 315                 320

Gly Phe Arg Thr Gly Ser Thr Pro Gln Val Gly Ala Ile Ala Cys Trp
                325                 330                 335

Asn Asp Gly Gly Tyr Gly His Val Ala Val Val Thr Ala Val Glu Ser
            340                 345                 350

Thr Thr Arg Ile Gln Val Ser Glu Ser Asn Tyr Ala Gly Asn Arg Thr
        355                 360                 365

Ile Gly Asn His Arg Gly Trp Phe Asn Pro Thr Thr Thr Ser Glu Gly
    370                 375                 380

Phe Val Thr Tyr Ile Tyr Ala Asp
385                 390

<210>  31
<211>  659
<212>  PRT
<213>  Streptococcus pneumoniae CDC 1 (serotype 1)

<400>  31

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  32
<211>  659
<212>  PRT
<213>  Streptococcus pneumoniae CDC 2 (serotype 2)

<400>  32

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210> 33
<211> 659
<212> PRT
<213> Streptococcus pneumoniae CDC 3 (serotype 3)

<400> 33

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala

145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser

            420                 425                 430

Ser Gly Lys Gln Ser Phe Gln Ile Ser Asn Tyr Val Gly Arg Lys Ser
        435                 440                 445

Ser Asp Val Ile Ser Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210> 34
<211> 659
<212> PRT
<213> Streptococcus pneumoniae CDC 4 (serotype 4)

<400> 34

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ser Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  35
<211>  659
<212>  PRT
<213>  Streptococcus pneumoniae CDC 5 (serotype 5)

<400>  35

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Thr Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210> 36
<211> 659
<212> PRT
<213> Streptococcus pneumoniae CDC 8 (serotype 7A)

<400> 36

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Ile Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
                500                 505                 510

Gly Asn Tyr Ile Gly Arg Asn Ser Thr Glu Val Ile Ser Glu Leu Lys
            515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
                580                 585                 590

Glu Phe Thr Lys Asn Asn Leu Ile Gln Ile Val Gly Ile Lys Glu Ala
            595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>   37
<211>   659
<212>   PRT
<213>   Streptococcus pneumoniae CDC 11 (serotype 7F)

<400>   37

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly

65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu

            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu

610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  38
<211>  659
<212>  PRT
<213>  Streptococcus pneumoniae CDC 13 (serotype 9A)

<400>  38

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Ile Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  39
<211>  659
<212>  PRT
<213>  Streptococcus pneumoniae CDC 16 (serotype 9V)

<400>  39

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Ile Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  40
<211>  659
<212>  PRT
<213>  Streptococcus pneumoniae CDC 17 (serotype 10A)

<400>  40

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Thr Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Ile Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  41
<211>  659
<212>  PRT
<213>  Streptococcus pneumoniae CDC 18 (serotype 10B)

<400> 41

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser

            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser

530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  42
<211>  659
<212>  PRT
<213>  Streptococcus pneumoniae CDC 19 (serotype 10C)

<400>  42

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Ala Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Phe Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210> 43
<211> 659
<212> PRT
<213> Streptococcus pneumoniae CDC 20 (serotype 10F)

<400> 43

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  44
<211>  659
<212>  PRT
<213>  Streptococcus pneumoniae CDC 22 (serotype 11B)

<400>  44

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Ser Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  45
<211>  659
<212>  PRT
<213>  Streptococcus pneumoniae CDC 23 (serotype 11C)

<400>  45

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln

            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu

450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  46
<211>  659
<212>  PRT
<213>  Streptococcus pneumoniae CDC 25 (serotype 11F)

<400>  46

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  47
<211>  659
<212>  PRT
<213>  Streptococcus pneumoniae CDC 26 (serotype 12A)

<400>  47

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln His Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Ala Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  48
<211>  659
<212>  PRT

<213>  Streptococcus pneumoniae CDC 28 (serotype 12F)

<400>  48
   Met Ile Gln Ile Gly Lys Ile Phe Ala Gly Arg Tyr Arg Ile Val Lys
   1               5                   10                  15
   Gln Ile Gly Arg Gly Gly Met Ala Asp Val Tyr Leu Ala Lys Asp Leu
               20                  25                  30
   Ile Leu Asp Gly Glu Glu Val Ala Val Lys Val Leu Arg Thr Asn Tyr
           35                  40                  45
   Gln Thr Asp Pro Ile Ala Val Ala Arg Phe Gln Arg Glu Ala Arg Ala
       50                  55                  60
   Met Ala Asp Leu Asp His Pro His Ile Val Arg Ile Thr Asp Ile Gly
   65                  70                  75                  80
   Glu Glu Asp Gly Gln Gln Tyr Leu Ala Met Glu Tyr Val Ala Gly Leu
                   85                  90                  95
   Asp Leu Lys Arg Tyr Ile Lys Glu His Tyr Pro Leu Ser Asn Glu Glu
               100                 105                 110
   Ala Val Arg Ile Met Gly Gln Ile Leu Leu Ala Met Arg Leu Ala His
           115                 120                 125
   Thr Arg Gly Ile Val His Arg Asp Leu Lys Pro Gln Asn Ile Leu Leu
       130                 135                 140
   Thr Pro Asp Gly Thr Ala Lys Val Thr Asp Phe Gly Ile Ala Val Ala
   145                 150                 155                 160
   Phe Ala Glu Thr Ser Leu Thr Gln Thr Asn Ser Met Leu Gly Ser Val
                   165                 170                 175
   His Tyr Leu Ser Pro Glu Gln Ala Arg Gly Ser Lys Ala Thr Val Gln
               180                 185                 190
   Ser Asp Ile Tyr Ala Met Gly Ile Ile Phe Tyr Glu Met Leu Thr Gly
           195                 200                 205
   His Ile Pro Tyr Asp Gly Asp Ser Ala Val Thr Ile Ala Leu Gln His
       210                 215                 220
   Phe Gln Lys Pro Leu Pro Ser Val Ile Ala Glu Asn Pro Ser Val Pro
   225                 230                 235                 240
   Gln Ala Leu Glu Asn Val Ile Ile Lys Ala Thr Ala Lys Lys Leu Thr
                   245                 250                 255
   Asn Arg Tyr Arg Ser Val Ser Glu Met Tyr Val Asp Leu Ser Ser Ser
               260                 265                 270
   Leu Ser Tyr Asn Arg Arg Asn Glu Ser Lys Leu Ile Phe Asp Glu Thr
           275                 280                 285
   Ser Lys Ala Asp Thr Lys Thr Leu Pro Lys Val Ser Gln Ser Thr Leu
       290                 295                 300
   Thr Ser Ile Pro Lys Val Gln Ala Gln Thr Glu His Lys Ser Ile Lys
   305                 310                 315                 320
   Asn Pro Ser Gln Ala Val Thr Glu Glu Thr Tyr Gln Pro Gln Ala Pro
                   325                 330                 335
   Lys Lys His Arg Phe Lys Met Arg Tyr Leu Ile Leu Leu Ala Ser Leu
               340                 345                 350
   Val Leu Val Ala Ala Ser Leu Ile Trp Ile Leu Ser Arg Thr Pro Ala
           355                 360                 365
   Thr Ile Ala Ile Pro Asp Val Ala Gly Gln Thr Val Ala Glu Ala Lys
       370                 375                 380
   Ala Thr Leu Lys Lys Ala Asn Phe Glu Ile Gly Glu Glu Lys Thr Glu
   385                 390                 395                 400
   Ala Ser Glu Lys Val Glu Glu Gly Arg Ile Ile Arg Thr Asp Pro Gly
                   405                 410                 415
   Ala Gly Thr Gly Arg Lys Glu Gly Thr Lys Ile Asn Leu Val Val Ser
               420                 425                 430
   Ser Gly Lys Gln Ser Phe Gln Ile Ser Asn Tyr Val Gly Arg Lys Ser
           435                 440                 445
   Ser Asp Val Ile Ala Glu Leu Lys Glu Lys Lys Val Pro Asp Asn Leu
       450                 455                 460
   Ile Lys Ile Glu Glu Glu Glu Ser Asn Glu Ser Glu Ala Gly Thr Val
   465                 470                 475                 480
   Leu Lys Gln Ser Leu Pro Glu Gly Thr Thr Tyr Asp Leu Ser Lys Ala
                   485                 490                 495
   Thr Gln Ile Val Leu Thr Val Ala Lys Lys Ala Thr Thr Ile Gln Leu
               500                 505                 510
   Gly Asn Tyr Ile Gly Arg Asn Ser Thr Glu Val Ile Ser Glu Leu Lys
           515                 520                 525
   Gln Lys Lys Val Pro Glu Asn Leu Ile Lys Ile Glu Glu Glu Glu Ser
       530                 535                 540
   Ser Glu Ser Glu Pro Gly Thr Ile Met Lys Gln Ser Pro Gly Ala Gly
   545                 550                 555                 560
   Thr Thr Tyr Asp Val Ser Lys Pro Thr Gln Ile Val Leu Thr Val Ala
                   565                 570                 575
   Lys Lys Val Thr Ser Val Ala Met Pro Ser Tyr Ile Gly Ser Ser Leu
               580                 585                 590
   Glu Phe Thr Lys Asn Asn Leu Ile Gln Ile Val Gly Ile Lys Glu Ala
           595                 600                 605
   Asn Ile Glu Val Val Glu Val Thr Thr Ala Pro Ala Gly Ser Ala Glu
       610                 615                 620
   Gly Met Val Val Glu Gln Ser Pro Arg Ala Gly Glu Lys Val Asp Leu
   625                 630                 635                 640
   Asn Lys Thr Arg Val Lys Ile Ser Ile Tyr Lys Pro Lys Thr Thr Ser
                   645                 650                 655
   Ala Thr Pro

<210>  49
<211>  659
<212>  PRT
<213>  Streptococcus pneumoniae CDC 33 (serotype 15C)

<400>  49

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu

            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys

370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Leu Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser

                645                 650                 655

Ala Thr Pro

<210> 50
<211> 659
<212> PRT
<213> Streptococcus pneumoniae CDC 34 (serotype 15F)

<400> 50

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
                500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
                580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  51
<211>  659
<212>  PRT
<213>  Streptococcus pneumoniae CDC 37 (serotype 17A)

<400>  51

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Ser Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  52
<211>  659
<212>  PRT
<213>  Streptococcus pneumoniae CDC 38 (serotype 17F)

<400>  52

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  53
<211>  659
<212>  PRT
<213>  Streptococcus pneumoniae CDC 39 (serotype 18A)

<400>  53

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu

20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu

290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala

                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210> 54
<211> 659
<212> PRT
<213> Streptococcus pneumoniae CDC 41 (serotype 18C)

<400> 54

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Ala Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  55
<211>  659

<212>   PRT
<213>   Streptococcus pneumoniae CDC 42 (serotype 18F)

<400>   55

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  56
<211>  659
<212>  PRT
<213>  Streptococcus pneumoniae CDC 49 (serotype 22A)

<400>  56

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Glu Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210> 57
<211> 659
<212> PRT
<213> Streptococcus pneumoniae CDC 50 (serotype 22F)

<400> 57

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His

    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala

                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210> 58
<211> 659
<212> PRT
<213> Streptococcus pneumoniae CDC 53 (serotype 23F)

<400> 58

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Ile Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  59
<211>  659
<212>  PRT
<213>  Streptococcus pneumoniae CDC 55 (serotype 24B)

<400>  59

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Lys
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Ser Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  60
<211>  659
<212>  PRT
<213>  Streptococcus pneumoniae CDC 57 (serotype 25A)

<400>  60

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  61
<211>  659
<212>  PRT
<213>  Streptococcus pneumoniae CDC 58 (serotype 25F)

<400>  61

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu

130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Ala Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly

405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Ile Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  62

<211>  659
<212>  PRT
<213>  Streptococcus pneumoniae CDC 60 (serotype 28A)

<400>  62

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Asn Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Thr Val Thr Glu Glu Thr Tyr Gln Pro Gln Ala Pro
                325                 330                 335

Lys Lys His Arg Phe Lys Met Arg Tyr Leu Ile Leu Leu Ala Asn Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  63
<211>  659
<212>  PRT
<213>  Streptococcus pneumoniae CDC 62 (serotype 29)

<400>  63

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  64
<211>  659
<212>  PRT
<213>  Streptococcus pneumoniae CDC 65 (serotype 32F)

<400>  64

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro


<210>  65
<211>  659
<212>  PRT
<213>  Streptococcus pneumoniae CDC 66 (serotype 33A)

<400>  65

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala

50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Ser Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro

                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Ala Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala

595                    600                    605

Asn Ile Glu Val Val Glu Val Thr Thr Ala Pro Ala Gly Ser Val Glu
    610                    615                    620

Gly Met Val Val Glu Gln Ser Pro Arg Ala Gly Glu Lys Val Asp Leu
625                    630                    635                    640

Asn Lys Thr Arg Val Lys Ile Ser Ile Tyr Lys Pro Lys Thr Thr Ser
                645                    650                    655

Ala Thr Pro

<210>   66
<211>   659
<212>   PRT
<213>   Streptococcus pneumoniae CDC 67 (serotype 33B)

<400>   66

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Ser Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  67
<211>  659
<212>  PRT
<213>  Streptococcus pneumoniae CDC 68 (serotype 33C)

<400>  67

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Ala Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Asn Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  68
<211>  659
<212>  PRT
<213>  Streptococcus pneumoniae CDC 69 (serotype 33D)

<400>  68

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Lys Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>   69
<211>   659
<212>   PRT
<213>   Streptococcus pneumoniae CDC 70 (serotype 33F)

<400>   69

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Ser Ser Val Pro
225                 230                 235                 240

Gln Ala Leu Glu Asn Val Ile Ile Lys Ala Thr Ala Lys Lys Leu Thr

245                    250                    255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Ala Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys

515                    520                    525

Gln Lys Lys Val Pro Glu Asn Leu Ile Lys Ile Glu Glu Glu Glu Ser
    530                    535                    540

Ser Glu Ser Glu Pro Gly Thr Ile Met Lys Gln Ser Pro Gly Ala Gly
545                    550                    555                    560

Thr Thr Tyr Asp Val Ser Lys Pro Thr Gln Ile Val Leu Thr Val Ala
                565                    570                    575

Lys Lys Val Thr Ser Val Ala Met Pro Ser Tyr Ile Gly Ser Ser Leu
            580                    585                    590

Glu Phe Thr Lys Asn Asn Leu Ile Gln Ile Val Gly Ile Lys Glu Ala
        595                    600                    605

Asn Ile Glu Val Val Glu Val Thr Thr Ala Pro Ala Gly Ser Val Glu
    610                    615                    620

Gly Met Val Val Glu Gln Ser Pro Arg Ala Gly Glu Lys Val Asp Leu
625                    630                    635                    640

Asn Lys Thr Arg Val Lys Ile Ser Ile Tyr Lys Pro Lys Thr Thr Ser
                645                    650                    655

Ala Thr Pro

<210>  70
<211>  659
<212>  PRT
<213>  Streptococcus pneumoniae CDC 71 (serotype 34)

<400>  70

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  71
<211>  659
<212>  PRT
<213>  Streptococcus pneumoniae CDC 74 (serotype 35C)

<400>  71

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Ala Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  72
<211>  659
<212>  PRT
<213>  Streptococcus pneumoniae CDC 76 (serotype 36)

<400>  72

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Val Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  73
<211>  659
<212>  PRT
<213>  Streptococcus pneumoniae CDC 77 (serotype 37)

<400>  73

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val

                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser

435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Ala Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Ile
625                 630                 635                 640

Asn Lys Thr Arg Val Lys Ile Ser Ile Tyr Lys Pro Lys Thr Thr Ser
                645                 650                 655

Ala Thr Pro

<210>  74
<211>  659
<212>  PRT
<213>  Streptococcus pneumoniae CDC 78 (serotype 38)

<400>  74

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Ile Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  75
<211>  659
<212>  PRT
<213>  Streptococcus pneumoniae CDC 80 (serotype 40)

<400>  75

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Ser Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  76
<211>  659
<212>  PRT
<213>  Streptococcus pneumoniae CDC 81 (serotype 41A)

<400>  76

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Ala Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Asn Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  77
<211>  659
<212>  PRT
<213>  Streptococcus pneumoniae CDC 83 (serotype 42)

<400>  77

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu

                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Ala Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala

        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu

625                 630                 635                 640

Asn Lys Thr Arg Val Lys Ile Ser Ile Tyr Lys Pro Lys Thr Thr Ser
                645                 650                 655

Ala Thr Pro

<210>  78
<211>  659
<212>  PRT
<213>  Streptococcus pneumoniae CDC 84 (serotype 43)

<400>  78

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Glu Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro


<210>  79
<211>  659
<212>  PRT
<213>  Streptococcus pneumoniae CDC 86 (serotype 45)

<400>  79

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ile
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  80
<211>  659
<212>  PRT
<213>  Streptococcus pneumoniae CDC 89 (serotype 47F)

<400>  80

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Ser Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Ala Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Asn Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro


<210>  81
<211>  658
<212>  PRT
<213>  Streptococcus pneumoniae CDC 93 (serotype 9V)

<400>  81

Met Ile Gln Ile Gly Lys Ile Phe Ala Gly Arg Tyr Arg Ile Val Lys

1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Leu Gln His Phe
    210                 215                 220

Gln Lys Pro Leu Pro Ser Val Ile Ala Glu Asn Pro Ser Val Pro Gln
225                 230                 235                 240

Ala Leu Glu Asn Val Ile Ile Lys Ala Thr Ala Lys Lys Leu Thr Asn
                245                 250                 255

Arg Tyr Arg Ser Val Ser Glu Met Tyr Val Asp Leu Ser Ser Ser Leu
            260                 265                 270

Ser Tyr Asn Arg Arg Asn Glu Ser Lys Leu Ile Phe Asp Glu Thr Ser

275                 280                 285

Lys Ala Asp Thr Lys Thr Leu Pro Lys Val Ser Gln Ser Thr Leu Thr
    290                 295                 300

Ser Ile Pro Lys Val Gln Ala Gln Thr Glu His Lys Ser Ile Lys Asn
305                 310                 315                 320

Pro Ser Gln Ala Val Thr Glu Glu Thr Tyr Gln Pro Gln Ala Pro Lys
                325                 330                 335

Lys His Arg Phe Lys Met Arg Tyr Leu Ile Leu Leu Ala Ser Leu Val
            340                 345                 350

Leu Val Ala Ala Ser Leu Ile Trp Ile Leu Ser Arg Thr Pro Ala Thr
        355                 360                 365

Ile Ala Ile Pro Asp Val Ala Gly Gln Thr Val Ala Glu Ala Lys Ala
    370                 375                 380

Thr Leu Lys Lys Ala Asn Phe Glu Ile Gly Glu Glu Lys Thr Glu Ala
385                 390                 395                 400

Ser Glu Lys Val Glu Glu Gly Arg Ile Ile Arg Thr Asp Pro Gly Ala
                405                 410                 415

Gly Thr Gly Arg Lys Glu Gly Thr Lys Ile Asn Leu Val Val Ser Ser
            420                 425                 430

Gly Lys Gln Ser Phe Gln Ile Ser Asn Tyr Val Gly Arg Lys Ser Ser
        435                 440                 445

Asp Val Ile Ala Glu Leu Lys Glu Lys Lys Val Pro Asp Asn Leu Ile
    450                 455                 460

Lys Ile Glu Glu Glu Glu Ser Asn Glu Ser Glu Ala Gly Thr Val Leu
465                 470                 475                 480

Lys Gln Ser Leu Pro Glu Gly Thr Thr Tyr Asp Leu Ser Lys Ala Thr
                485                 490                 495

Gln Ile Ile Leu Thr Val Ala Lys Lys Ala Thr Thr Ile Gln Leu Gly
            500                 505                 510

Asn Tyr Ile Gly Arg Asn Ser Thr Glu Val Ile Ser Glu Leu Lys Gln
        515                 520                 525

Lys Lys Val Pro Glu Asn Leu Ile Lys Ile Glu Glu Glu Glu Ser Ser
    530                 535                 540

Glu Ser Glu Pro Gly Thr Ile Met Lys Gln Ser Pro Gly Ala Gly Thr

545                 550                 555                 560

Thr Tyr Asp Val Ser Lys Pro Thr Gln Ile Val Leu Thr Val Ala Lys
                565                 570                 575

Lys Val Thr Ser Val Ala Met Pro Ser Tyr Ile Gly Ser Ser Leu Glu
            580                 585                 590

Phe Thr Lys Asn Asn Leu Ile Gln Ile Val Gly Ile Lys Glu Ala Asn
        595                 600                 605

Ile Glu Val Val Glu Val Thr Thr Ala Pro Ala Gly Ser Ala Glu Gly
    610                 615                 620

Met Val Val Glu Gln Ser Pro Arg Ala Gly Glu Lys Val Asp Leu Asn
625                 630                 635                 640

Lys Thr Arg Val Lys Ile Ser Ile Tyr Lys Pro Lys Thr Thr Ser Ala
                645                 650                 655

Thr Pro


<210>  82
<211>  659
<212>  PRT
<213>  Streptococcus pneumoniae CDC 95 (serotype 14)

<400>  82

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  83
<211>  659
<212>  PRT
<213>  Streptococcus pneumoniae CDC 100 (serotype 14)

<400>  83

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  84
<211>  659
<212>  PRT
<213>  Streptococcus pneumoniae CDC 103 (serotype 19A)

<400>  84

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  85
<211>  659
<212>  PRT
<213>  Streptococcus pneumoniae CDC 104 (serotype 19F)

<400>  85

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly

195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val

465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  86
<211>  658
<212>  PRT
<213>  Streptococcus pneumoniae CDC 108 (serotype 14)

<400>  86

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Asp Val Ile Ala Glu Leu Lys Glu Lys Lys Val Pro Asp Asn Leu Ile
    450                 455                 460

Lys Ile Glu Glu Glu Glu Ser Asn Glu Ser Glu Ala Gly Thr Val Leu
465                 470                 475                 480

Lys Gln Ser Leu Pro Glu Gly Thr Thr Tyr Asp Leu Ser Lys Ala Thr
                485                 490                 495

Gln Ile Val Leu Thr Val Ala Lys Lys Ala Thr Thr Ile Gln Leu Gly
            500                 505                 510

Asn Tyr Ile Gly Arg Asn Ser Thr Glu Val Ile Ser Glu Leu Lys Gln
        515                 520                 525

Lys Lys Val Pro Glu Asn Leu Ile Lys Ile Glu Glu Glu Glu Ser Ser
    530                 535                 540

Glu Ser Glu Pro Gly Thr Ile Met Lys Gln Ser Pro Gly Ala Gly Thr
545                 550                 555                 560

Thr Tyr Asp Val Ser Lys Pro Thr Gln Ile Val Leu Thr Val Ala Lys
                565                 570                 575

Lys Val Thr Ser Val Ala Met Pro Ser Tyr Ile Gly Ser Ser Leu Glu
            580                 585                 590

Phe Thr Lys Asn Asn Leu Ile Gln Ile Val Gly Ile Lys Glu Ala Asn
        595                 600                 605

Ile Glu Val Val Glu Val Thr Thr Ala Pro Ala Gly Ser Val Glu Gly
    610                 615                 620

Met Val Val Glu Gln Ser Pro Arg Ala Gly Glu Lys Val Asp Leu Asn
625                 630                 635                 640

Lys Thr Arg Val Lys Ile Ser Ile Tyr Lys Pro Lys Thr Thr Ser Ala
                645                 650                 655

Thr Pro

<210>  87
<211>  659
<212>  PRT
<213>  Streptococcus pneumoniae CDC 109 (serotype 5)

<400>  87

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Ser Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  88
<211>  659
<212>  PRT
<213>  Streptococcus pneumoniae CDC 110 (serotype 6B)

<400>  88

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  89
<211>  659
<212>  PRT
<213>  Streptococcus pneumoniae CDC 111 (serotype 19F)

<400>  89

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His

        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Ser Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu

385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210> 90
<211> 659
<212> PRT
<213> Streptococcus pneumoniae CDC 112 (serotype 6B)

<400> 90

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  91
<211>  659
<212>  PRT
<213>  Streptococcus pneumoniae CDC 113 (serotype 6A)

<400>  91

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Ile Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  92
<211>  659
<212>  PRT
<213>  Streptococcus pneumoniae CDC 115 (serotype 15A)

<400>  92
   Met Ile Gln Ile Gly Lys Ile Phe Ala Gly Arg Tyr Arg Ile Val Lys
   1               5                   10                  15
   Gln Ile Gly Arg Gly Gly Met Ala Asp Val Tyr Leu Ala Lys Asp Leu
               20                  25                  30
   Ile Leu Asp Gly Glu Glu Val Ala Val Lys Val Leu Arg Thr Asn Tyr
           35                  40                  45
   Gln Thr Asp Pro Ile Ala Val Ala Arg Phe Gln Arg Glu Ala Arg Ala
       50                  55                  60
   Met Ala Asp Leu Asp His Pro His Ile Val Arg Ile Thr Asp Ile Gly
   65                  70                  75                  80
   Glu Glu Asp Gly Gln Gln Tyr Leu Ala Met Glu Tyr Val Ala Gly Leu
                   85                  90                  95
   Asp Leu Lys Arg Tyr Ile Lys Glu His Tyr Pro Leu Ser Asn Glu Glu
               100                 105                 110
   Ala Val Arg Ile Met Gly Gln Ile Leu Leu Ala Met Arg Leu Ala His
           115                 120                 125
   Thr Arg Gly Ile Val His Arg Asp Leu Lys Pro Gln Asn Ile Leu Leu
       130                 135                 140
   Thr Pro Asp Gly Thr Ala Lys Val Thr Asp Phe Gly Ile Ala Val Ala
   145                 150                 155                 160
   Phe Ala Glu Thr Ser Leu Thr Gln Thr Asn Ser Met Leu Gly Ser Val
                   165                 170                 175
   His Tyr Leu Ser Pro Glu Gln Ala Arg Gly Ser Lys Ala Thr Val Gln
               180                 185                 190
   Ser Asp Ile Tyr Ala Met Gly Ile Ile Phe Tyr Glu Met Leu Thr Gly
           195                 200                 205
   His Ile Pro Tyr Asp Gly Asp Ser Ala Val Thr Ile Ala Leu Gln His
       210                 215                 220
   Phe Gln Lys Pro Leu Pro Ser Val Ile Ala Glu Asn Pro Ser Val Pro
   225                 230                 235                 240
   Gln Ala Leu Glu Asn Val Ile Ile Lys Ala Thr Ala Lys Lys Leu Thr
                   245                 250                 255
   Asn Arg Tyr Arg Ser Val Ser Glu Met Tyr Val Asp Leu Ser Ser Ser
               260                 265                 270
   Leu Ser Tyr Asn Arg Arg Asn Glu Ser Lys Leu Ile Phe Asp Glu Thr
           275                 280                 285
   Ser Lys Ala Asp Thr Lys Thr Leu Pro Lys Val Ser Gln Ser Thr Leu
       290                 295                 300
   Thr Ser Ile Pro Lys Val Gln Ala Gln Thr Glu His Lys Ser Ile Lys
   305                 310                 315                 320
   Asn Pro Ser Gln Ala Val Thr Glu Glu Thr Tyr Gln Pro Gln Ala Pro
                   325                 330                 335
   Lys Lys His Arg Phe Lys Met Arg Tyr Leu Ile Leu Leu Ala Ser Leu
               340                 345                 350
   Val Leu Val Ala Ala Ser Leu Ile Trp Ile Leu Ser Arg Thr Pro Ala
           355                 360                 365
   Thr Ile Ala Ile Pro Asp Val Ala Gly Gln Thr Val Ala Glu Ala Lys
       370                 375                 380
   Ala Thr Leu Lys Lys Ala Asn Phe Glu Ile Gly Glu Glu Lys Thr Glu
   385                 390                 395                 400
   Ala Ser Glu Lys Val Glu Glu Gly Arg Ile Ile Arg Thr Asp Pro Gly
                   405                 410                 415
   Ala Gly Thr Gly Arg Lys Glu Gly Thr Lys Ile Asn Leu Val Val Ser
               420                 425                 430
   Ser Gly Lys Gln Ser Phe Gln Ile Ser Asn Tyr Val Gly Arg Lys Ser
           435                 440                 445
   Ser Asp Val Ile Ala Glu Leu Lys Glu Lys Lys Val Pro Asp Asn Leu
       450                 455                 460
   Ile Lys Ile Glu Glu Glu Glu Ser Asn Glu Ser Glu Ala Gly Thr Val
   465                 470                 475                 480
   Leu Lys Gln Ser Leu Pro Glu Gly Thr Thr Tyr Asp Leu Ser Lys Ala
                   485                 490                 495
   Thr Gln Ile Val Leu Thr Val Ala Lys Lys Ala Thr Thr Ile Gln Leu
               500                 505                 510
   Gly Asn Tyr Ile Gly Arg Asn Ser Thr Glu Val Ile Ser Glu Leu Lys
           515                 520                 525
   Gln Lys Lys Val Pro Glu Asn Leu Ile Lys Ile Glu Glu Glu Glu Ser
       530                 535                 540
   Ser Glu Ser Glu Pro Gly Thr Ile Met Lys Gln Ser Pro Gly Ala Gly
   545                 550                 555                 560
   Thr Thr Tyr Asp Val Ser Lys Pro Thr Gln Ile Val Leu Thr Val Ala
                   565                 570                 575
   Lys Lys Val Thr Ser Val Ala Met Pro Ser Tyr Ile Gly Ser Ser Leu
               580                 585                 590
   Glu Phe Thr Lys Asn Asn Leu Ile Gln Ile Val Gly Ile Lys Glu Ala
           595                 600                 605
   Asn Ile Glu Val Val Glu Val Thr Thr Ala Pro Ala Gly Ser Ala Glu
       610                 615                 620
   Gly Met Val Val Glu Gln Ser Pro Arg Ala Gly Glu Lys Val Asp Leu
   625                 630                 635                 640
   Asn Lys Thr Arg Val Lys Ile Ser Ile Tyr Lys Pro Lys Thr Thr Ser
                   645                 650                 655
   Ala Thr Pro

<210>  93
<211>  659
<212>  PRT
<213>  Streptococcus pneumoniae PJ-1293_1 (serotype 1)

<400>  93

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr

35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys

305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu

580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  94
<211>  644
<212>  PRT
<213>  Streptococcus pneumoniae I-33_3 (serotype 3)

<400>  94

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Phe Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Val Ile Lys Ala Thr Ala Lys Lys Leu Ser
                245                 250                 255

Asp Arg Tyr Gln Ser Val Ser Glu Met Tyr Val Asp Leu Ser Thr Ser
            260                 265                 270

Leu Ser Tyr Asn Arg Arg Asn Glu Pro Lys Leu Val Phe Asp Asp Ala
        275                 280                 285

Ser Lys Ala Asp Thr Lys Thr Leu Pro Lys Val Pro Gln Ser Thr Leu
    290                 295                 300

Thr Ser Ile Pro Lys Ala Pro Ala Gln Glu Glu Arg Pro Gln Pro Lys
305                 310                 315                 320

Lys Pro Thr Gln Pro Val Ala Glu Pro Ala Pro Ala Pro Lys Pro Ala
                325                 330                 335

Lys Lys Arg Lys Phe Lys Ala Arg Tyr Met Ile Leu Leu Ala Ser Leu
            340                 345                 350

Leu Leu Val Ala Ala Ser Leu Val Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Glu Ala Leu Lys Lys Ser Lys Phe Glu Ala Gly Glu Glu Lys Ser Glu
385                 390                 395                 400

Ala Ser Asp Thr Val Ala Glu Gly Arg Val Ile Arg Thr Asp Pro Glu
                405                 410                 415

Ala Gly Ser Gly Arg Lys Glu Gly Thr Lys Val Asn Leu Ile Val Ser
            420                 425                 430

Ser Gly Lys Gln Ser Phe Gln Leu Ser Asn Tyr Val Gly Arg Lys Tyr
        435                 440                 445

Thr Asp Val Val Ala Glu Leu Lys Glu Lys Lys Val Pro Glu Asn Leu
    450                 455                 460

Ile Lys Met Glu Glu Glu Glu Ser Ser Glu Ser Glu Pro Gly Thr Val
465                 470                 475                 480

Leu Arg Gln Thr Pro Ala Ser Gly Ser Thr Tyr Asp Leu Ser Lys Ala
                485                 490                 495

Thr Thr Ile Thr Leu Thr Val Ala Lys Lys Val Thr Ser Val Ser Met
            500                 505                 510

Pro Asn Tyr Ile Gly Ser Ser Leu Glu Phe Thr Lys Asn Asn Leu Thr
        515                 520                 525

Gln Ile Val Gly Val Lys Glu Ala Asn Ile Glu Val Val Glu Val Ser
    530                 535                 540

Asn Ala Pro Glu Gly Thr Ala Glu Gly Thr Val Val Ser Gln Thr Pro
545                 550                 555                 560

Arg Ala Gly Glu Leu Val Asp Leu Ala Ser Thr Arg Ile Lys Leu Ser
                565                 570                 575

Ile Tyr Lys Pro Lys Thr Pro Pro Ser Thr Ser Ser Ser Asn Pro Ala
            580                 585                 590

Gln Arg Gly Asn Gln Gly Ser Pro Thr Ser Pro Thr Gln Gly Asn Gln
        595                 600                 605

Gln Gly Asn Gln Gln Gly Asn Gln Gln Gly Asn Asn Pro Pro Thr Asn
    610                 615                 620

Gln Pro Val Arg Arg Phe Val Cys Tyr Thr Pro Glu Gln Cys Ser Ala
625                 630                 635                 640

Arg Asp Met Ser

<210>  95
<211>  659
<212>  PRT
<213>  Streptococcus pneumoniae PJ-1319_4 (serotype 4)

<400>  95

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ser Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  96
<211>  659
<212>  PRT
<213>  Streptococcus pneumoniae PJ-1321_4 (serotype 4)

<400>  96

Met Ser Pro Phe Arg Tyr Ile Phe Val Gly Phe Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

His Thr Asp Pro Ile Ala Val Ala Arg Phe Gln Arg Glu Ala Arg Ala
    50                  55                  60

Met Ala His Leu Asn His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Ile Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210> 97
<211> 659
<212> PRT
<213> Streptococcus pneumoniae PJ-1241_6A (serotype 6A)

<400> 97
   Met Ile Gln Ile Gly Lys Ile Phe Ala Gly Arg Tyr Arg Ile Val Lys
   1 5 10 15
   Gln Ile Gly Arg Gly Gly Met Ala Asp Val Tyr Leu Ala Lys Asp Leu
   20 25 30
   Ile Leu Asp Gly Glu Glu Val Ala Val Lys Val Leu Arg Thr Asn Tyr
   35 40 45
   Gln Thr Asp Pro Ile Ala Val Ala Arg Phe Gln Arg Glu Ala Arg Ala
   50 55 60
   Met Ala Asp Leu Asp His Pro His Ile Val Arg Ile Thr Asp Ile Gly
   65 70 75 80
   Glu Glu Asp Gly Gln Gln Tyr Leu Ala Met Glu Tyr Val Ala Gly Leu
   85 90 95
   Asp Leu Lys Arg Tyr Ile Lys Glu His Tyr Pro Leu Ser Asn Glu Glu
   100 105 110
   Ala Val Arg Ile Met Gly Gln Ile Leu Leu Ala Met Arg Leu Ala His
   115 120 125
   Thr Arg Gly Ile Val His Arg Asp Leu Lys Pro Gln Asn Ile Leu Leu
   130 135 140
   Thr Pro Asp Gly Thr Ala Lys Val Thr Asp Phe Gly Ile Ala Val Ala
   145 150 155 160
   Phe Ala Glu Thr Ser Leu Thr Gln Thr Asn Ser Met Leu Gly Ser Val
   165 170 175
   His Tyr Leu Ser Pro Glu Gln Ala Arg Gly Ser Lys Ala Thr Val Gln
   180 185 190
   Ser Asp Ile Tyr Ala Met Gly Ile Ile Phe Tyr Glu Met Leu Thr Gly
   195 200 205
   His Ile Pro Tyr Asp Gly Asp Ser Ala Val Thr Ile Ala Leu Gln His
   210 215 220
   Phe Gln Lys Pro Leu Pro Ser Val Ile Ala Glu Asn Pro Ser Val Pro
   225 230 235 240
   Gln Ala Leu Glu Asn Val Ile Ile Lys Ala Thr Ala Lys Lys Leu Thr
   245 250 255
   Asn Arg Tyr Arg Ser Val Ser Glu Met Tyr Val Asp Leu Ser Ser Ser
   260 265 270
   Leu Ser Tyr Asn Arg Arg Asn Glu Ser Lys Leu Ile Phe Asp Glu Thr
   275 280 285
   Ser Lys Ala Asp Thr Lys Thr Leu Pro Lys Val Ser Gln Ser Thr Leu
   290 295 300
   Thr Ser Ile Pro Lys Val Gln Ala Gln Thr Glu His Lys Ser Ile Lys
   305 310 315 320
   Asn Pro Ser Gln Ala Val Thr Glu Glu Thr Tyr Gln Pro Gln Ala Pro
   325 330 335
   Lys Lys His Arg Phe Lys Met Arg Tyr Leu Ile Leu Leu Ala Ser Leu
   340 345 350
   Val Leu Val Ala Ala Ser Leu Ile Trp Ile Leu Ser Arg Thr Pro Ala
   355 360 365
   Thr Ile Ala Ile Pro Asp Val Ala Gly Gln Thr Val Ala Glu Ala Lys
   370 375 380
   Ala Thr Leu Lys Lys Ala Asn Phe Glu Ile Gly Glu Glu Lys Thr Glu
   385 390 395 400
   Ala Ser Glu Lys Val Glu Glu Gly Arg Ile Ile Arg Thr Asp Pro Gly
   405 410 415
   Ala Gly Thr Gly Arg Lys Glu Gly Thr Lys Ile Asn Leu Val Val Ser
   420 425 430
   Ser Gly Lys Gln Ser Phe Gln Ile Ser Asn Tyr Val Gly Arg Lys Ser
   435 440 445
   Ser Asp Val Ile Ala Glu Leu Lys Glu Lys Lys Val Pro Asp Asn Leu
   450 455 460
   Ile Lys Ile Glu Glu Glu Glu Ser Asn Glu Ser Glu Ala Gly Thr Val
   465 470 475 480
   Leu Lys Gln Ser Leu Pro Glu Gly Thr Thr Tyr Asp Leu Ser Lys Ala
   485 490 495
   Thr Gln Ile Val Leu Thr Val Ala Lys Lys Ala Thr Thr Ile Gln Leu
   500 505 510
   Gly Asn Tyr Ile Gly Arg Asn Ser Thr Glu Val Ile Ser Glu Leu Lys
   515 520 525
   Gln Lys Lys Val Pro Glu Asn Leu Ile Lys Ile Glu Glu Glu Glu Ser
   530 535 540
   Ser Glu Ser Glu Pro Gly Thr Ile Met Lys Gln Ser Pro Gly Ala Gly
   545 550 555 560
   Thr Thr Tyr Asp Val Ser Lys Pro Thr Gln Ile Val Leu Thr Val Ala
   565 570 575
   Lys Lys Val Thr Ser Val Ala Met Pro Ser Tyr Ile Gly Ser Ser Leu
   580 585 590
   Glu Phe Thr Lys Asn Asn Leu Ile Gln Ile Val Gly Ile Lys Glu Ala
   595 600 605
   Asn Ile Glu Val Val Glu Val Thr Thr Ala Pro Ala Gly Ser Val Glu
   610 615 620
   Gly Met Val Val Glu Gln Ser Pro Arg Val Gly Glu Lys Val Asp Leu
   625 630 635 640
   Asn Lys Thr Arg Val Lys Ile Ser Ile Tyr Lys Pro Lys Thr Thr Ser
   645 650 655
   Ala Thr Pro

<210>  98
<211>  659
<212>  PRT
<213>  Streptococcus pneumoniae PJ-1296_7F (serotype 7F)

<400>  98

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Val Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Glu Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  99
<211>  659
<212>  PRT
<213>  Streptococcus pneumoniae PJ-1466_7F (serotype 7F)

<400>  99

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Val Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Glu Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  100
<211>  659
<212>  PRT
<213>  Streptococcus pneumoniae PJ-615_9A (serotype 9A)

<400>  100

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60
Met Ala Asp Leu Asp His Pro His Ile Val Arg Ile Thr Asp Ile Gly
65                  70                  75                  80
Glu Glu Asp Gly Gln Gln Tyr Leu Ala Met Glu Tyr Val Ala Gly Leu
                85                  90                  95
Asp Leu Lys Arg Tyr Ile Lys Glu His Tyr Pro Leu Ser Asn Glu Glu
            100                 105                 110
Ala Val Arg Ile Met Gly Gln Ile Leu Leu Ala Met Arg Leu Ala His
        115                 120                 125
Thr Arg Gly Ile Val His Arg Asp Leu Lys Pro Gln Asn Ile Leu Leu
    130                 135                 140
Thr Pro Asp Gly Thr Ala Lys Val Thr Asp Phe Gly Ile Ala Val Ala
145                 150                 155                 160
Phe Ala Glu Thr Ser Leu Thr Gln Thr Asn Ser Met Leu Gly Ser Val
                165                 170                 175
His Tyr Leu Ser Pro Glu Gln Ala Arg Gly Ser Lys Ala Thr Val Gln
            180                 185                 190
Ser Asp Ile Tyr Ala Met Gly Ile Ile Phe Tyr Glu Met Leu Thr Gly
        195                 200                 205
His Ile Pro Tyr Asp Gly Asp Ser Ala Val Thr Ile Ala Leu Gln His
    210                 215                 220
Phe Gln Lys Pro Leu Pro Ser Val Ile Ala Glu Asn Pro Ser Val Pro
225                 230                 235                 240
Gln Ala Leu Glu Asn Val Ile Ile Lys Ala Thr Ala Lys Lys Leu Thr
                245                 250                 255
Asn Arg Tyr Arg Ser Val Ser Glu Met Tyr Val Asp Leu Ser Ser Ser
            260                 265                 270
Leu Ser Tyr Asn Arg Arg Asn Glu Ser Lys Leu Ile Phe Asp Glu Thr
        275                 280                 285
Ser Lys Ala Asp Thr Lys Thr Leu Pro Lys Val Ser Gln Ser Thr Leu
    290                 295                 300
Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Ile Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  101
<211>  659
<212>  PRT
<213>  Streptococcus pneumoniae PJ-1255_9V (serotype 9V)

<400>  101

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val

                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser

435                    440                    445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Ile Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  102
<211>  659
<212>  PRT
<213>  Streptococcus pneumoniae PJ-1410_9V (serotype 9V)

<400>  102

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  103
<211>  659
<212>  PRT
<213>  Streptococcus pneumoniae PJ-1374_10C (serotype 10C)

<400>  103

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Ile Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  104
<211>  659
<212>  PRT
<213>  Streptococcus pneumoniae PJ02-912_12F (serotype 12F)

<400>  104
   Met Ile Gln Ile Gly Lys Ile Phe Ala Gly Arg Tyr Arg Ile Val Lys
   1               5                   10                  15
   Gln Ile Gly Arg Gly Gly Met Ala Asp Val Tyr Leu Ala Lys Asp Leu
               20                  25                  30
   Ile Leu Asp Gly Glu Glu Val Ala Val Lys Val Leu Arg Thr Asn Tyr
           35                  40                  45
   Gln Thr Asp Pro Ile Ala Val Ala Arg Phe Gln Arg Glu Ala Arg Ala
       50                  55                  60
   Met Ala Asp Leu Asp His Pro His Ile Val Arg Ile Thr Asp Ile Gly
   65                  70                  75                  80
   Glu Glu Asp Gly Gln Gln Tyr Leu Ala Met Glu Tyr Val Ala Gly Leu
                   85                  90                  95
   Asp Leu Lys Arg Tyr Ile Lys Glu His Tyr Pro Leu Ser Asn Glu Glu
               100                 105                 110
   Ala Val Arg Ile Met Gly Gln Ile Leu Leu Ala Met Arg Leu Ala His
           115                 120                 125
   Thr Arg Gly Ile Val His Arg Asp Leu Lys Pro Gln Asn Ile Leu Leu
       130                 135                 140
   Thr Pro Asp Gly Thr Ala Lys Val Thr Asp Phe Gly Ile Ala Val Ala
   145                 150                 155                 160
   Phe Ala Glu Thr Ser Leu Thr Gln Thr Asn Ser Met Leu Gly Ser Val
                   165                 170                 175
   His Tyr Leu Ser Pro Glu Gln Ala Arg Gly Ser Lys Ala Thr Val Gln
               180                 185                 190
   Ser Asp Ile Tyr Ala Met Gly Ile Ile Phe Tyr Glu Met Leu Thr Gly
           195                 200                 205
   His Ile Pro Tyr Asp Gly Asp Ser Ala Val Thr Ile Ala Leu Gln His
       210                 215                 220
   Phe Gln Lys Pro Leu Pro Ser Val Ile Ala Glu Asn Pro Ser Val Pro
   225                 230                 235                 240
   Gln Ala Leu Glu Asn Val Ile Ile Lys Ala Thr Ala Lys Lys Leu Thr
                   245                 250                 255
   Asn Arg Tyr Arg Ser Val Ser Glu Met Tyr Val Asp Leu Ser Ser Ser
               260                 265                 270
   Leu Ser Tyr Asn Arg Arg Asn Glu Ser Lys Leu Ile Phe Asp Glu Thr
           275                 280                 285
   Ser Lys Ala Asp Thr Lys Thr Leu Pro Lys Val Ser Gln Ser Thr Leu
       290                 295                 300
   Thr Ser Ile Pro Lys Val Gln Ala Gln Thr Glu His Lys Ser Ile Lys
   305                 310                 315                 320
   Asn Pro Ser Gln Ala Val Thr Glu Glu Thr Tyr Gln Pro Gln Ala Pro
                   325                 330                 335
   Lys Lys His Arg Phe Lys Met Arg Tyr Leu Ile Leu Leu Ala Ser Leu
               340                 345                 350
   Val Leu Val Ala Ala Ser Leu Ile Trp Ile Leu Ser Arg Thr Pro Ala
           355                 360                 365
   Thr Ile Ala Ile Pro Asp Val Ala Gly Gln Thr Val Ala Glu Ala Lys
       370                 375                 380
   Ala Thr Leu Lys Lys Ala Asn Phe Glu Ile Gly Glu Glu Lys Thr Glu
   385                 390                 395                 400
   Ala Ser Glu Lys Val Glu Glu Gly Arg Ile Ile Arg Thr Asp Pro Gly
                   405                 410                 415
   Ala Gly Thr Gly Arg Lys Glu Gly Thr Lys Ile Asn Leu Val Val Ser
               420                 425                 430
   Ser Gly Lys Gln Ser Phe Gln Ile Ser Asn Tyr Val Gly Arg Lys Ser
           435                 440                 445
   Ser Asp Val Ile Ala Glu Leu Lys Glu Lys Lys Val Pro Asp Asn Leu
       450                 455                 460
   Ile Lys Ile Glu Glu Glu Glu Ser Asn Glu Ser Glu Ala Gly Thr Val
   465                 470                 475                 480
   Leu Lys Gln Ser Leu Pro Glu Gly Thr Thr Tyr Asp Leu Ser Lys Ala
                   485                 490                 495
   Thr Gln Ile Val Leu Thr Val Ala Lys Lys Ala Thr Thr Ile Gln Leu
               500                 505                 510
   Gly Asn Tyr Ile Gly Arg Asn Ser Thr Glu Val Ile Ser Glu Leu Lys
   515 520 525
   Gln Lys Lys Val Pro Glu Asn Leu Ile Lys Ile Glu Glu Glu Glu Ser
   530 535 540
   Ser Glu Ser Glu Pro Gly Thr Ile Met Lys Gln Ser Pro Gly Ala Gly
   545 550 555 560
   Thr Thr Tyr Asp Val Ser Lys Pro Thr Gln Ile Val Leu Thr Val Ala
   565 570 575
   Lys Lys Val Thr Ser Val Ala Met Pro Ser Tyr Ile Gly Ser Ser Leu
   580 585 590
   Glu Phe Thr Lys Asn Asn Leu Ile Gln Ile Val Gly Ile Lys Glu Ala
   595 600 605
   Asn Ile Glu Val Val Glu Val Thr Thr Ala Pro Ala Gly Ser Ala Glu
   610 615 620
   Gly Met Val Val Glu Gln Ser Pro Arg Ala Gly Glu Lys Val Asp Leu
   625 630 635 640
   Asn Lys Thr Arg Val Lys Ile Ser Ile Tyr Lys Pro Lys Thr Thr Ser
   645 650 655
   Ala Thr Pro

<210>  105
<211>  659
<212>  PRT
<213>  Streptococcus pneumoniae PJ-1364_14 (serotype 14)

<400>  105

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu

                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala

355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Asp Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu

625                 630                 635                 640

Asn Lys Thr Arg Val Lys Ile Ser Ile Tyr Lys Pro Lys Thr Thr Ser
                645                 650                 655

Ala Thr Pro

<210>  106
<211>  659
<212>  PRT
<213>  Streptococcus pneumoniae PJ-1445_15B (serotype 15B)

<400>  106

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Ala Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  107
<211>  659
<212>  PRT
<213>  Streptococcus pneumoniae PJ-3_15C (serotype 15C)

<400>  107

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Ala Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  108
<211>  659
<212>  PRT
<213>  Streptococcus pneumoniae PJ-2449_16F (serotype 16F)

<400>  108

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro


<210>  109
<211>  659
<212>  PRT
<213>  Streptococcus pneumoniae PJ-1874_17A (serotype 17A)

<400>  109

Met Ile Gln Ile Gly Lys Ile Phe Ala Gly Arg Tyr Arg Ile Val Lys

1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr

        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly

545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  110
<211>  659
<212>  PRT
<213>  Streptococcus pneumoniae PJ-2129_17F (serotype 17F)

<400>  110

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Val Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Glu Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  111
<211>  659
<212>  PRT
<213>  Streptococcus pneumoniae PJ-2245_18C (serotype 18C)

<400>  111

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Ile Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  112
<211>  659
<212>  PRT
<213>  Streptococcus pneumoniae PJ-132_18F (serotype 18F)

<400>  112

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  113
<211>  659
<212>  PRT
<213>  Streptococcus pneumoniae PJ-1327_19A (serotype 19A)

<400>  113

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly

        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ser Glu Leu Lys Glu Lys Lys Val Pro Asp Asn Leu
    450                 455                 460

Ile Lys Ile Glu Glu Glu Glu Ser Asn Glu Ser Glu Ala Gly Thr Val

465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  114
<211>  659
<212>  PRT
<213>  Streptococcus pneumoniae PJ-1498_19A (serotype 19A)

<400>  114

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  115
<211>  659
<212>  PRT
<213>  Streptococcus pneumoniae PJ-1329_19F (serotype 19F)

<400>  115

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro


<210>  116
<211>  659
<212>  PRT
<213>  Streptococcus pneumoniae PJ-1494_19F (serotype 19F)

<400>  116

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  117
<211>  659
<212>  PRT
<213>  Streptococcus pneumoniae RP-2896_21 (serotype 21)

<400>  117

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His

115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu

385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ser Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  118
<211>  659
<212>  PRT
<213>  Streptococcus pneumoniae PJ-362_22A (serotype 22A)

<400>  118

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  119
<211>  633
<212>  PRT
<213>  Streptococcus pneumoniae PJ-1291_22F (serotype 22F)

<400>  119

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Phe Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Val Ile Lys Ala Thr Ala Lys Lys Leu Ser
                245                 250                 255

Asp Arg Tyr Gln Ser Val Ser Glu Met Tyr Val Asp Leu Ser Thr Ser
            260                 265                 270

Leu Ser Tyr Asn Arg Arg Asn Glu Pro Lys Leu Val Phe Asp Asp Ala
        275                 280                 285

Ser Lys Ala Asp Thr Lys Thr Leu Pro Lys Val Pro Gln Ser Thr Leu
    290                 295                 300

Thr Ser Ile Pro Lys Ala Pro Ala Gln Glu Glu Arg Pro Gln Pro Lys
305                 310                 315                 320

Lys Pro Thr Gln Pro Val Ala Glu Pro Ala Pro Ala Pro Lys Pro Ala
                325                 330                 335

Lys Lys Arg Lys Phe Lys Ala Arg Tyr Met Ile Leu Leu Ala Ser Leu
            340                 345                 350

Leu Leu Val Ala Ala Ser Leu Val Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Glu Ala Leu Lys Lys Ser Lys Phe Glu Ala Gly Glu Glu Lys Ser Glu
385                 390                 395                 400

Ala Ser Asp Thr Val Ala Glu Gly Arg Val Ile Arg Thr Asp Pro Glu
                405                 410                 415

Ala Gly Ser Gly Arg Lys Glu Gly Thr Lys Val Asn Leu Ile Val Ser
            420                 425                 430

Ser Gly Lys Gln Ser Phe Gln Leu Ser Asn Tyr Val Gly Arg Lys Tyr
        435                 440                 445

Thr Asp Val Val Ala Glu Leu Lys Glu Lys Lys Val Pro Glu Asn Leu
    450                 455                 460

Ile Lys Met Glu Glu Glu Glu Ser Asn Glu Ser Glu Pro Gly Ile Val
465                 470                 475                 480

Leu Arg Gln Thr Pro Ala Ser Gly Ser Thr Tyr Asp Leu Ser Lys Ala
                485                 490                 495

Thr Thr Ile Thr Leu Thr Val Ala Lys Lys Val Thr Ser Val Ser Met
            500                 505                 510

Pro Asn Tyr Ile Gly Ser Ser Leu Glu Phe Thr Lys Asn Asn Leu Thr
        515                 520                 525

Gln Ile Val Gly Val Lys Glu Ala Asn Ile Glu Val Val Glu Val Ser
    530                 535                 540

Thr Ala Pro Glu Gly Thr Ala Glu Gly Thr Val Val Asn Gln Thr Pro
545                 550                 555                 560

Arg Ala Gly Glu Gln Val Asp Leu Ala Ser Thr Arg Ile Lys Leu Ser
                565                 570                 575

Ile Tyr Lys Pro Lys Thr Pro Pro Ser Thr Ser Ser Ser Asn Pro Ala
            580                 585                 590

Gln Arg Gly Asn Gln Gly Ser Pro Thr Ser Pro Asn Gln Gly Asn Gln
        595                 600                 605

Gln Gly Asn Gln Gln Gly Asn Thr Pro Ser Ser Ser His Pro Ile Ser
    610                 615                 620

Ile Arg Cys Ala Asn Asn Ala Gly Ala
625                 630

<210>  120
<211>  659
<212>  PRT
<213>  Streptococcus pneumoniae PJ-1248_23F (serotype 23F)

<400>  120

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  121
<211>  659
<212>  PRT
<213>  Streptococcus pneumoniae PJ-2250_24A (serotype 24A)

<400>  121

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly

65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu

            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Ala Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu

610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  122
<211>  659
<212>  PRT
<213>  Streptococcus pneumoniae I-59_25F (serotype 25F)

<400>  122

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Ile Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  123
<211>  659
<212>  PRT
<213>  Streptococcus pneumoniae PJ-1857_27 (serotype 27)

<400>  123

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Leu Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  124
<211>  659
<212>  PRT
<213>  Streptococcus pneumoniae PJ-1287_31 (serotype 31)

<400>  124

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Ala Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  125
<211>  659
<212>  PRT
<213>  Streptococcus pneumoniae PJ-1283_33F (serotype 33F)

<400> 125

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Ser Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser

            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Ala Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser

530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  126
<211>  659
<212>  PRT
<213>  Streptococcus pneumoniae PJ-1176_34 (serotype 34)

<400>  126

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Val Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210> 127
<211> 659
<212> PRT
<213> Streptococcus pneumoniae PJ-1322_35F (serotype 35F)

<400> 127

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Ala Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro


<210>  128
<211>  659
<212>  PRT
<213>  Streptococcus pneumoniae PJ-1369_35F (serotype 35F)

<400>  128

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln
            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Pro Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu
    450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Ile Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro

<210>  129
<211>  659
<212>  PRT
<213>  Streptococcus pneumoniae PJ-2185_40 (serotype 40)

<400>  129

Met Ile Gln Ile Gly Lys Ile Phe Ala Gly Arg Tyr Arg Ile Val Lys
1               5                   10                  15

Gln Ile Gly Arg Gly Gly Met Ala Asp Val Tyr Leu Ala Lys Asp Leu
            20                  25                  30

Ile Leu Asp Gly Glu Glu Val Ala Val Lys Val Leu Arg Thr Asn Tyr
        35                  40                  45

Gln Thr Asp Pro Ile Ala Val Ala Arg Phe Gln Arg Glu Ala Arg Ala
    50                  55                  60

Met Ala Asp Leu Asp His Pro His Ile Val Arg Ile Thr Asp Ile Gly
65                  70                  75                  80

Glu Glu Asp Gly Gln Gln Tyr Leu Ala Met Glu Tyr Val Ala Gly Leu
                85                  90                  95

Asp Leu Lys Arg Tyr Ile Lys Glu His Tyr Pro Leu Ser Asn Glu Glu
            100                 105                 110

Ala Val Arg Ile Met Gly Gln Ile Leu Leu Ala Met Arg Leu Ala His
        115                 120                 125

Thr Arg Gly Ile Val His Arg Asp Leu Lys Pro Gln Asn Ile Leu Leu
    130                 135                 140

Thr Pro Asp Gly Thr Ala Lys Val Thr Asp Phe Gly Ile Ala Val Ala
145                 150                 155                 160

Phe Ala Glu Thr Ser Leu Thr Gln Thr Asn Ser Met Leu Gly Ser Val
                165                 170                 175

His Tyr Leu Ser Pro Glu Gln Ala Arg Gly Ser Lys Ala Thr Val Gln

            180                 185                 190

Ser Asp Ile Tyr Ala Met Gly Ile Ile Phe Tyr Glu Met Leu Thr Gly
        195                 200                 205

His Ile Pro Tyr Asp Gly Asp Ser Ala Val Thr Ile Ala Leu Gln His
    210                 215                 220

Phe Gln Lys Pro Leu Pro Ser Val Ile Ala Glu Asn Ser Ser Val Pro
225                 230                 235                 240

Gln Ala Leu Glu Asn Val Ile Ile Lys Ala Thr Ala Lys Lys Leu Thr
                245                 250                 255

Asn Arg Tyr Arg Ser Val Ser Glu Met Tyr Val Asp Leu Ser Ser Ser
            260                 265                 270

Leu Ser Tyr Asn Arg Arg Asn Glu Ser Lys Leu Ile Phe Asp Glu Thr
        275                 280                 285

Ser Lys Ala Asp Thr Lys Thr Leu Pro Lys Val Ser Gln Ser Thr Leu
    290                 295                 300

Thr Ser Ile Pro Lys Val Gln Ala Gln Thr Glu His Lys Ser Ile Lys
305                 310                 315                 320

Asn Pro Ser Gln Ala Val Thr Glu Glu Thr Tyr Gln Pro Gln Ala Pro
                325                 330                 335

Lys Lys His Arg Phe Lys Met Arg Tyr Leu Ile Leu Leu Ala Ser Leu
            340                 345                 350

Val Leu Val Ala Ala Ser Leu Ile Trp Ile Leu Ser Arg Thr Pro Ala
        355                 360                 365

Thr Ile Ala Ile Pro Asp Val Ala Gly Gln Thr Val Ala Glu Ala Lys
    370                 375                 380

Ala Thr Leu Lys Lys Ala Asn Phe Glu Ile Gly Glu Glu Lys Thr Glu
385                 390                 395                 400

Ala Ser Glu Lys Val Glu Glu Gly Arg Ile Ile Arg Thr Asp Pro Gly
                405                 410                 415

Ala Gly Thr Gly Arg Lys Glu Gly Thr Lys Ile Asn Leu Val Val Ser
            420                 425                 430

Ser Gly Lys Gln Ser Phe Gln Ile Ser Asn Tyr Val Gly Arg Lys Ser
        435                 440                 445

Ser Asp Val Ile Ala Glu Leu Lys Glu Lys Lys Val Pro Asp Asn Leu

450                 455                 460

Ile Lys Ile Glu Glu Glu Glu Ser Asn Glu Ser Glu Ala Gly Thr Val
465                 470                 475                 480

Leu Lys Gln Ser Leu Pro Glu Gly Thr Thr Tyr Asp Leu Ser Lys Ala
                485                 490                 495

Thr Gln Ile Val Leu Thr Val Ala Lys Lys Ala Thr Thr Ile Gln Leu
            500                 505                 510

Gly Asn Tyr Ile Gly Arg Asn Ser Thr Glu Val Ile Ser Glu Leu Lys
        515                 520                 525

Gln Lys Lys Val Pro Glu Asn Leu Ile Lys Ile Glu Glu Glu Glu Ser
    530                 535                 540

Ser Glu Ser Glu Pro Gly Thr Ile Met Lys Gln Ser Pro Gly Ala Gly
545                 550                 555                 560

Thr Thr Tyr Asp Val Ser Lys Pro Thr Gln Ile Val Leu Thr Val Ala
                565                 570                 575

Lys Lys Val Thr Ser Val Ala Met Pro Ser Tyr Ile Gly Ser Ser Leu
            580                 585                 590

Glu Phe Thr Lys Asn Asn Leu Ile Gln Ile Val Gly Ile Lys Glu Ala
        595                 600                 605

Asn Ile Glu Val Val Glu Val Thr Thr Ala Pro Ala Gly Ser Ala Glu
    610                 615                 620

Gly Met Val Val Glu Gln Ser Pro Arg Ala Gly Glu Lys Val Asp Leu
625                 630                 635                 640

Asn Lys Thr Arg Val Lys Ile Ser Ile Tyr Lys Pro Lys Thr Thr Ser
                645                 650                 655

Ala Thr Pro


<210>  130
<211>  309
<212>  PRT
<213>  Streptococcus pneumoniae CDC 5 (serotype 5)

<400>  130

Met Lys Lys Leu Gly Thr Leu Leu Val Leu Phe Leu Ser Ala Ile Ile
1               5                   10                  15

Leu Val Ala Cys Ala Ser Gly Lys Lys Asp Thr Thr Ser Gly Gln Lys
            20                  25                  30

Leu Lys Val Val Ala Thr Asn Ser Ile Ile Ala Asp Ile Thr Lys Asn
        35                  40                  45

Ile Ala Gly Asp Lys Ile Asp Leu His Ser Ile Val Pro Ile Gly Gln
    50                  55                  60

Asp Pro His Glu Tyr Glu Pro Leu Pro Glu Asp Val Lys Lys Thr Ser
65                  70                  75                  80

Glu Ala Asp Leu Ile Phe Tyr Asn Gly Ile Asn Leu Glu Thr Gly Gly
                85                  90                  95

Asn Ala Trp Phe Thr Lys Leu Val Glu Asn Ala Lys Lys Thr Glu Asn
            100                 105                 110

Lys Asp Tyr Phe Ala Val Ser Asp Gly Val Asp Val Ile Tyr Leu Glu
        115                 120                 125

Gly Gln Asn Glu Lys Gly Lys Glu Asp Pro His Ala Trp Leu Asn Leu
    130                 135                 140

Glu Asn Gly Ile Ile Phe Ala Lys Asn Ile Ala Lys Gln Leu Ser Ala
145                 150                 155                 160

Lys Asp Pro Asn Asn Lys Glu Phe Tyr Glu Lys Asn Leu Lys Glu Tyr
                165                 170                 175

Thr Asp Lys Leu Asp Lys Leu Asp Lys Glu Ser Lys Asp Lys Phe Asn
            180                 185                 190

Lys Ile Pro Ala Glu Lys Lys Leu Ile Val Thr Ser Glu Gly Ala Phe
        195                 200                 205

Lys Tyr Phe Ser Lys Ala Tyr Gly Val Pro Ser Ala Tyr Ile Trp Glu
    210                 215                 220

Ile Asn Thr Glu Ala Glu Gly Thr Pro Glu Gln Ile Lys Thr Leu Val
225                 230                 235                 240

Glu Lys Leu Arg Gln Thr Lys Val Pro Ser Leu Phe Val Glu Ser Ser
                245                 250                 255

Val Asp Asp Arg Pro Met Lys Thr Val Ser Gln Asp Thr Asn Ile Pro
            260                 265                 270

Ile Tyr Ala Gln Ile Phe Thr Asp Ser Ile Ala Glu Gln Gly Lys Glu
        275                 280                 285

Gly Asp Ser Tyr Tyr Ser Met Met Lys Tyr Asn Leu Asp Lys Ile Ala
    290                 295                 300

Glu Gly Leu Ala Lys
305

<210> 131
<211> 309
<212> PRT
<213> Streptococcus pneumoniae CDC 8 (serotype 7A)

<400> 131

Met Lys Lys Leu Gly Thr Leu Leu Val Leu Phe Leu Ser Ala Ile Ile
1               5                   10                  15

Leu Val Ala Cys Ala Ser Gly Lys Lys Asp Thr Thr Ser Gly Gln Lys
            20                  25                  30

Leu Lys Val Val Ala Thr Asn Ser Ile Ile Ala Asp Ile Thr Lys Asn
        35                  40                  45

Ile Ala Gly Asp Lys Ile Asp Leu His Ser Ile Val Pro Ile Gly Gln
    50                  55                  60

Asp Pro His Glu Tyr Glu Pro Leu Pro Glu Asp Val Lys Lys Thr Ser
65                  70                  75                  80

Glu Ala Asp Leu Ile Phe Tyr Asn Gly Ile Asn Leu Glu Thr Gly Gly
                85                  90                  95

Asn Ala Trp Phe Thr Lys Leu Val Glu Asn Ala Lys Lys Thr Glu Asn
            100                 105                 110

Lys Asp Tyr Phe Ala Val Ser Asp Gly Val Asp Val Ile Tyr Leu Glu
        115                 120                 125

Gly Gln Asn Glu Lys Gly Lys Glu Asp Pro His Ala Trp Leu Asn Leu
    130                 135                 140

Glu Asn Gly Ile Ile Phe Ala Lys Asn Ile Ala Lys Gln Leu Ser Ala
145                 150                 155                 160

Lys Asp Pro Asn Asn Lys Glu Phe Tyr Glu Lys Asn Leu Lys Glu Tyr
                165                 170                 175

Thr Asp Lys Leu Asp Lys Leu Asp Lys Glu Ser Lys Asp Lys Phe Asn
            180                 185                 190

Lys Ile Pro Ala Glu Lys Lys Leu Ile Val Thr Ser Glu Gly Ala Phe
        195                 200                 205

Lys Tyr Phe Ser Lys Ala Tyr Gly Val Pro Ser Ala Tyr Ile Trp Glu
    210                 215                 220

Ile Asn Thr Glu Lys Glu Gly Thr Pro Glu Gln Ile Lys Thr Leu Val
225                 230                 235                 240

Glu Lys Leu Arg Gln Thr Lys Val Pro Ser Leu Phe Val Glu Ser Ser
                245                 250                 255

Val Asp Asp Arg Pro Met Lys Thr Val Ser Gln Asp Thr Asn Ile Pro
            260                 265                 270

Ile Tyr Ala Gln Ile Phe Thr Asp Ser Ile Ala Glu Gln Gly Lys Glu
        275                 280                 285

Gly Asp Ser Tyr Tyr Ser Met Met Lys Tyr Asn Leu Asp Lys Ile Ala
    290                 295                 300

Glu Gly Leu Ala Lys
305

<210>  132
<211>  309
<212>  PRT
<213>  Streptococcus pneumoniae CDC 12 (serotype 8)

<400>  132

Met Lys Lys Leu Gly Thr Leu Leu Val Leu Phe Leu Ser Ala Ile Ile
1               5                   10                  15

Leu Val Ala Cys Ala Ser Gly Lys Lys Asp Thr Thr Ser Gly Gln Lys
            20                  25                  30

Leu Lys Val Val Ala Thr Asn Ser Ile Ile Ala Asp Ile Thr Lys Asn
        35                  40                  45

Ile Ala Gly Asp Lys Ile Asp Leu His Ser Ile Val Pro Ile Gly Gln
    50                  55                  60

Asp Pro His Glu Tyr Glu Pro Leu Pro Glu Asp Val Lys Lys Thr Ser
65                  70                  75                  80

Glu Ala Asn Leu Ile Phe Tyr Asn Gly Ile Asn Leu Glu Thr Gly Gly
                85                  90                  95

Asn Ala Trp Phe Thr Lys Leu Val Glu Asn Ala Lys Lys Thr Glu Asn
            100                 105                 110

Lys Asp Tyr Phe Ala Val Ser Asp Gly Val Asp Val Ile Tyr Leu Glu
        115                 120                 125

Gly Gln Asn Glu Lys Gly Lys Glu Asp Pro His Ala Trp Leu Asn Leu
    130                 135                 140

Glu Asn Gly Ile Ile Phe Ala Lys Asn Ile Ala Lys Gln Leu Ser Ala
145                 150                 155                 160

Lys Asp Pro Asn Asn Lys Glu Phe Tyr Glu Lys Asn Leu Lys Glu Tyr
                165                 170                 175

Thr Asp Lys Leu Asp Lys Leu Asp Lys Glu Ser Lys Asp Lys Phe Asn
            180                 185                 190

Lys Ile Pro Ala Glu Lys Lys Leu Ile Val Thr Ser Glu Gly Ala Phe
        195                 200                 205

Lys Tyr Phe Ser Lys Ala Tyr Gly Val Pro Ser Ala Tyr Ile Trp Glu
    210                 215                 220

Ile Asn Thr Glu Lys Glu Gly Thr Pro Glu Gln Ile Lys Thr Leu Val
225                 230                 235                 240

Glu Lys Leu Arg Gln Thr Lys Val Pro Ser Leu Phe Val Glu Ser Ser
                245                 250                 255

Val Asp Asp Arg Pro Met Lys Thr Val Ser Gln Asp Thr Asn Ile Pro
            260                 265                 270

Ile Tyr Ala Gln Ile Phe Thr Asp Ser Ile Ala Glu Gln Gly Lys Glu
        275                 280                 285

Gly Asp Ser Tyr Tyr Ser Met Met Lys Tyr Asn Leu Asp Lys Ile Ala
    290                 295                 300

Glu Gly Leu Ala Lys
305

<210>  133
<211>  309
<212>  PRT
<213>  Streptococcus pneumoniae CDC 33 (serotype 15C)

<400>  133

Met Lys Lys Leu Gly Thr Leu Leu Val Leu Phe Leu Ser Ala Ile Ile
1               5                   10                  15

Leu Val Ala Cys Ala Ser Gly Lys Lys Ala Thr Thr Ser Gly Gln Lys
            20                  25                  30

Leu Lys Val Val Ala Thr Asn Ser Ile Ile Ala Asp Ile Thr Lys Asn
        35                  40                  45

Ile Ala Gly Asp Lys Ile Asp Leu His Ser Ile Val Pro Ile Gly Gln
    50                  55                  60

Asp Pro His Glu Tyr Glu Pro Leu Pro Glu Asp Val Lys Lys Thr Ser

65                  70                  75                  80

Glu Ala Asp Leu Ile Phe Tyr Asn Gly Ile Asn Leu Glu Thr Gly Gly
                85                  90                  95

Asn Ala Trp Phe Thr Lys Leu Val Glu Asn Ala Lys Lys Thr Glu Asn
            100                 105                 110

Lys Asp Tyr Phe Ala Val Ser Asp Gly Val Asp Val Ile Tyr Leu Glu
        115                 120                 125

Gly Gln Asn Glu Lys Gly Lys Glu Asp Pro His Ala Trp Leu Asn Leu
    130                 135                 140

Glu Asn Gly Ile Ile Phe Ala Lys Asn Ile Ala Lys Gln Leu Ser Ala
145                 150                 155                 160

Lys Asp Pro Asn Asn Lys Glu Phe Tyr Glu Lys Asn Leu Lys Glu Tyr
                165                 170                 175

Thr Asp Lys Leu Asp Lys Leu Asp Lys Glu Ser Lys Asp Lys Phe Asn
            180                 185                 190

Lys Ile Pro Ala Glu Lys Lys Leu Ile Val Thr Ser Glu Gly Ala Phe
        195                 200                 205

Lys Tyr Phe Ser Lys Ala Tyr Gly Val Pro Ser Ala Tyr Ile Trp Glu
    210                 215                 220

Ile Asn Thr Glu Glu Glu Gly Thr Pro Glu Gln Ile Lys Thr Leu Val
225                 230                 235                 240

Glu Lys Leu Arg Gln Thr Lys Val Pro Ser Leu Phe Val Glu Ser Ser
                245                 250                 255

Val Asp Asp Arg Pro Met Lys Thr Val Ser Gln Asp Thr Asn Ile Pro
            260                 265                 270

Ile Tyr Ala Gln Ile Phe Thr Asp Ser Ile Ala Glu Gln Gly Lys Glu
        275                 280                 285

Gly Asp Ser Tyr Tyr Ser Met Met Lys Tyr Asn Leu Asp Lys Ile Ala
    290                 295                 300

Glu Gly Leu Ala Lys
305

<210>  134
<211>  309
<212>  PRT
<213>  Streptococcus pneumoniae CDC 36 (serotype 16F)

<400> 134

Met Lys Lys Leu Gly Thr Leu Leu Val Leu Phe Leu Ser Val Ile Ala
1               5                   10                  15

Leu Val Ala Cys Ala Ser Gly Lys Lys Asp Ala Ala Ser Gly Gln Lys
            20                  25                  30

Leu Lys Val Val Ala Thr Asn Ser Ile Ile Ala Asp Ile Thr Lys Asn
        35                  40                  45

Ile Ala Gly Asp Lys Ile Asp Leu His Ser Ile Val Pro Val Gly Gln
    50                  55                  60

Asp Pro His Glu Tyr Glu Pro Leu Pro Glu Asp Val Lys Lys Thr Ser
65                  70                  75                  80

Glu Ala Asp Leu Ile Phe Tyr Asn Gly Ile Asn Leu Glu Thr Gly Gly
                85                  90                  95

Asn Ala Trp Phe Thr Lys Leu Val Glu Asn Ala Lys Lys Thr Glu Asn
            100                 105                 110

Lys Asp Tyr Phe Ala Val Ser Glu Gly Val Asp Val Ile Tyr Leu Glu
        115                 120                 125

Gly Lys Asn Glu Lys Gly Lys Glu Asp Pro His Ala Trp Leu Asn Leu
    130                 135                 140

Glu Asn Gly Ile Ile Phe Ala Lys Asn Ile Ala Lys Gln Leu Ser Ala
145                 150                 155                 160

Lys Asp Pro Ser Asn Lys Glu Phe Tyr Glu Lys Asn Leu Lys Glu Tyr
                165                 170                 175

Thr Asp Lys Leu Asp Lys Leu Asp Lys Glu Ala Lys Ala Lys Phe Asn
            180                 185                 190

Asn Ile Pro Ala Glu Lys Lys Leu Ile Val Thr Ser Glu Gly Cys Phe
        195                 200                 205

Lys Tyr Phe Ser Lys Ala Tyr Gly Val Pro Ser Ala Tyr Ile Trp Glu
    210                 215                 220

Ile Asn Thr Glu Glu Glu Gly Thr Pro Glu Gln Ile Lys Thr Leu Val
225                 230                 235                 240

Glu Lys Leu Arg Gln Thr Lys Val Pro Ser Leu Phe Val Glu Ser Ser
                245                 250                 255

Val Asp Asp Arg Pro Met Lys Thr Val Ser Gln Asp Thr Asn Ile Pro
            260                 265                 270

Ile Tyr Ala Gln Ile Phe Thr Asp Ser Ile Ala Glu Gln Gly Lys Glu
        275                 280                 285

Gly Asp Ser Tyr Tyr Asn Met Met Lys Tyr Asn Leu Asp Lys Ile Ala
    290                 295                 300

Glu Gly Leu Ala Lys
305

<210>  135
<211>  309
<212>  PRT
<213>  Streptococcus pneumoniae CDC 38 (serotype 17F)

<400>  135

Met Lys Lys Leu Gly Thr Leu Leu Val Leu Phe Leu Ser Ala Ile Ile
1               5                   10                  15

Leu Val Ala Cys Ala Ser Gly Lys Lys Ala Thr Thr Ser Gly Gln Lys
            20                  25                  30

Leu Lys Val Val Ala Thr Asn Ser Ile Ile Ala Asp Ile Thr Lys Asn
        35                  40                  45

Ile Ala Gly Asp Lys Ile Asp Leu His Ser Ile Val Pro Ile Gly Gln
    50                  55                  60

Asp Pro His Glu Tyr Glu Pro Leu Pro Glu Asp Val Lys Lys Thr Ser
65                  70                  75                  80

Glu Ala Asp Leu Ile Phe Tyr Asn Gly Ile Asn Leu Glu Thr Gly Gly
                85                  90                  95

Asn Ala Trp Phe Thr Lys Leu Val Glu Asn Ala Lys Lys Thr Glu Asn
            100                 105                 110

Lys Asp Tyr Phe Ala Val Ser Asp Gly Val Asp Val Ile Tyr Leu Glu
        115                 120                 125

Gly Gln Asn Glu Lys Gly Lys Glu Asp Pro His Ala Trp Leu Asn Leu
    130                 135                 140

Glu Asn Gly Ile Ile Phe Ala Lys Asn Ile Ala Lys Gln Leu Ser Ala
145                 150                 155                 160

Lys Asp Pro Asn Asn Lys Glu Phe Tyr Glu Lys Asn Leu Lys Glu Tyr
                165                 170                 175

Thr Asp Lys Leu Asp Lys Leu Asp Lys Glu Ser Lys Asp Lys Phe Asn
            180                 185                 190

Lys Ile Pro Ala Glu Lys Lys Leu Ile Val Thr Ser Glu Gly Ala Phe
        195                 200                 205

Lys Tyr Phe Ser Lys Ala Tyr Gly Val Pro Ser Ala Tyr Ile Trp Glu
    210                 215                 220

Ile Asn Thr Glu Glu Glu Gly Thr Pro Glu Gln Ile Lys Thr Leu Val
225                 230                 235                 240

Glu Lys Leu Arg Gln Thr Lys Val Pro Ser Leu Phe Val Glu Ser Ser
                245                 250                 255

Val Asp Asp Arg Pro Met Lys Thr Val Ser Gln Asp Thr Asn Ile Pro
            260                 265                 270

Ile Tyr Ala Gln Ile Phe Thr Asp Ser Ile Ala Glu Gln Gly Lys Glu
        275                 280                 285

Gly Asp Ser Tyr Tyr Ser Met Met Lys Tyr Asn Leu Asp Lys Ile Ala
    290                 295                 300

Glu Gly Leu Ala Lys
305

<210>  136
<211>  309
<212>  PRT
<213>  Streptococcus pneumoniae CDC 56 (serotype 24F)

<400>  136

Met Lys Lys Leu Gly Thr Leu Leu Val Leu Phe Leu Ser Ala Ile Ile
1               5                   10                  15

Leu Val Ala Cys Ala Ser Gly Lys Lys Asp Thr Thr Ser Gly Gln Lys
            20                  25                  30

Leu Lys Val Val Ala Thr Asn Ser Ile Ile Ala Asp Ile Thr Lys Asn
        35                  40                  45

Ile Ala Gly Asp Lys Ile Asp Leu His Ser Ile Val Pro Ile Gly Gln
    50                  55                  60

Asp Pro His Glu Tyr Glu Pro Leu Pro Glu Asp Val Lys Lys Thr Ser
65                  70                  75                  80

Glu Ala Asp Leu Ile Phe Tyr Asn Gly Ile Asn Leu Glu Thr Gly Gly
                85                  90                  95

Asn Ala Trp Phe Thr Lys Leu Val Glu Asn Ala Lys Lys Thr Glu Asn
            100                 105                 110

Lys Asp Tyr Phe Ala Val Ser Asp Gly Val Asp Val Ile Tyr Leu Glu
        115                 120                 125

Gly Gln Asn Glu Lys Gly Lys Glu Asp Pro His Ala Trp Leu Asn Leu
    130                 135                 140

Glu Asn Gly Ile Ile Phe Ala Lys Asn Ile Ala Lys Gln Leu Ser Ala
145                 150                 155                 160

Lys Asp Pro Asn Asn Lys Glu Phe Tyr Glu Lys Asn Leu Lys Glu Tyr
                165                 170                 175

Thr Asp Lys Leu Asp Lys Leu Asp Lys Glu Ser Lys Asp Lys Phe Asn
            180                 185                 190

Lys Ile Pro Ala Glu Lys Lys Leu Ile Val Thr Ser Glu Gly Ala Phe
        195                 200                 205

Lys Tyr Phe Ser Lys Ala Tyr Gly Val Pro Ser Ala Tyr Ile Trp Glu
    210                 215                 220

Ile Asn Thr Glu Lys Glu Gly Thr Pro Glu Gln Ile Lys Thr Leu Val
225                 230                 235                 240

Glu Lys Leu Arg Gln Thr Lys Val Pro Ser Leu Phe Val Glu Ser Ser
                245                 250                 255

Val Asp Asp Arg Pro Met Lys Thr Val Ser Gln Asp Thr Asn Ile Pro
            260                 265                 270

Ile Tyr Ala Gln Ile Phe Thr Asp Ser Ile Ala Glu Gln Gly Lys Glu
        275                 280                 285

Gly Asp Ser Tyr Tyr Ser Met Met Lys Tyr Asn Leu Asp Lys Ile Ala
    290                 295                 300

Glu Gly Leu Ala Lys
305

<210> 137
<211> 309
<212> PRT
<213> Streptococcus pneumoniae CDC 58 (serotype 25F)

<400> 137

Met Lys Lys Leu Gly Thr Leu Leu Val Leu Phe Leu Ser Val Ile Ala
1               5                   10                  15

Leu Val Ala Cys Ala Ser Gly Lys Lys Asp Ala Ala Ser Gly Gln Lys
            20                  25                  30

Leu Lys Val Val Ala Thr Asn Ser Ile Ile Ala Asp Ile Thr Lys Asn

        35                  40                  45

Ile Ala Gly Asp Lys Ile Asp Leu His Ser Ile Val Pro Val Gly Gln
    50                  55                  60

Asp Pro His Glu Tyr Glu Pro Leu Pro Glu Asp Val Lys Lys Thr Ser
65                  70                  75                  80

Glu Ala Asp Leu Ile Phe Tyr Asn Gly Ile Asn Leu Glu Thr Gly Gly
                85                  90                  95

Asn Ala Trp Phe Thr Lys Leu Val Glu Asn Ala Lys Lys Thr Glu Asn
            100                 105                 110

Lys Asp Tyr Phe Ala Val Ser Glu Gly Val Asp Val Ile Tyr Leu Glu
        115                 120                 125

Gly Lys Asn Glu Lys Gly Lys Glu Asp Pro His Ala Trp Leu Asn Leu
    130                 135                 140

Glu Asn Gly Ile Ile Phe Ala Lys Asn Ile Ala Lys Gln Leu Ser Ala
145                 150                 155                 160

Lys Asp Pro Ser Asn Lys Glu Phe Tyr Glu Lys Asn Leu Lys Glu Tyr
                165                 170                 175

Thr Asp Lys Leu Asp Lys Leu Asp Lys Glu Ala Lys Ala Lys Phe Asn
            180                 185                 190

Asn Ile Pro Ala Glu Lys Lys Leu Ile Val Thr Ser Glu Gly Cys Phe
        195                 200                 205

Lys Tyr Phe Ser Lys Ala Tyr Gly Val Pro Ser Ala Tyr Ile Trp Glu
    210                 215                 220

Ile Asn Thr Glu Glu Glu Gly Thr Pro Glu Gln Ile Lys Thr Leu Val
225                 230                 235                 240

Glu Lys Leu Arg Gln Thr Lys Val Pro Ser Leu Phe Val Glu Ser Ser
                245                 250                 255

Val Asp Asp Arg Pro Met Lys Thr Val Ser Gln Asp Thr Asn Ile Pro
            260                 265                 270

Ile Tyr Ala Gln Ile Phe Thr Asp Ser Ile Ala Glu Gln Gly Lys Glu
        275                 280                 285

Gly Asp Ser Tyr Tyr Asn Met Met Lys Tyr Asn Leu Asp Lys Ile Ala
    290                 295                 300

Glu Gly Leu Ala Lys

305

<210>  138
<211>  309
<212>  PRT
<213>  Streptococcus pneumoniae CDC 66 (serotype 33A)

<400>  138

Met Lys Lys Leu Gly Thr Leu Leu Val Leu Phe Leu Ser Ala Ile Ile
1               5                   10                  15

Leu Val Ala Cys Ala Ser Gly Lys Lys Asp Thr Thr Ser Gly Gln Lys
            20                  25                  30

Leu Lys Val Val Ala Thr Asn Ser Ile Ile Ala Asp Ile Thr Lys Asn
        35                  40                  45

Ile Ala Gly Asp Lys Ile Asp Leu His Ser Ile Val Pro Ile Gly Gln
    50                  55                  60

Asp Pro His Glu Tyr Glu Pro Leu Pro Glu Asp Val Lys Lys Thr Ser
65                  70                  75                  80

Glu Ala Asn Leu Ile Phe Tyr Asn Gly Ile Asn Leu Glu Thr Gly Gly
                85                  90                  95

Asn Ala Trp Phe Thr Lys Leu Val Glu Asn Ala Lys Lys Thr Glu Asn
            100                 105                 110

Lys Asp Tyr Phe Ala Val Ser Asp Gly Val Asp Val Ile Tyr Leu Glu
        115                 120                 125

Gly Gln Asn Glu Lys Gly Lys Glu Asp Pro His Ala Trp Leu Asn Leu
    130                 135                 140

Glu Asn Gly Ile Ile Phe Ala Lys Asn Ile Ala Lys Gln Leu Ser Ala
145                 150                 155                 160

Lys Asp Pro Asn Asn Lys Glu Phe Tyr Glu Lys Asn Leu Lys Glu Tyr
                165                 170                 175

Thr Asp Lys Leu Asp Lys Leu Asp Lys Glu Ser Lys Asp Lys Phe Asn
            180                 185                 190

Lys Ile Pro Ala Glu Lys Lys Leu Ile Val Thr Ser Glu Gly Ala Phe
        195                 200                 205

Lys Tyr Phe Ser Lys Ala Tyr Gly Val Pro Ser Ala Tyr Ile Trp Glu
    210                 215                 220

Ile Asn Thr Glu Glu Glu Gly Thr Pro Glu Gln Ile Lys Thr Leu Val
225                 230                 235                 240

Glu Lys Leu Arg Gln Thr Lys Val Pro Ser Leu Phe Val Glu Ser Ser
                245                 250                 255

Val Asp Asp Arg Pro Met Lys Thr Val Ser Gln Asp Thr Asn Ile Pro
            260                 265                 270

Ile Tyr Ala Gln Ile Phe Thr Asp Ser Ile Ala Glu Gln Gly Lys Glu
        275                 280                 285

Gly Asp Ser Tyr Tyr Ser Met Met Lys Tyr Asn Leu Asp Lys Ile Ala
    290                 295                 300

Glu Gly Leu Ala Lys
305

<210>  139
<211>  309
<212>  PRT
<213>  Streptococcus pneumoniae CDC 69 (serotype 33D)

<400>  139

Met Lys Lys Leu Gly Thr Leu Leu Val Leu Phe Leu Ser Ala Ile Ile
1               5                   10                  15

Leu Val Ala Cys Ala Ser Gly Lys Lys Asp Thr Thr Ser Gly Gln Lys
            20                  25                  30

Leu Lys Val Val Ala Thr Asn Ser Ile Ile Ala Asp Ile Thr Lys Asn
        35                  40                  45

Ile Ala Gly Asp Lys Ile Asp Leu His Ser Ile Val Pro Ile Gly Gln
    50                  55                  60

Asp Pro His Glu Tyr Glu Pro Leu Pro Glu Asp Val Lys Lys Thr Ser
65                  70                  75                  80

Glu Ala Asp Leu Ile Phe Tyr Asn Gly Ile Asn Leu Glu Thr Gly Gly
                85                  90                  95

Asn Ala Trp Phe Thr Lys Leu Val Glu Asn Ala Lys Lys Thr Glu Asn
            100                 105                 110

Lys Asp Tyr Phe Ala Val Ser Asp Gly Val Asp Val Ile Tyr Leu Glu
        115                 120                 125

Gly Gln Asn Glu Lys Gly Lys Glu Asp Pro His Ala Trp Leu Asn Leu
    130                 135                 140

Glu Asn Gly Ile Ile Phe Ala Lys Asn Ile Ala Lys Gln Leu Ser Ala
145                 150                 155                 160

Lys Asp Pro Asn Asn Lys Glu Phe Tyr Glu Lys Asn Leu Lys Glu Tyr
                165                 170                 175

Thr Asp Lys Leu Asp Lys Leu Asp Lys Glu Ser Lys Asp Lys Phe Asn
            180                 185                 190

Lys Ile Pro Ala Glu Lys Lys Leu Ile Val Thr Ser Glu Gly Ala Phe
        195                 200                 205

Lys Tyr Phe Ser Lys Ala Tyr Gly Val Pro Ser Ala Tyr Ile Trp Glu
    210                 215                 220

Ile Asn Thr Glu Glu Glu Gly Thr Pro Glu Gln Ile Lys Thr Leu Val
225                 230                 235                 240

Glu Lys Leu Arg Gln Thr Lys Val Pro Ser Leu Phe Val Glu Ser Ser
                245                 250                 255

Val Asp Asp Arg Pro Met Lys Thr Val Ser Gln Asp Thr Asn Ile Pro
            260                 265                 270

Ile Tyr Ala Gln Ile Phe Thr Asp Ser Ile Ala Glu Gln Gly Lys Glu
        275                 280                 285

Gly Asp Ser Tyr Tyr Ser Met Met Lys Tyr Asn Leu Asp Lys Ile Ala
    290                 295                 300

Glu Gly Leu Ala Lys
305

<210>  140
<211>  309
<212>  PRT
<213>  Streptococcus pneumoniae CDC 74 (serotype 35C)

<400>  140

Met Lys Lys Leu Gly Thr Leu Leu Val Leu Phe Leu Ser Ala Ile Ile
1               5                   10                  15

Leu Val Ala Cys Ala Ser Gly Lys Lys Asp Thr Thr Ser Gly Gln Lys
            20                  25                  30

Leu Lys Val Val Ala Thr Asn Ser Ile Ile Ala Asp Ile Thr Lys Asn
        35                  40                  45

Ile Ala Gly Asp Lys Ile Asp Leu His Ser Ile Val Pro Ile Gly Gln
    50                  55                  60

Asp Pro His Glu Tyr Glu Pro Leu Pro Glu Asp Val Lys Lys Thr Ser
65                  70                  75                  80

Glu Ala Asp Leu Ile Phe Tyr Asn Gly Ile Asn Leu Glu Thr Gly Gly
                85                  90                  95

Asn Ala Trp Phe Thr Lys Leu Val Glu Asn Ala Lys Lys Thr Glu Asn
            100                 105                 110

Lys Asp Tyr Phe Ala Val Ser Asp Gly Val Asp Val Ile Tyr Leu Glu
        115                 120                 125

Gly Gln Asn Glu Lys Gly Lys Glu Asp Pro His Ala Trp Leu Asn Leu
    130                 135                 140

Glu Asn Gly Ile Ile Phe Ala Lys Asn Ile Ala Lys Gln Leu Ser Ala
145                 150                 155                 160

Lys Asp Pro Asn Asn Lys Glu Phe Tyr Glu Lys Asn Leu Lys Glu Tyr
                165                 170                 175

Thr Asp Lys Leu Asp Lys Leu Asp Lys Glu Ser Lys Asp Lys Phe Asn
            180                 185                 190

Lys Ile Pro Ala Glu Lys Lys Leu Ile Val Thr Ser Glu Gly Ala Phe
        195                 200                 205

Lys Tyr Phe Ser Lys Ala Tyr Gly Val Pro Ser Ala Tyr Ile Trp Glu
    210                 215                 220

Ile Asn Thr Glu Glu Glu Gly Thr Pro Glu Gln Ile Lys Thr Leu Val
225                 230                 235                 240

Glu Lys Leu Arg Gln Thr Lys Val Pro Ser Leu Phe Ala Glu Ser Ser
                245                 250                 255

Val Asp Asp Arg Pro Met Lys Thr Val Ser Gln Asp Thr Asn Ile Pro
            260                 265                 270

Ile Tyr Ala Gln Ile Phe Thr Asp Ser Ile Ala Glu Gln Gly Lys Glu
        275                 280                 285

Gly Asp Ser Tyr Tyr Ser Met Met Lys Tyr Asn Leu Asp Lys Ile Ala
    290                 295                 300

Glu Gly Leu Ala Lys
305

<210>  141
<211>  309
<212>  PRT
<213>  Streptococcus pneumoniae CDC 96 (serotype 19A)

<400>  141

Met Lys Lys Leu Gly Thr Leu Leu Val Leu Phe Leu Ser Ala Ile Ile

1               5                   10                  15

Leu Val Ala Cys Ala Ser Gly Lys Lys Asp Thr Thr Ser Gly Gln Lys
            20                  25                  30

Leu Lys Val Val Ala Thr Asn Ser Ile Ile Ala Asp Ile Thr Lys Asn
        35                  40                  45

Ile Ala Gly Asp Lys Ile Asp Leu His Ser Ile Val Pro Ile Gly Gln
    50                  55                  60

Asp Pro His Glu Tyr Glu Pro Leu Pro Glu Asp Val Lys Lys Thr Ser
65                  70                  75                  80

Glu Ala Asp Leu Ile Phe Tyr Asn Gly Ile Asn Leu Glu Thr Gly Gly
                85                  90                  95

Asn Ala Trp Phe Thr Lys Leu Val Glu Asn Ala Lys Lys Thr Glu Asn
            100                 105                 110

Lys Asp Tyr Phe Ala Val Ser Asp Gly Val Asp Val Ile Tyr Leu Glu
        115                 120                 125

Gly Gln Asn Glu Lys Gly Lys Glu Asp Pro His Ala Trp Leu Asn Leu
    130                 135                 140

Glu Asn Gly Ile Ile Phe Ala Lys Asn Ile Ala Lys Gln Leu Ser Ala
145                 150                 155                 160

Lys Asp Pro Asn Asn Lys Glu Phe Tyr Glu Lys Asn Leu Lys Glu Tyr
                165                 170                 175

Thr Asp Lys Leu Asp Lys Leu Asp Lys Glu Ser Lys Asn Lys Phe Asn
            180                 185                 190

Lys Ile Pro Ala Glu Lys Lys Leu Ile Val Thr Ser Glu Gly Ala Phe
        195                 200                 205

Lys Tyr Phe Ser Lys Ala Tyr Gly Val Pro Ser Ala Tyr Ile Trp Glu
    210                 215                 220

Ile Asn Thr Glu Glu Glu Gly Thr Pro Glu Gln Ile Lys Thr Leu Val
225                 230                 235                 240

Glu Lys Leu Arg Gln Thr Lys Val Pro Ser Leu Phe Val Glu Ser Ser
                245                 250                 255

Val Asp Asp Arg Pro Met Lys Thr Val Ser Gln Asp Thr Asn Ile Pro
            260                 265                 270

Ile Tyr Ala Gln Ile Phe Thr Asp Ser Ile Ala Glu Gln Gly Lys Glu

275                    280                    285

Gly Asp Ser Tyr Tyr Ser Met Met Lys Tyr Asn Leu Asp Lys Ile Ala
    290                    295                    300

Glu Gly Leu Ala Lys
305

<210>  142
<211>  309
<212>  PRT
<213>  Streptococcus pneumoniae CDC 114 (serotype 35B)

<400>  142

Met Lys Lys Leu Gly Thr Leu Leu Val Leu Phe Leu Ser Ala Ile Ile
1               5                   10                  15

Leu Val Ala Cys Ala Ser Gly Lys Lys Asp Thr Thr Ser Gly Gln Lys
            20                  25                  30

Leu Lys Val Val Ala Thr Asn Ser Ile Ile Ala Asp Ile Thr Lys Asn
        35                  40                  45

Ile Ala Gly Asp Lys Ile Asp Leu His Ser Ile Val Pro Ile Gly Gln
    50                  55                  60

Asp Pro His Glu Tyr Glu Pro Leu Pro Glu Asp Val Lys Lys Thr Ser
65                  70                  75                  80

Glu Ala Asp Leu Ile Phe Tyr Asn Gly Ile Asn Leu Glu Thr Gly Gly
                85                  90                  95

Asn Ala Trp Phe Thr Lys Leu Val Glu Asn Ala Lys Lys Thr Glu Asn
            100                 105                 110

Lys Asp Tyr Phe Ala Val Ser Asp Gly Val Asp Val Ile Tyr Leu Glu
        115                 120                 125

Gly Gln Asn Glu Lys Gly Lys Glu Asp Pro His Ala Trp Leu Asn Leu
    130                 135                 140

Glu Asn Gly Ile Ile Phe Ala Lys Asn Ile Ala Lys Gln Leu Ser Ala
145                 150                 155                 160

Lys Asp Pro Asn Asn Lys Glu Phe Tyr Glu Lys Asn Leu Lys Glu Tyr
                165                 170                 175

Thr Asp Lys Leu Asp Lys Leu Asp Lys Glu Ser Lys Asp Lys Phe Asn
            180                 185                 190

Lys Ile Ser Ala Glu Lys Lys Leu Ile Val Thr Ser Glu Gly Ala Phe
        195                 200                 205

Lys Tyr Phe Ser Lys Ala Tyr Gly Val Pro Ser Ala Tyr Ile Trp Glu
    210                 215                 220

Ile Asn Thr Glu Glu Glu Gly Thr Pro Glu Gln Ile Lys Thr Leu Val
225                 230                 235                 240

Glu Lys Leu Arg Gln Thr Lys Val Pro Ser Leu Phe Val Glu Ser Ser
                245                 250                 255

Val Asp Asp Arg Pro Met Lys Thr Val Ser Gln Asp Thr Asn Ile Pro
            260                 265                 270

Ile Tyr Ala Gln Ile Phe Thr Asp Ser Ile Ala Glu Gln Gly Lys Glu
        275                 280                 285

Gly Asp Ser Tyr Tyr Ser Met Met Lys Tyr Asn Leu Asp Lys Ile Ala
    290                 295                 300

Glu Gly Leu Ala Lys
305

<210>  143
<211>  309
<212>  PRT
<213>  Streptococcus pneumoniae CDC 116 (serotype 23F)

<400>  143

Met Lys Lys Leu Gly Thr Leu Leu Val Leu Phe Leu Ser Ala Ile Ile
1               5                   10                  15

Leu Val Ala Cys Ala Ser Gly Lys Lys Asp Thr Thr Ser Gly Gln Lys
            20                  25                  30

Leu Lys Val Val Ala Thr Asn Ser Ile Ile Ala Asp Ile Thr Lys Asn
        35                  40                  45

Ile Ala Gly Asp Lys Ile Asp Leu His Ser Ile Val Pro Ile Gly Gln
    50                  55                  60

Asp Pro His Glu Tyr Glu Pro Leu Pro Glu Asp Val Lys Lys Thr Ser
65                  70                  75                  80

Glu Ala Asn Leu Ile Phe Tyr Asn Gly Ile Asn Leu Glu Thr Gly Gly
                85                  90                  95

Asn Ala Trp Phe Thr Lys Leu Val Glu Asn Ala Lys Lys Thr Glu Asn
            100                 105                 110

Lys Asp Tyr Phe Ala Val Ser Asp Gly Val Asp Val Ile Tyr Leu Glu
        115                 120                 125

Gly Gln Asn Glu Lys Gly Lys Glu Asp Pro His Ala Trp Leu Asn Leu
    130                 135                 140

Glu Asn Gly Ile Ile Phe Ala Lys Asn Ile Ala Lys Gln Leu Ser Ala
145                 150                 155                 160

Lys Asp Pro Asn Asn Lys Glu Phe Tyr Glu Lys Asn Leu Lys Glu Tyr
                165                 170                 175

Thr Asp Lys Leu Asp Lys Leu Asp Lys Glu Ser Lys Asp Lys Phe Asn
            180                 185                 190

Lys Ile Pro Ala Glu Lys Lys Leu Ile Val Thr Ser Glu Gly Ala Phe
        195                 200                 205

Lys Tyr Phe Ser Lys Ala Tyr Gly Val Pro Ser Ala Tyr Ile Trp Glu
    210                 215                 220

Ile Asn Thr Glu Glu Glu Gly Thr Pro Glu Gln Ile Lys Thr Leu Val
225                 230                 235                 240

Glu Lys Leu Arg Gln Thr Lys Val Pro Ser Leu Phe Val Glu Ser Ser
                245                 250                 255

Val Asp Asp Arg Pro Met Lys Thr Val Ser Gln Asp Thr Asn Ile Pro
            260                 265                 270

Ile Tyr Ala Gln Ile Phe Thr Asp Ser Ile Ala Glu Gln Gly Lys Glu
        275                 280                 285

Gly Asp Ser Tyr Tyr Ser Met Met Lys Tyr Asn Leu Asp Lys Ile Ala
    290                 295                 300

Glu Gly Leu Ala Lys
305

## Description

The present invention relates to a protective peptide or a functionally active variant of the protective peptide; a composition comprising at least two proteins selected from the group consisting of i) a first type of protective peptide or functionally active variant thereof, ii) a second type of protective peptide or functionally active variant thereof and iii) a supportive peptide or a functionally active variant thereof; one or more nucleic acid(s) encoding the protective peptide or functionally active variant thereof or the at least two proteins comprised in the composition; a pharmaceutical composition comprising the protective peptide or functionally active variant thereof, the composition, or the nucleic acid(s); a method of producing an antibody using the protective peptide or functionally active variant thereof or the composition; the use of the protective peptide or functionally active variant thereof and/or the composition and/or the nucleic acid(s) for the manufacture of a medicament for the immunization or treatment of a subject; a method of diagnosing a *S. pneumoniae* infection using the protective peptide or a functionally active variant thereof, the composition or a primer and/or probe specific for the nucleic acid(s); a method for identifying a ligand capable of binding to a protective peptide or variant thereof; and the use of a protective peptide or variant thereof for the isolation and/or purification and/or identification of an interaction partner of the peptide.

*Streptococcus pneumoniae (Pneumococcus)* is a lancet-shaped, gram-positive, facultative anaerobic bacterium. It is only the encapsulated organism that is pathogenic for humans and experimental animals. Capsules are antigenic and form the basis for classifying pneumococci by serotypes. Ninety serotypes have been identified, based on their reaction with type-specific antisera. The genome of *S. pneumoniae* contains app. 2.16 Mb. It has an average GC content of 39.7%. *S*. *pneumoniae* is a strictly human pathogen. The complete genome sequence of a capsular serotype 4 isolate of *S. pneumoniae,* designated TIGR4 (T4), was determined by the random shotgun sequencing strategy (GenBank accession number AE005672). This clinical isolate was taken from the blood of a 30-year-old male patient in Kongsvinger, Norway, and is highly invasive and virulent in a mouse model of infection.

Most *S. pneumoniae* serotypes have been shown to cause serious disease, and the ten most common serotypes are estimated to account for about 62% of invasive disease worldwide. The ranking and serotype prevalence differs by age group and geographic area.

Pneumococci are common inhabitants of the respiratory tract, and may be isolated from the nasopharynx of 5% to 70% of normal adults. Rates of asymptomatic carriage vary with age, environment, and the presence of upper respiratory infections. Only 5%-10% of adults without children are carriers. In schools and orphanages, 27% to 58% of students and residents may be carriers. On military installations, as many as 50% to 60% of service personnel may be carriers. The duration of carriage varies and is generally longer in children than adults (reviewed in Epidemiology and Prevention of Vaccine-Preventable Diseases, 7th Edition-Second Printing, The Pink Book).

The relationship of carriage to the development of natural immunity as well as the immunologic mechanism that allows disease to occur in a carrier are poorly understood.

*Streptococcus pneumoniae* is an important agent of human disease at the extremities of age and in those who have underlying disease. Pneumococcal disease kills more people - in the US 40,000 or more each year - than all other vaccine preventable diseases combined. The major clinical syndromes of pneumococcal disease include pneumonia, bacteremia, and meningitis. The disease most often occurs when a predisposing condition exists, particularly pulmonary disease. It is a common bacterial complication of antecedent viral respiratory infection such as influenza and measles, and of chronic conditions such as chronic obstructive pulmonary disease, diabetes, congestive heart failure, renal failure, smoking and alcoholism. Pneumococcal infections are more common during the winter and in early spring when respiratory diseases are more prevalent. Immunodeficiency (splenic dysfunction, iatrogcn, etc.) is a risk factor for development of fatal pncumococcal infections, because of decreased bacterial clearance and lack of antibodies. The incubation period is short, 1-3 days. Symptoms include an abrupt onset of fever and shaking chills or rigor, productive cough, pleuritic chest pain, dyspnoe, tachycardia and hypoxia.

*S. pneumoniae* is responsible for 88% of bacteremia infections in the US. Pneumonia is the most common form of invasive pneumococcal diseases: 150,000-570,000 cases per year (US). 36% of adult community-acquired and 50% of hospital-acquired pneumonia is caused by *S. pneumoniae* (US). The incidence of disease among adults aged 65 years and older has been reported to be ∼60 cases/100,000. Case fatality rates for this disease increase from 1.4% for those aged two or younger to as high as 20.6% among those aged 80 or older. Diseases caused by influenza and *Pneumococcus* are together the fifth leading cause of death for persons aged 65 and older. Mortality attributable to these pathogens is more than 90% in this age group. Bacteremia occurs in about 25-30% of patients with pneumonia. The overall mortality rate of bacteremia is about 20%, but may be as high as 60% in elderly people. In 1998, 51% of all deaths attributable to invasive pneumococcal diseases occurred in age group above 65 years. Pneumococci cause 13%-19% of all cases of bacterial meningitis in the United States. An estimated 3,000 to 6,000 cases of pneumococcal meningitis occur each year. One-quarter of patients with pneumococcal meningitis also have pneumonia. The clinical symptoms, spinal fluid profile and neurologic complications are similar to other forms of purulent bacterial meningitis (reviewed in Epidemiology and Prevention of Vaccine-Preventable Diseases, 7th Edition-Second Printing, The Pink Book).

In children, Pneumococci are a common cause of acute otitis media, and are detected in 28%-55% of middle ear aspirates. By age 12 months, 62% of children have had at least one episode of acute otitis media. Middle ear infections are the most frequent reasons for pediatric office visits in the United States, resulting in over 20 million visits annually. Complications of pneumococcal otitis media may include mastoiditis and meningitis. Bacteremia without a known site of infection is the most common invasive clinical presentation among children <2 years of age, accounting for approximately 70% of invasive disease in this age group. Bacteremic pneumonia accounts for 12%-16% of invasive pneumococcal disease among children <2 years of age. With the decline of invasive Hib disease, *S. pneumoniae* has become the leading cause of bacterial meningitis among children <5 years of age in the United States. Children <1 year have the highest rates of pneumococcal meningitis, approximately 10 cases per 100,000 population. The burden of pneumococcal disease among children <5 years of age is significant. An estimated 17,000 cases of invasive disease occur each year, of which 13,000 are bacteremia without a known site of infection and about 700 are meningitis. An estimated 200 children die every year as a result of invasive pneumococcal disease. Although not considered invasive disease, an estimated 5 million cases of acute otitis media occur each year among children <5 years of age (reviewed Epidemiology and Prevention of Vaccine-Preventable Diseases, 7th Edition-Second Printing, The Pink Book).

A definitive diagnosis of infection with *Streptococcus pneumoniae* generally relies on isolation of the organism from blood or other normally sterile body sites. Tests are also available to detect capsular polysaccharide antigen in body fluids.

Penicillin is the drug of choice for treatment. However, successful implementation of anti-infective therapy has become increasingly difficult because of widespread antimicrobial resistance. Resistance to penicillin is rising, and according to recent reports it reaches ∼ 25% in the US (Whitney, C., et aL (2000). N Engl J Med 343: 1917-24). The proportion of macrolide-resistant strains reached ∼ 20 % (Hyde, T., et al. (2001). JAMA 286: 1857-62). Use of antimicrobial agents is highly correlated with the increase in resistance of *S. pneumoniae* to β-lactams and macrolides (McCormick, A., et al. (2003). Nat Med 9: 424-30).

However, even with effective antibiotic therapy (sensitive strains), the case fatality rate of invasive disease is high with an average of 10% in the developed world and can be much higher with certain serotypes, in elderly patients and in cases of bacteremia or meningitis (up to 80%).

Thus, there remains a need for an effective treatment to prevent or ameliorate pneumococcal infections. A vaccine could not only prevent infections by streptococci, but more specifically prevent or ameliorate colonization of host tissues (esp. in nasopharynx), thereby reducing the incidence of upper respiratory infections and other suppurative infections, such as otitis media. Elimination of invasive diseases - pneumonia, bacteremia and meningitis, and sepsis - would be a direct consequence of reducing the incidence of acute infection and carriage of the organism. Vaccines capable of showing cross-protection against the majority of *S. pneumoniae* strains causing human infections would also be useful to prevent or ameliorate infections caused by all other streptococcal species, namely groups A, B, C and G.

A vaccine can contain a whole variety of different antigens. Examples of antigens arc whole-killed or attenuated organisms, subtractions of these organisms/tissues, proteins, or, in their most simple form, peptides. Antigens can also be recognized by the immune system in form of glycosylated proteins or peptides and may also be or contain polysaccharides or lipids. Short peptides can be used since for example cytotoxic T-cells (CTL) recognize antigens in form of short, usually 8-11 amino acids long peptides in conjunction with major histocompatibility complex (MHC). B-cells can recognize linear epitopes as short as 4-5 amino acids, as well as three-dimensional structures (conformational epitopes). In some circumstances adjuvants may be useful for sustaining antigen-specific immune responses. Primarily, adjuvants are acting, but are not restricted in their mode of action, on so-called antigen presenting cells (APCs). These cells usually first encounter the antigen(s) followed by presentation of processed or unmodified antigen to immune effector cells. Intermediate cell types may also be involved. Only effector cells with the appropriate specificity are activated in a productive immune response. The adjuvant may also locally retain antigens and co-injected other factors. In addition the adjuvant may act as a chcmoattractant for other immune cells or may act locally and/or systemically as a stimulating agent for the immune system.

Efforts to develop effective pneumococcal vaccines began as early as 1911. However, with the advent of penicillin in the 1940s, interest in the vaccine declined, until it was observed that many patients still died despite antibiotic treatment. By the late 60s, efforts were again being made to develop a polyvalent vaccine. The first pneumococcal vaccines contained purified capsular polysaccharide antigen from 14 different types of pneumococcal bacteria. In 1983, a 23-valcnt polysaccharide vaccine (PPV23) was licensed and replaced the 14-valent vaccine, which is no longer produced. PPV23 contains polysaccharide antigen from 23 types of pneumococcal bacteria which cause 88% of bacteremic pneumococcal disease. In addition, cross-reactivity occurs for several capsular types which account for an additional 8% of bacteremic disease. Two polysaccharide vaccines are available in the United States (Pneumovax 23, Merck, and Pnu-Immune 23, Wyeth-Lederle). Both vaccines contain 25 µg of each antigen per dose and include either phenol or thimerosal as a preservative.

The first pneumococcal conjugate vaccine (PCV7, Prevnar) was licensed in the United States in 2000. It includes purified capsular polysaccharide of 7 serotypes of *S. pneumoniae* (4, 9V, 14, 19F, 23F, 18C, and 6B) conjugated to a nontoxic variant of diphtheria toxin known as CRM197. The serotypes included in Prevnar accounted for 86% of bacteremia, 83% of meningitis, and 65% of acute otitis media among children <6 years of age in the United States during 1978-1994 (reviewed in Epidemiology and Prevention of Vaccine-Preventable Diseases. 7th Edition-Second Printing, The Pink Book). Additional pneumococcal polysaccharide conjugate vaccines containing 9 and 11 serotypes of *S. pneumoniae* are being developed. The vaccine is administered intramuscularly. After 4 doses of Prevnar vaccine, virtually all healthy infants develop antibody to all 7 serotypes contained in the vaccine. Prevnar has also been shown to be immunogenic in infants and children, including those with sickle cell disease and HIV infection. In a large clinical trial, Prevnar was shown to reduce invasive disease caused by vaccine serotypes, and reduce invasive disease caused by all serotypes, including serotypes not in the vaccine. Children who received Prevnar had fewer episodes of acute otitis media and underwent fewer tympanostomy tube placements than unvaccinatcd children. The duration of protection following Prevnar is currently unknown. Immunization with Prevnar reduces the rate of nasopharyngeal carriage of the vaccine serotypes, while the overall carriage rate is unaffected. Unfortunately, it has also been shown to induce serotype redistribution, that is the replacement of vaccine serotypes by strains, which are not covered by Prevnar (Pelton, S., et al. (2003). Vaccine 21: 1562-71).

Pneumococcal vaccine is recommended to be administered routinely to i., all children as part of the routine childhood immunization schedule, ii., adults 65 years of age and older and iii., persons aged >2 years with normal immune systems who have chronic illnesses, including cardiovascular disease, pulmonary disease, diabetes, alcoholism, cirrhosis, or ccrcbrospinal fluid leaks. In the elderly population the target groups for pneumococcal vaccine and influenza vaccine overlap. These vaccines can be given at the same time at different sites without increased side effects.

High mortality is observed among high-risk individuals (with underlying disease - mainly viral respiratory infection, immunocompromised) even with effective antibiotic therapy. The mAb approach targets patients with serious disease and provides immediate immune enhancement for the clearance of the bacteria. Through opsonization bacteria are killed within phagocytic cells and not lysed in the blood by antibiotics. This mechanism of action can help to eliminate the release of toxins (such as pneumolysin and other cytotoxins), which worsen the clinical condition of septic patients. Recent advances in the technology of monoclonal antibody production provide the means to generate human antibody reagents and reintroduce antibody therapies, while avoiding the toxicities associated with scrum therapy. Immunoglobulins are an extremely versatile class of antimicrobial proteins that can be used to prevent and treat emerging infectious diseases. Antibody therapy has been effective against a variety of diverse microorganisms reviewed in (Burnic, J., ct al. (1998). J Antimicrob Chemother 41: 319-22).

Although capsular specific antibodies have been shown to be highly protective, it remains unclear what concentration of these serotype-specific antibodies protect against disease and more recently it has become clear that opsonic activity and avidity of these antibodies are more critical determinants of protection than concentration.

Protein conjugate vaccines are no doubt a great new addition to the amarmatorium in the battle against pneumococcal disease, but the vaccine contains a limited number of pneumococcal serotypes and given adequate ecological pressure, replacement disease by non-vaccine serotypes remains a real threat, particularly in areas with very high disease burden.

During the last decade the immunogenicity and protective capacity of several pneumococcal proteins have been described in animal models and these arc now being explored for the development of species-common protein based vaccines. Such proteins are the Pneumococcal surface protein A (PspA, McDaniel, L., et al. (1991). Infect Immun 59: 222-8; Rochc, H., ct al. (2003). Infect Immun 71: 1033-41), Pneumococcal surface adhesin A (PsaA, Talkington, D., et al. (1996). Microb Pathog 21: 17-22), Choline binding protein A (CbpA, Rosenow, C., et al. (1997). Mol Microbiol 25: 819-29), LytB glucosaminidase, LytC muramidase, PrtA serine protease, PhtA (histidine triad A) and Pneumococcal vaccine antigen A (PvaA) Wizemann, T., et al. (2001). Infect Immun 69: 1593-8; Adamou, J., et al. (2001). Infect Immun 69: 949-58).

Certain proteins or enzymes displayed on the surface of gram-positive organisms significantly contribute to pathogenesis, and might be involved in the disease process caused by these pathogens. Often, these proteins are involved in direct interactions with host tissues or in concealing the bacterial surface from the host defense mechanisms (Navarre, W., et al. (1999). Microbiol Mol Biol Rev 63: 174-229). *S. pneumoniae* is not an exception in this regard. Several surface proteins are characterized as virulence factors, important for pneumococcal pathogcnicity as reviewed in Jedrzejas, M. (2001). Microbiol Mol Biol Rev 65: 187-207. If antibodies to these proteins could offer better protection to humans, they could provide the source of a novel, protein-based pneumococcal vaccine to be used in conjunction with or in place of the more traditional capsular polysaccharide vaccine. The use of some of the above-described proteins as antigens for a potential vaccine as well as a number of additional candidates reviewed in Di Guilmi, A., et al. (2002). EMBO Rep 3: 728-34 resulted mainly from a selection based on easiness of identification or chance of availability. In order to meet the demand to identify relevant antigens for *S. pneumoniae* in a more comprehensive way methods for identification, isolation and production of hyperimmune scrum reactive antigens from a specific pathogen, especially from *Staphylococcus aureus* and *Staphylococcus epidermidis* (WO 02/059148) have been developed. Additionally, methods for identification of reactive antigens as well as reactive antigens of *Streptoroccus pneumoniae* have been provided (WO 2004/092209).

The problem underlying the present invention was to provide alternative means for the development of medicaments such as vaccines against *S. pneumoniae* infection. More particularly, the problem was to provide an alternative protective peptide or combinations thereof, particularly more effective proteins or combinations thereof, from *S. pneumoniae* that can be used for the manufacture of said medicaments.

Surprisingly, the object has been solved by one or more peptides consisting of the amino acid sequence of
- SEQ ID NO:1 and/or SEQ ID NO:2; and
- optionally SEQ ID NO:3.

However, a functionally active variant of these sequences may also be used in the context of the present invention.

Therefore, a first subject of the invention is a protective peptide consisting of the amino acid sequence of the SEQ ID NO:1, or a functionally active variant of the protective peptide. These peptides (protective peptide of the amino acid sequence of the SEQ ID NO:1 and functionally active variants thereof) are referred to as antigenic peptides of subgroup i).

The protective peptide consisting of the amino acid sequence of SEQ ID NO:1 is derived from *S. pneumoniae* serotype T4 and has been denoted by SP2216-1. The amino acid and DNA sequences of the full length protein (comprising 392 amino acids) from which the protective protein consisting of the amino acid sequence of the SEQ ID NO:1 is derived is disclosed in WO 2004/092209 as SEQ ID NO:243 and 99.

The amino acid sequence of SEQ ID NO:1 is disclosed in the Examples as well as in the attached Sequence Listing. The peptide of SEQ ID NO:1 has been shown to induce a protective immune response against different scrotypcs and/or to show protection against *S. pneumoniae* in a sepsis and/or pneumonia model (see Examples).

Functionally active variants may be obtained by changing the sequence of the protective peptide as defined below and are characterized by having a biological activity similar to that displayed by the protective peptide of the sequence of SEQ ID NO:1 from which the variant is derived, including the ability to induce protective immune responses and/or to show protection against *S. pneumoniae* e.g. in a sepsis and/or pneumonia model, wherein any variant may be tested in any of the tests described in the Examples.

The functionally active variant of a protective peptide may be obtained by sequence alterations in the protective peptide, wherein the peptide with the sequence alterations retains a function of the unaltered protective peptide, e.g. having a biological activity similar to that displayed by the unaltered protective peptide (see above). Such sequence alterations can include, but are not limited to, (conservative) substitutions, deletions, mutations and insertions.

In a preferred embodiment of the invention the functionally active variant of the protective peptide consisting of the amino acid sequence of the SEQ ID NO:1
a) is a functionally active fragment of the protective peptide, the functionally active fragment comprising at least 75% of the sequence of the protective peptide, preferably at least 80%, more preferably at least 85%, still more preferably at least 90%, even more preferably at least 95% and most preferably at least 97%, 98% or 99%;
b) is derived from the protective pcptidc by at least one amino acid substitution and/or deletion, wherein the functionally active variant has a sequence identity to the protective peptide or to the functionally active fragment as defined in a) of at least 75%, preferably at least 80%, more preferably at least 85%, still more preferably at least 90%, even more preferably at least 95% and most preferably at least 97%, 98% or 99%; and/or
c) consists of the protective peptide or a functionally active variant thereof, preferably the variant of a) and/or b), and additionally at least one amino acid heterologous to the protective peptide.

The functionally active variant of the invention is characterized by having a biological activity similar to that displayed by the protective peptide, including the ability to induce protective immune responses and/or to show protection against *S. pneumoniae* e.g. in a sepsis and/or pneumonia model. The variant of the protective peptide is functionally active in the context of the present invention, if the activity of the variant amounts to at least 10%, preferably at least 25%, more preferably at least 50%, even more preferably at least 70%, still more preferably at least 80%, especially at least 90%, particularly at least 95%, most preferably at least 99% of the activity of the protective peptide without sequence alteration. The activity of the variant may be determined or measured as described in the Examples and then compared to that obtained for the protective peptide of the amino acid sequence of SEQ ID NO:1.

The functionally active fragment of the protective peptide is characterized by being derived from the protective peptide of SEQ ID NO:1 by one or more deletions resulting in a peptide comprising at least 75% of the sequence of the protective peptide, preferably at least 80%, more preferably at least 85%, still more preferably at least 90%, even more preferably at least 95% and most preferably at least 97%, 98% or 99%. Sequence identity may be determined as described below. The deletion(s) may be C-terminally, N-terminally and/or internally. Preferably the fragment is obtained by 1, 2, 3, 4, 5, 6, 7, 8, 9 or 10, more preferably 1, 2, 3, 4 or 5, even more preferably 1, 2 or 3, still more preferably 1 or 2, most preferably 1 deletion(s).

Alternatively or additionally the variant may be obtained from the protective peptide by at least one amino acid substitution and/or deletion, wherein the functionally active variant has a sequence identity to the protective peptide or to the functionally active fragment as defined in a) of at least 75%, preferably at least 80%, more preferably at least 85%, still more preferably at least 90%, even more preferably at least 95% and most preferably at least 97%, 98% or 99%. Sequence identity may be determined as described below. The substitution(s) and/or deletion(s) may be C-terminally, N-terminally and/or internally. Preferably the functionally active variant is obtained from the protective peptide or the fragment, preferably the protective peptide, by 1, 2, 3, 4, 5, 6, 7, 8, 9 or 10, more preferably 1, 2, 3, 4 or 5, even more preferably 1, 2 or 3, still more preferably 1 or 2, most preferably 1 amino acid substitution(s) and/or deletion(s).

Furthermore, the variant may consist of the protective peptide or the functionally active variant thereof, preferably the variant of a) and/or b), and at least one amino acid residue heterologous to the protective peptide or variant thereof, such as a marker protein. The feature "heterologous amino acid" or "amino acid heterologous to the protective peptide or variant thereof refers to any amino acid which is different from that amino acid located adjacent to the protective protein in any naturally occurring protein of *S. pneumoniae,* particularly from that of *S. pneumoniae* serotype T4, especially the sequence made reference to above. Therefore, the protein of the invention encompassing at least one heterologous amino acid refers to a protein which is different from any naturally occurring protein of *S. pneumoniae,* particularly from that of *S. pneumoniae* serotype T4. The one or more additional amino acids may be C-terminally, N-terminally or C- and N-terminally to the protective peptide or variant thereof.

A second subject of the invention is a protective peptide consisting of the amino acid sequence of the SEQ ID NO:2, or a functionally active variant of the protective peptide. These peptides (protective peptide of the amino acid sequence of the SEQ ID NO:2 and functionally active variants thereof) are referred to as antigenic peptides of subgroup ii). Antigenic peptides of subgroup i) and ii) are referred to as antigenic peptides.

The protective peptide consisting of the amino acid sequence of SEQ ID NO:2 is derived from *S. pneumoniae* serotype 6B and has been denoted by SP1732-3. The amino acid and DNA sequences of the full length protein (comprising 659 amino acids) from which the protective protein consisting of the amino acid sequence of the (SEQ ID NO:2 is derived is disclosed in WO 2004/092209 as SEQ ID NO:214 and 70. However, the sequence disclosed in WO 2004/092209 relates to *S. pneumoniae* serotype T4, whereas the SEQ ID NO:2 of the present invention relates to *S. pneumoniae* serotype 6B. It is noted that SEQ ID NO:2 of the present invention differs from that of WO 2004/092209 in that at position 279 of SEQ ID NO:2 there is a V (valine) as compared to an A (alanine) in the sequence of WO 2004/092209. (See position 623 in Fig. 6.)

The amino acid sequence of SEQ ID NO:2 is disclosed in the Examples as well as in the attached Sequence Listing. The peptide of SEQ ID NO:2 has been shown to induce a protective immune response against different serotypes and/or to show protection against *S. pneumoniae* in a sepsis and/or pneumonia model (see Examples). Functionally active variants may be obtained by changing the sequence of the protective peptide as defined below and are characterized by having a biological activity similar to that displayed by the protective peptide of the sequence of SEQ ID NO:2 from which the variant is derived, including the ability to induce protective immune responses and/or to show protection against *S. pneumoniae* e.g. in a sepsis and/or pneumonia model, wherein any variant may be tested in any of the tests described in the Examples.

The functionally active variant of a protective peptide may be obtained as described above.

In a preferred embodiment of the invention the functionally active variant of the protective peptide consisting of the amino acid sequence of the SEQ ID NO:2
a) is a functionally active fragment of the protective peptide, the functionally active fragment comprising at least 75% of the sequence of the protective peptide, preferably at least 80%, more preferably at least 85%, still more preferably at least 90%, even more preferably at least 95% and most preferably at least 97%, 98% or 99%;
b) is derived from the protective peptide by at least one amino acid substitution, addition and/or deletion, wherein the functionally active variant has a sequence identity to the protective peptide or to the functionally active fragment as defined in a) of at least 75%, preferably at least 80%, more preferably at least 85%, still more preferably at least 90%, even more preferably at least 95% and most preferably at least 97%, 98% or 99%; and/or
c) consists of the protective peptide or a functionally active variant thereof, preferably the variant of a) and/or b), and additionally at least one amino acid heterologous to the protective peptide.

The functionally active variant of the invention is characterized by having a biological activity similar to that displayed by the protective peptide, including the ability to induce protective immune responses and/or to show protection against *S. pneumoniae* e.g. in a sepsis and/or pneumonia model. The variant of the protective peptide is functionally active in the context of the present invention, if the activity of the variant amounts to at least 10%, preferably at least 25%, more preferably at least 50%, even more preferably at least 70%, still more preferably at least 80%, especially at least 90%, particularly at least 95%, most preferably at least 99% of the activity of the protective peptide without sequence alteration. The activity of the variant may be determined or measured as described in the Examples and then compared to that obtained for the protective peptide of the amino acid sequence of SEQ ID NO:2.

The functionally active fragment of the protective peptide is characterized by being derived from the protective peptide of SEQ ID NO:2 by one or more deletions resulting in a peptide comprising at least 75% of the sequence of the protective peptide, preferably at least 80%, more preferably at least 85%, still more preferably at least 90%, even more preferably at least 95% and most preferably at least 97%, 98% or 99%. Sequence identity may be determined as described below. The deletion(s) may be C-terminally, N-terminally and/or internally. Preferably the fragment is obtained by 1, 2, 3, 4, 5, 6, 7, 8, 9 or 10, more preferably 1, 2, 3, 4 or 5, even more preferably 1, 2 or 3, still more preferably 1 or 2, most preferably 1 deletion(s).

Alternatively or additionally the variant may be obtained from the protective peptide by at least one amino acid substitution, addition and/or deletion, wherein the functionally active variant has a sequence identity to the protective pcptidc or to the functionally active fragment as defined in a) of at least 75%, preferably at least 80%, more preferably at least 85%, still more preferably at least 90%, even more preferably at least 95% and most preferably at least 97%, 98% or 99%. Sequence identity may be determined as described below. The substitution(s), addition(s) and/or deletion(s) may be C-terminally, N-terminally and/or internally. Preferably the functionally active variant is obtained from the protective peptide or the fragment, preferably the protective peptide, by 1, 2, 3, 4, 5, 6, 7, 8, 9 or 10, more preferably 1, 2, 3, 4 or 5, even more preferably 1, 2 or 3, still more preferably 1 or 2, most preferably 1 amino acid substitution(s), addition(s) and/or deletion(s).

Furthermore, the variant may consist of the protective peptide or the functionally active variant thereof, preferably the variant of a) and/or b), and at least one amino acid residue heterologous to the protective peptide or variant thereof, such as a marker protein. The feature "heterologous amino acid" or "amino acid heterologous to the protective peptide or variant thereof" refers to any amino acid which is different from that amino acid located adjacent to the protective protein in any naturally occurring protein of *S. pneumoniae,* particularly from that of *S. pneumoniae* serotype 6B, especially the sequence made reference to above. Therefore, the protein of the invention encompassing at least one heterologous amino acid refers to a protein which is different from any naturally occurring protein of *S. pneumoniae,* particularly from that of *S. pneumoniae* serotype T4. The one or more additional amino acids may be C-terminally, N-terminally or C- and N-terminally to the protective peptide or variant thereof.

The following details are intended to refer to the protective peptides of subgroup i) and ii) as well as the supportive peptide as defined below and the variants of these:

The substituted or additional sequence or amino acid residue(s) as defined above consists of (an) amino acid residue(s), which may be any amino acid, which may be either an Land/or a D-amino acid, naturally occurring and otherwise. Preferably the amino acid is any naturally occurring amino acid such as alanine, cysteine, aspartic acid, glutamic acid, phenylalanine, glycine, histidine, isoleucine, lysine, leucine, methionine, asparagine, proline, glutamine, arginine, serine, threonine, valine, tryptophan or tyrosine.

However, the amino acid residue(s) may also be (a) modified or (an) unusual amino acid(s). Examples of those arc 2-aminoadipic acid, 3-aminoadipic acid, bcta-alaninc, 2-aminobutyric acid, 4-aminobutyric acid, 6-aminocaproic acid, 2-aminoheptanoic acid, 2-aminoisobutyric acid, 3-aminoisobutyric acid, 2-aminopimelic acid, 2,4-diaminobutyric acid, desmosine, 2,2'-diaminopimelic acid, 2,3-diaminopropionic acid, N-ethylglycine, N-ethylasparagine, hydroxylysine, allo-hydroxylysine, 3-hydroxyproline, 4-hydroxyproline, isodesmosine, allo-isoleucine, N-methylglycine, N-methylisoleucine, 6-N-methyllysine, N-methylvaline, norvaline, norleucine or ornithine. Additionally, the amino acid(s) may be subject to modifications such as posttranslational modifications. Examples of modifications include acctylation, amidation, blocking, formylation, gamma-carboxyglutamic acid hydroxylation, glycosilation, methylation, phosphorylation and sulfatation. If more than one substituted or additional or heterologous amino acid residue is present in the peptide, the amino acid residues may be the same or different from one another.

In one preferred embodiment of the invention, the functionally active variant of the peptide of the invention is essentially identical to the protective peptide of subgroup i), the protective peptide of subgroup ii) or supportive peptide, but differs from the peptide of the SEQ ID NOS:1, 2 or 3, respectively, in that it is derived from a homologous sequence of a different scrotypc of *S. pneumoniae.* As detailed above more than 90 different serotypes of pneumococci have been identified so far. Accordingly, any of these serotypes may be the basis for the functionally active variant. However, preferably the serotype is R6, T4, 1, 2, 3, 4, 5, 6A, 6B, 7F, 8, 9N, 9V, 10A, 11A, 12F, 14, 15B, 17F, 18C, 19 such as 19A or 19F, 20, 22F, 23F or 33F; especially the serotype is T4, 6B, 14, 19 or 23F.

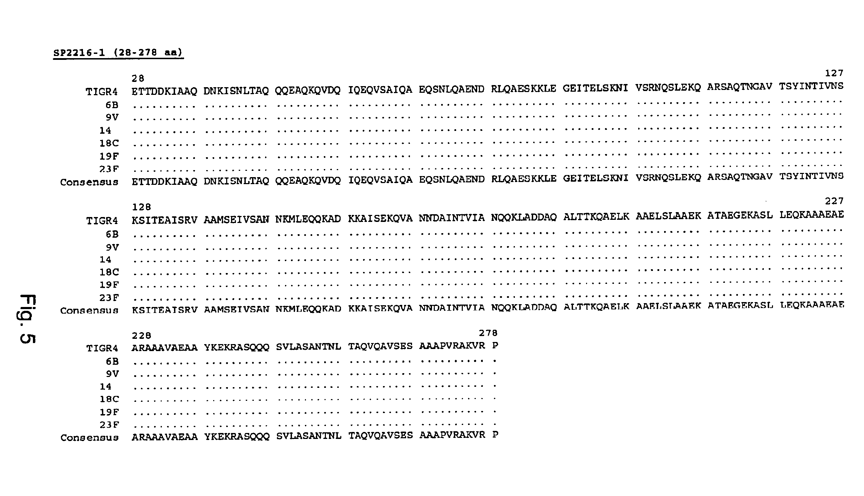

Examples of variants of peptides of SEQ ID NOS: 1, 2 and 3 derived from other serotypes of *S. pneumoniae* are shown in Figs. 5 to 7 and SEQ ID NOS: 15 to 17.

Further examples of homologous sequences of different serotypes and/or different *S. pneumoniae* strains are detailed below and disclosed also in the attached sequence data.

Table A lists several different *S. pneumoniae* strains and their scrotypcs. From most of those strains one or more of the three full length proteins SP2216, SP1732 and SP1650 have been sequenced; **"*n.d*"** shows, when a sequence has not been determined.

If the full length amino acid sequence of SP2216 from the respective strain listed in Table A has been sequenced and is identical to the full length amino acid sequence of SP2216 from TIGR4, this is indicated with **"IDENT."** in the third column of Table A. If the full length amino acid sequence of SP2216 from the respective strain is different from the full length amino acid sequence of SP2216 from TIGR4, i.e. has at least one amino acid substitution, insertion or deletion, the respective SEQ ID NO (as listed in the Sequence Listing) of the full length SP2216 of said strain is given in the third column of Table A. Accordingly, the full length amino acid sequences of SP2216 from strains with at least one amino acid difference compared to TIGR4 are shown as SEQ ID NOs:18 to 30.

If the full length amino acid sequence of SP1732 from the respective strain listed in Table A has been sequenced and is identical to the full length amino acid sequence of SP1732 from 6B, this is indicated with **"IDENT."** in the fourth column of Table A. If the full length amino acid sequence of SP1732 from the respective strain is different from the full length amino acid sequence of SP1732 from 6B, i.e. has at least one amino acid substitution, insertion or deletion, the respective SEQ ID NO (as listed in the Sequence Listing) of the full length SP1732 of said strain is given in the fourth column of Table A. Accordingly, the full length amino acid sequences of SP1732 from strains with at least one amino acid difference compared to 6B are shown as SEQ ID NOs:31 to 129.

If the full length amino acid sequence of SP1650 (PsaA) from the respective strain listed in Table A has been sequenced and is identical to the full length amino acid sequence of SP1650 from 6B, this is indicated with **"IDENT."** in the fifth column of Table A. If the full length amino acid sequence of SP1650 from the respective strain is different from the full length amino acid sequence of SP1650 from 6B, i.e. has at least one amino acid substitution, insertion or deletion, the respective SEQ ID NO (as listed in the Sequence Listing) of the full length SP1650 of said strain is given in the fifth column of Table A. Accordingly, the full length amino acid sequences of SP1650 from strains with at least one amino acid difference compared to 6B are shown as SEQ ID NOs:130 to 143.

**Table A:**

| **Strain name** | **Serotype** | **SP2216** | **SP1732** | **SP1650** |
|---|---|---|---|---|
| CDC 1 | 1 | **IDENT.** | **SEQ ID NO:31** | **IDENT.** |
| CDC 2 | 2 | **IDENT.** | **SEQ ID NO:32** | **IDENT.** |
| CDC 3 | 3 | **SEQ ID NO:18** | **SEQ ID NO:33** | **IDENT.** |
| CDC 4 | 4 | ***n.d.*** | **SEQ ID NO:34** | **IDENT.** |
| CDC 5 | 5 | **IDENT.** | **SEQ ID NO:35** | **SEQ ID NO:130** |
| CDC 6 | 6A | **IDENT.** | **IDENT.** | **IDENT.** |
| CDC 7 | 6B | **IDENT.** | **IDENT.** | **IDENT.** |
| CDC 8 | 7A | **IDENT.** | **SEQ ID NO:36** | **SEQ ID NO:131** |
| CDC 9 | 7B | **IDENT.** | **IDENT.** | **IDENT.** |
| CDC 10 | 7C | **IDENT.** | **IDENT.** | **IDENT.** |
| CDC 11 | 7F | **SEQ ID NO:19** | **SEQ ID NO:37** | **IDENT.** |
| CDC 12 | 8 | **IDENT.** | **IDENT.** | **SEQ ID NO:132** |
| CDC 13 | 9A | **IDENT.** | **SEQ ID NO:38** | **IDENT.** |
| CDC 14 | 9L | **IDENT.** | **IDENT.** | **IDENT.** |
| CDC 15 | 9N | **IDENT.** | **IDENT.** | **IDENT.** |
| CDC 16 | 9V | **IDENT.** | **SEQ ID NO:39** | **IDENT.** |
| CDC 17 | 10A | **IDENT.** | **SEQ ID NO:40** | **IDENT.** |
| CDC 18 | 10B | **IDENT.** | **SEQ ID NO:41** | **IDENT.** |
| CDC 19 | 10C | **IDENT.** | **SEQ ID NO:42** | **IDENT.** |
| CDC 20 | 10F | **IDENT.** | **SEQ ID NO:43** | **IDENT.** |
| CDC 21 | 11A | **IDENT.** | **IDENT.** | **IDENT.** |
| CDC 22 | 11B | **IDENT.** | **SEQ ID NO:44** | **IDENT.** |
| CDC 23 | 11C | **IDENT.** | **SEQ ID NO:45** | **IDENT.** |
| CDC 24 | 11D | **SEQ ID NO:20** | **IDENT.** | **IDENT.** |
| CDC 25 | 11F | **SEQ ID NO:21** | **SEQ ID NO:46** | **IDENT.** |
| CDC 26 | 12A | **IDENT.** | **SEQ ID NO:47** | **IDENT.** |
| CDC 27 | 12B | **SEQ ID NO:22** | ***n.d.*** | **IDENT.** |
| CDC 28 | 12F | **IDENT.** | **SEQ ID NO:48** | **IDENT.** |
| CDC 29 | 13 | ***n.d.*** | ***n.d.*** | ***n.d.*** |
| CDC 30 | 14 | **IDENT.** | **IDENT.** | **IDENT.** |
| CDC 31 | 15A | **IDENT.** | **IDENT.** | **IDENT.** |
| CDC 32 | 15B | **IDENT.** | **IDENT.** | **IDENT.** |
| CDC 33 | 15C | **IDENT.** | **SEQ ID NO:49** | **SEQ ID NO:133** |
| CDC 34 | 15F | **IDENT.** | **SEQ ID NO:50** | **IDENT.** |
| CDC 35 | 16A | **IDENT.** | **IDENT.** | **IDENT.** |
| CDC 36 | 16F | **IDENT.** | **IDENT.** | **SEO ID NO:134** |
| CDC 37 | 17A | **IDENT.** | **SEQ ID NO:51** | **IDENT.** |
| CDC 38 | 17F | **IDENT.** | **SEQ ID NO:52** | **SEQ ID NO:135** |
| CDC 39 | 18A | **IDENT.** | **SEQ ID NO:53** | **IDENT.** |
| CDC 40 | 18B | **IDENT.** | **IDENT.** | **IDENT.** |
| CDC 41 | 18C | **IDENT.** | **SEQ ID NO:54** | **IDENT.** |
| CDC 42 | 18F | **IDENT.** | **SEQ ID NO:55** | **IDENT.** |
| CDC 43 | 19A | **IDENT.** | **IDENT.** | **IDENT.** |
| CDC 44 | 19B | **IDENT.** | **IDENT.** | **IDENT.** |
| CDC 45 | 19C | **IDENT.** | **IDENT.** | **IDENT.** |
| CDC 46 | 19F | **IDENT.** | **IDENT.** | **IDENT.** |
| CDC 47 | 20 | **IDENT.** | **IDENT.** | **IDENT.** |
| CDC 48 | 21 | **IDENT.** | **IDENT.** | **IDENT.** |
| CDC 49 | 22A | **SEQ ID NO:23** | **SEQ ID NO:56** | **IDENT.** |
| CDC 50 | 22F | **IDENT.** | **SEQ ID NO:57** | **IDENT.** |
| CDC 51 | 23A | **IDENT.** | **IDENT.** | **IDENT.** |
| CDC 52 | 23B | **IDENT.** | **IDENT.** | **IDENT.** |
| CDC 53 | 23F | **IDENT.** | **SEQ ID NO:58** | **IDENT.** |
| CDC 54 | 24A | **IDENT.** | **IDENT.** | **IDENT.** |
| CDC 55 | 24B | **IDENT.** | **SEO ID NO:59** | **IDENT.** |
| CDC 56 | 24F | **SEQ ID NO:24** | **IDENT.** | **SEQ ID NO:136** |
| CDC 57 | 25A | **IDENT.** | **SEQ ID NO:60** | **IDENT.** |
| CDC 58 | 25F | **IDENT.** | **SEQ ID NO:61** | **SEQ ID NO:137** |
| CDC 59 | 27 | **IDENT.** | **IDENT.** | **IDENT.** |
| CDC 60 | 28A | **IDENT.** | **SEQ ID NO:62** | **IDENT.** |
| CDC 61 | 28F | **SEQ ID NO:25** | **IDENT.** | **IDENT.** |
| CDC 62 | 29 | **IDENT.** | **SEQ ID NO:63** | **IDENT.** |
| CDC 63 | 31 | **IDENT.** | ***n.d.*** | ***n.d.*** |
| CDC 64 | 32A | **IDENT.** | **IDENT.** | **IDENT.** |
| CDC 65 | 32F | **IDENT.** | **SEQ ID NO:64** | **IDENT.** |
| CDC 66 | 33A | **IDENT.** | **SEO ID NO:65** | **SEO ID NO:138** |
| CDC 67 | 33B | **IDENT.** | **SEO ID NO:66** | **IDENT.** |
| CDC 68 | 33C | **IDENT.** | **SEO ID NO:67** | **IDENT.** |
| CDC 69 | 33D | **IDENT.** | **SEO ID NO:68** | **SEQ ID NO:139** |
| CDC 70 | 33F | **IDENT.** | **SEQ ID NO:69** | **IDENT.** |
| CDC 71 | 34 | **IDENT.** | **SEO ID NO:70** | **IDENT.** |
| CDC 72 | 35A | **IDENT.** | **IDENT.** | **IDENT.** |
| CDC 73 | 35B | **IDENT.** | **IDENT.** | **IDENT.** |
| CDC 74 | 35C | **IDENT.** | **SEO ID NO:71** | **SEQ ID NO:140** |
| CDC 75 | 35F | **IDENT.** | ***n.d.*** | **IDENT.** |
| CDC 76 | 36 | **IDENT.** | **SEQ ID NO:72** | **IDENT.** |
| CDC 77 | 37 | **IDENT.** | **SEQ ID NO:73** | **IDENT.** |
| CDC 78 | 38 | **IDENT.** | **SEQ ID NO:74** | ***n.d.*** |
| CDC 79 | 39 | **IDENT.** | **IDENT.** | **IDENT.** |
| CDC 80 | 40 | **IDENT.** | **SEQ ID NO:75** | **IDENT.** |
| CDC 81 | 41A | **IDENT.** | **SEQ ID NO:76** | **IDENT.** |
| CDC 82 | 41F | **IDENT.** | **IDENT.** | **IDENT.** |
| CDC 83 | 42 | **IDENT.** | **SEQ ID NO:77** | **IDENT.** |
| CDC 84 | 43 | **IDENT.** | **SEQ ID NO:78** | **IDENT.** |
| CDC 85 | 44 | **IDENT.** | **IDENT.** | **IDENT.** |
| CDC 86 | 45 | **IDENT.** | **SEQ ID NO:79** | **IDENT.** |
| CDC 87 | 46 | **IDENT.** | **IDENT.** | **IDENT.** |
| CDC 88 | 47A | **IDENT.** | **IDENT.** | **IDENT.** |
| CDC 89 | 47F | **IDENT.** | **SEQ ID NO:80** | **IDENT.** |
| CDC 90 | 48 | ***n.d.*** | ***n.d.*** | **IDENT.** |
| CDC 91 | 23F | **IDENT.** | **IDENT.** | **IDENT.** |
| CDC 92 | 6B | **IDENT.** | **IDENT.** | **IDENT.** |
| CDC 93 | 9V | **IDENT.** | **SEQ ID NO:81** | **IDENT.** |
| CDC 94 | 23F | **IDENT.** | **IDENT.** | **IDENT.** |
| CDC 95 | 14 | **IDENT.** | **SEQ ID NO:82** | **IDENT.** |
| CDC 96 | 19A | **IDENT.** | **IDENT.** | **SEQ ID NO:141** |
| CDC 97 | 19A | **SEQ ID NO:26** | **IDENT.** | **IDENT.** |
| CDC 98 | 6B | ***n.d.*** | **IDENT.** | **IDENT.** |
| CDC 99 | 14 | **IDENT.** | **IDENT.** | **IDENT.** |
| CDC 100 | 14 | **IDENT.** | **SEQ ID NO:83** | **IDENT.** |
| CDC 101 | 19A | **IDENT.** | **IDENT.** | **IDENT.** |
| CDC 102 | 6B | **IDENT.** | **IDENT.** | ***n.d.*** |
| CDC 103 | 19A | **SEQ ID NO:27** | **SEQ ID NO:84** | **IDENT.** |
| CDC 104 | 19F | **IDENT.** | **SEQ ID NO:85** | ***n.d.*** |
| CDC 105 | 23F | **IDENT.** | **IDENT.** | **IDENT.** |
| CDC 106 | 23F | **SEQ ID NO:28** | **IDENT.** | **IDENT.** |
| CDC 107 | 68 | **IDENT.** | **IDENT.** | ***n.d.*** |
| CDC 108 | 14 | **IDENT.** | **SEQ ID NO:86** | **IDENT.** |
| CDC 109 | 5 | **IDENT.** | **SEQ ID NO:87** | **IDENT.** |
| CDC 110 | 6B | **IDENT.** | **SEQ ID NO:88** | **IDENT.** |
| CDC 111 | 19F | **IDENT.** | **SEQ ID NO:89** | **IDENT.** |
| CDC 112 | 6B | **SEQ ID NO:29** | **SEQ ID NO:90** | **IDENT.** |
| CDC 113 | 6A | **IDENT.** | **SEQ ID NO:91** | **IDENT.** |
| CDC 114 | 35B | ***n.d.*** | **IDENT.** | **SEQ ID NO:142** |
| CDC 115 | 15A | **IDENT.** | **SEQ ID NO:92** | **IDENT.** |
| CDC 116 | 23F | **IDENT.** | **IDENT.** | **SEQ ID NO:143** |
| PJ-1293_1 | 1 | **IDENT.** | **SEQ ID NO:93** | ***n.d.*** |
| PJ-1354_1 | 1 | ***n.d.*** | **IDENT.** | ***n.d.*** |
| PJ-1455_2 | 2 | **IDENT.** | **IDENT.** | ***n.d.*** |
| I-33_3 | 3 | ***n.d.*** | **SEQ ID NO:94** | ***n.d.*** |
| PJ02-157_3 | 3 | ***n.d.*** | **IDENT.** | ***n.d.*** |
| PJ-1319_4 | 4 | **IDENT.** | **SEQ ID NO:95** | ***n.d.*** |
| PJ-1321_4 | 4 | ***n.d.*** | **SEQ ID NO:96** | ***n.d.*** |
| PJ-896_5 | 5 | **IDENT.** | **IDENT.** | ***n.d.*** |
| PJ-1241_6A | 6A | **IDENT.** | **SEQ ID NO:97** | ***n.d.*** |
| PJ-1311_6A | 6A | ***n.d.*** | **IDENT.** | ***n.d.*** |
| PJ-1259_6B | 6B | **IDENT.** | **IDENT.** | ***n.d.*** |
| PJ-1324_6B | 6B | ***n.d.*** | **IDENT.** | ***n.d.*** |
| PJ-1296_7F | 7F | **IDENT.** | **SEQ ID NO:98** | ***n.d.*** |
| PJ-1466_7F | 7F | ***n.d.*** | **SEQ ID NO:99** | ***n.d.*** |
| PJ-738_8 | 8 | **IDENT.** | **IDENT.** | ***n.d.*** |
| PJ-615_9A | 9A | **IDENT.** | **SEQ ID NO:100** | ***n.d.*** |
| PJ-1266_9N | 9N | **IDENT.** | **IDENT.** | ***n.d.*** |
| PJ-1441_9N | 9N | ***n.d.*** | **IDENT.** | ***n.d.*** |
| PJ-1255_9V | 9V | **IDENT.** | **SEQ ID NO:101** | ***n.d.*** |
| PJ-1410_9V | 9V | ***n.d.*** | **SEQ ID NO:102** | ***n.d.*** |
| PJ-1280_10A | 10A | **SEQ ID NO:30** | **IDENT.** | ***n.d.*** |
| PJ-1374_10C | 10C | **IDENT.** | **SEQ ID NO:103** | ***n.d.*** |
| PJ-1256_11A | 11A | **IDENT.** | **IDENT.** | ***n.d.*** |
| PJ02-912_12F | 12F | **IDENT.** | **SEQ ID NO:104** | ***n.d.*** |
| PJ02-441_13 | 13 | **IDENT.** | **IDENT.** | ***n.d.*** |
| PJ-75_14 | 14 | **IDENT.** | **IDENT.** | ***n.d.*** |
| PJ-1364_14 | 14 | ***n.d.*** | **SEQ ID NO:105** | ***n.d.*** |
| RP-968_15A | 15A | **IDENT.** | **IDENT.** | ***n.d.*** |
| PJ-1445_15B | 15B | **IDENT.** | **SEQ ID NO:106** | ***n.d.*** |
| PJ-3_15C | 15C | **IDENT.** | **SEQ ID NO:107** | ***n.d.*** |
| PJ-2449_16F | 16F | **IDENT.** | **SEO ID NO:108** | ***n.d.*** |
| PJ-1874_17A | 17A | ***n.d.*** | **SEQ ID NO:109** | ***n.d.*** |
| PJ-2129_17F | 17F | **IDENT.** | **SEQ ID NO:110** | ***n.d.*** |
| PJ-2245_18C | 18C | **IDENT.** | **SEQ ID NO:111** | ***n.d.*** |
| PJ-132_18F | 18F | **IDENT.** | **SEQ ID NO:112** | ***n.d.*** |
| PJ-1327_19A | 19A | **IDENT.** | **SEO ID NO:113** | ***n.d.*** |
| PJ-1498_19A | 19A | ***n.d.*** | **SEQ ID NO:114** | ***n.d.*** |
| PJ-546_19B | 19B | **IDENT.** | **IDENT.** | ***n.d.*** |
| PJ-1329_19F | 19F | **IDENT.** | **SEQ ID NO:115** | ***n.d.*** |
| PJ-1494_19F | 19F | ***n.d.*** | **SEQ ID NO:116** | ***n.d.*** |
| PJ-1246_20 | 20 | **IDENT.** | **IDENT.** | ***n.d.*** |
| RP-2896_21 | 21 | **IDENT.** | **SEQ ID NO:117** | ***n.d.*** |
| PJ-362_22A | 22A | **IDENT.** | **SEQ ID NO:118** | ***n.d.*** |
| PJ-1291_22F | 22F | **IDENT.** | **SEQ ID NO:119** | ***n.d.*** |
| PJ-1437_23A | 23A | **IDENT.** | **IDENT.** | ***n.d.*** |
| PJ02-709 23B | 23B | **IDENT.** | **IDENT.** | ***n.d.*** |
| PJ-1248_23F | 23F | **IDENT.** | **SEQ ID NO:120** | ***n.d.*** |
| PJ-2250_24A | 24A | **IDENT.** | **SEQ ID NO:121** | ***n.d.*** |
| PJ-1452_24F | 24F | **IDENT.** | **IDENT.** | ***n.d.*** |
| I-59_25F | 25F | **IDENT.** | **SEQ ID NO:122** | ***n.d.*** |
| PJ-1857_27 | 27 | **IDENT.** | **SEQ ID NO:123** | ***n.d.*** |
| PJ02-787_29 | 29 | **IDENT.** | **IDENT.** | ***n.d.*** |
| PJ-1287_31 | 31 | **IDENT.** | **SEQ ID NO:124** | ***n.d.*** |
| PJ-I283_33F | 33F | **IDENT.** | **SEQ ID NO:125** | ***n.d.*** |
| PJ-1176_34 | 34 | **IDENT.** | **SEQ ID NO:126** | ***n.d.*** |
| PJ-1297_35A | 35A | **IDENT.** | **IDENT.** | ***n.d.*** |
| PJ-940_35B | 35B | **IDENT.** | **IDENT.** | ***n.d.*** |
| PJ-1322_35F | 35F | **IDENT.** | **SEQ ID NO:127** | ***n.d.*** |
| PJ-1369_35F | 35F | ***n.d.*** | **SEQ ID NO:128** | ***n.d.*** |
| PJ-2185_40 | 40 | **IDENT.** | **SEQ ID NO:129** | ***n.d.*** |

The genomic sequences from different *S*. *pneumoniae* strains may be obtained from the following sources:
a) *Streptococcus pneumoniae* TIGR4
   (Genbank acc.: AE005672; remark: completed)
   http://cmr.tigr.org/tigr-scripts/CMR/GenomePage.cgi?org=bsp
b) *Streptococcus pneumoniae* R6
   (Genbank acc.: AE007317; remark: completed)
   http://cmr.tigr.org/tigr-scripts/CMR/GenomePage.cgi?org=ntsp02
c) *Streptococcus pneumoniae* Serotype 2 Strain D39
   (Gcnbank acc.: CP000410; remark: complctcd)
d) *Streptococcus pneumoniae* G54
   (Genbank acc.: -; remark: unfinished)
   http://cmr.tigr.org/tigr-scripts/CMR/GenomePage.cgi?org=ntsp05

The term "functionally active variant" includes naturally occurring allelic variants, as well as mutants or any other non-naturally occurring variants. As is known in the art, an allelic variant is an alternate form of a (poly)peptide that is characterized as having a substitution, deletion, or addition of one or more amino acids that does essentially not alter the biological function of the polypeptide. By "biological function" is meant a function of the peptide in the cells in which it naturally occurs, even if the function is not necessary for the growth or survival of the cells. For example, the biological function of a porin is to allow the entry into cells of compounds present in the extracellular medium. The biological function is distinct from the antigenic function. A polypeptide can have more than one biological function.

Accordingly, the present invention also relates to antigenic peptides, i.e. protective peptides and functionally active variants thereof optionally in combination with a supportive peptide or variant thereof as defined below, of different *S. pneumoniae* isolates. Such homologues may easily be identified and isolated based on the nucleic acid and amino acid sequences disclosed herein. A homologous protective peptide of a different scrotypc may be identified by e.g. sequence alignment. The homologous sequence may vary from the protective peptide of subgroup i), the protective peptide of subgroup ii) or the supportive peptide of the sequence of SEQ ID NOS:1, 2 or 3, respectively, by one or more amino acid substitutions, deletions and/or additions.

Percentage of sequence identity can be determined e.g. by sequence alignment. Methods of alignment of sequences for comparison are well known in the art. Various programs and alignment algorithms have been described e.g. in Smith and Waterman, Adv. Appl. Math. 2: 482, 1981 or Pcarson and Lipman, Proc. Natl. Acad. Sci. U.S.A. 85: 2444-2448, 1988.

The NCBI Basic Local Alignment Search Tool (BLAST) (Altschul et al., J. Mol. Biol. 215: 403-410, 1990) is available from several sources, including the National Center for Biotechnology Information (NCBI, Bethesda, MD) and on the Internet, for use in connection with the sequence analysis programs blastp, blastn, blastx, tblastn and tblastx. Variants, e.g. of any protective peptide of the sequences of SEQ ID NOS: 1 or 2 or the supportive peptide of SEQ ID NO:3, are typically characterized using the NCBI Blast 2.0, gapped blastp set to default parameters. For comparisons of amino acid sequences of e.g. at least 35 amino acids, the Blast 2 sequences function may be employed using the default BLOSUM62 matrix set to default parameters, (gap existence cost of 11, and a per residue gap cost of 1).

In a preferred embodiment, the functionally active variant derived from the peptide as defined above by amino acid exchanges, deletions or insertions may also conserve, or more preferably improve, the activity (as defined above). Furthermore, these peptides may also cover epitopes, which trigger the same or preferably an improved T cell response. These epitope are referred to as "heteroclitic". They have a similar or preferably greater affinity to MHC/HLA molecules, and the ability to stimulate the T cell receptors (TCR) directed to the original epitope in a similar or preferably stronger manner. Heteroclitic epitopes can be obtained by rational design i. e. taking into account the contribution of individual residues to binding to MHC/HLA as for instance described by (Rammensee, H. et al., 1999, Immunogenetics. 50: 213-219), combined with a systematic exchange of residues potentially interacting with the TCR and testing the resulting sequences with T cells directed against the original epitope. Such a design is possible for a skilled man in the art without much experimentation.

Conservative substitutions are those that take place within a family of amino acids that are related in their side chains and chemical properties. Examples of such families are amino acids with basic side chains, with acidic side chains, with non-polar aliphatic side chains, with non-polar aromatic side chains, with uncharged polar side chains, with small side chains, with large side chains etc.. In one embodiment, one conservative substitution is included in the peptide. In another embodiment, two conservative substitutions or less are included in the peptide. In a further embodiment, three conservative substitutions or less are included in the peptide.

Examples of conservative amino acid substitutions include, but are not limited to, those listed below:

| Original Residue | Conservative Substitutions |
|---|---|
| Ala | Ser |
| Arg | Lys |
| Asn | Gln; His |
| Asp | Glu |
| Cys | Ser |
| Gln | Asn |
| Glu | Asp |
| His | Asn; Gln |
| Ile | Leu; Val |
| Leu | Ile; Val |
| Lys | Arg; Gln; Asn |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

In another embodiment of the invention the peptide as defined above may be modified by one or more of a variety of chemical techniques to produce derivatives having essentially the same activity (as defined above for fragments and variants) as the modified pcptidcs, and optionally having other desirable properties. For example, carboxylic acid groups of the protein, whether C-terminal or side chain, may be provided in the form of a salt of a pharmaceutically-acceptable cation or cstcrificd to form an ester, or converted to an amide. Amino groups of the peptide, whether amino-terminal or side chain, may be in the form of a pharmaceutically-acceptable acid addition salt, such as the HCl, HBr, acetic, benzoic, toluene sulfonic, maleic, tartaric and other organic salts, or may be converted to an amide.

Hydroxyl groups of the peptide side chains may be converted to alkoxy or to an ester using well recognized techniques. Phenyl and phenolic rings of the peptide side chains may be substituted with one or more halogen atoms, such as fluorine, chlorine, bromine or iodine, or with alkyl, alkoxy, carboxylic acids and esters thereof, or amides of such carboxylic acids. Thiols can be protected with any one of a number of well recognized protecting groups, such as acetamide groups.

Peptides of this invention may be in combination with outer surface proteins or other proteins or antigens of other proteins. In such combination, the peptide may be in the form of a fusion protein. The antigenic peptide or supportive peptide of the invention may be optionally fused to a selected peptide or protein derived from other microorganisms. For example, a peptide or protein of this invention may be fused at its N-tcrminus or C-terminus to a polypeptide from another pathogen or to more than one polypeptide in sequence. Peptides which may be useful for this purpose include polypeptides identified by the prior art.

In a preferred embodiment of the invention the peptide of the invention is fused to an epitope tag which provides an epitope to which an anti-tag substance can selectively bind. The epitope tag is generally placed at the N- or C-tcrminus of the peptide but may be incorporated as an internal insertion or substitution as the biological activity permits. The presence of such epitope-tagged forms of a peptide can be detected using a substance such as an antibody against the tagged peptide. Also, provision of the epitope tag enables the peptide to be readily purified by affinity purification using an anti-tag antibody or another type of affinity matrix that binds to the epitope tag. Various tag polypeptides and their respective antibodies are well known in the art. Examples include a poly-histidine (poly-his) tag, e.g. a hexa-histidine tag as described in the Examples, a poly-histidine-glycine (poly-his-gly) tag, the HA tag polypeptide, the c-myc tag, the Strcp tag and the FLAG tag.

Fusions also may include the peptides of this invention fused or coupled to moieties other than amino acids, including lipids and carbohydrates. Further, peptides/proteins/compositions of this invention may be employed in combination with other vaccinal agents described by the prior art, as well as with other species of vaccinal agents derived from other microorganisms. Such proteins are useful in the prevention, treatment and diagnosis of diseases caused by a wide spectrum of Streptococcus isolates.

These fusion proteins are constructed for use in the methods and compositions of this invention. These fusion proteins or multimeric proteins may be produced recombinantly, or may be Synthesized chemically.

The peptides and proteins described herein may be prepared by any of a number of conventional techniques. Desired peptides may be chemically synthesized. An alternative approach involves generating the fragments of known peptides by enzymatic digestion, e.g., by treating the protein with an enzyme known to cleave proteins at sites defined by particular amino acid residues, or by digesting the DNA with suitable restriction enzymes, expressing the digested DNA and isolating the desired fragment. Yet another suitable technique involves isolating and amplifying a DNA fragment encoding a desired peptide fragment, by polymerase chain reaction (PCR). Oligonucleotides that define the desired termini of the DNA fragment are employed as the 5' and 3' primers in the PCR. Techniques for making mutations, such as deletions, insertions and substitutions, at predetermined sites in DNA, and therefore in proteins, having a known sequence are well known. One of skill in the art using conventional techniques, such as PCR, may readily use the pcptidcs, proteins and compositions provided herein to identify and isolate other similar proteins. Such methods are routine and not considered to require undue experimentation, given the information provided herein. For example, variations can be made using oligonucleotide-mediated site-directed mutagenesis (Carter et al., Nucl. Acids Res., 13:4431 (1985); Zoller et al., Nucl. Acids Res. 10:6487 (1987)), cassette mutagenesis (Wells et al., Gene, 34:315 (1985)), restriction selection mutagenesis (Wells et al., Philos. Trans. R. Soc. London SerA, 317:415 (1986)), PCR mutagenesis, or other known techniques can be performed on the cloned DNA to produce the pcptidc or composition of the invention.

Another subject of the invention relates to a composition comprising at least two proteins selected from the group consisting of
i) a protective protein comprising or consisting of the antigenic peptide of subgroup i);
ii) a protective protein comprising or consisting of the antigenic peptide of subgroup ii); and
iii) a supportive protein comprising or consisting of the supportive peptide of the SEQ ID NO:3 or a functionally active variant of the supportive peptide,
wherein the at least two proteins are selected from at least two of the subgroups i), ii) and iii).

The supportive peptide consisting of the amino acid sequence of SEQ ID NO:3 is derived from pneumococcal surface adhesin A (PsaA) from *S*. *pneumoniae.* The amino acid and DNA sequences of the full length protein from which the supportive peptide consisting of the amino acid sequence of the SEQ ID NO:3 is derived is disclosed in US 5,854,416 as SEQ ID NO:2 and 1 (GenBank Accession numbers: AAE22907 and AR069091). Throughout the entire description of the present invention (including the Figures), PsaA may further be denoted as SP1650.

The amino acid sequence of SEQ ID NO:3 is disclosed in the Examples as well as in the attached Sequence Listing. The peptide of SEQ ID NO:3 has been shown to support induction of a protective immune response against different serotypes and/or to show protection against *S. pneumoniae* in a sepsis and/or pneumonia model (see Examples), if used in combination with the antigenic peptides of the invention.

Functionally active variants may be obtained by changing the sequence of the supportive peptide as defined below and are characterized by having a supportive activity similar to that displayed by the supportive peptide of the sequence of SEQ ID NO:3 from which the variant is derived, including the ability to induce protective immune responses and/or to show protection against *S. pneumoniae* e.g. in a sepsis and/or pneumonia model, wherein any variant may be tested in any of the tests described in the Examples.

The functionally active variant of a supportive peptide may be obtained by sequence alterations in the supportive peptide, wherein the peptide with the sequence alterations retains a function of the unaltered protective peptide, e.g. having a biological activity similar to that displayed by the unaltered supportive peptide (see above). Such sequence alterations can include, but are not limited to, (conservative) substitutions, deletions, mutations and insertions. For further details on alterations and variants see above.

In a preferred embodiment the functionally active variant of the supportive peptide
a) is a functionally active fragment of the supportive peptide, the functionally active fragment comprising at least 60% of the sequence of the supportive peptide, preferably at least 70%, more preferably at least 80%, still more preferably at least 90%, even more preferably at least 95% and most preferably at least 97%, 98% or 99%;
b) is derived from the supportive peptide by at least one amino acid substitution, addition and/or deletion and has a sequence identity to the supportive peptide or to the functionally active fragment as defined in a) of at least 60%, preferably at least 70%, more preferably at least 80%, still more preferably at least 90%, even more preferably at least 95% and most preferably at least 97%, 98% or 99%; and/or
c) consists of the supportive pcptidc or a functionally active variant thereof, preferably the variant of a) and/or b), and additionally at least one amino acid heterologous to the supportive peptide.

The functionally active variant of the invention is characterized by having a biological activity similar to that displayed by the supportive peptide, including the ability to support induction of a protective immune response against different serotypes and/or protection against *S. pneumoniae* in a sepsis and/or pneumonia model (see Examples), if used in combination with the antigenic peptides of the invention.

The variant of the supportive peptide is functionally active in the context of the present invention, if the activity of the variant in combination with the antigenic peptide(s) of the invention amounts to at least 10%, preferably at least 25%, more preferably at least 50%, even more preferably at least 70%, still more preferably at least 80%, especially at least 90%, particularly at least 95%, most preferably at least 99% of the activity of the supportive peptide in combination with the antigenic pcptidc(s) of the invention without sequence alteration. The activity of the variant in combination with the antigenic peptide(s) of the invention may be determined or measured as described in the Examples and then compared to that obtained for the supportive peptide of the amino acid sequence of SEQ ID NO:3 in combination with the antigenic peptide(s) of the invention.

The functionally active fragment of the supportive peptide is characterized by being derived from the supportive peptide of SEQ ID NO:3 by one or more deletions resulting in a peptide comprising at least 60% of the sequence of the supportive peptide, preferably at least 70%, more preferably at least 80%, still more preferably at least 90%, even more preferably at least 95% and most preferably at least 97%, 98% or 99%. Sequence identity may be determined as described above. The deletion(s) may be C-terminally, N-terminally and/or internally. Preferably the fragment is obtained by 1, 2, 3, 4, 5, 6, 7, 8, 9 or 10, more preferably 1, 2, 3, 4 or 5, even more preferably 1, 2 or 3, still more preferably 1 or 2, most preferably 1 deletion(s).

Alternatively or additionally the variant may be obtained from the supportive peptide by at least one amino acid substitution, addition and/or deletion, wherein the functionally active variant has a sequence identity to the supportive peptide or to the functionally active fragment as defined in a) of at least 60%, preferably at least 70%, more preferably at least 80%, still more preferably at least 90%, even more preferably at least 95% and most preferably at least 97%, 98% or 99%. Sequence identity may be determined as described above. The substitution(s), addition(s) and/or deletion(s) may be C-terminally, N-terminally and/or internally. Preferably the functionally active variant is obtained from the supportive peptide or the fragment, preferably the protective peptide, by 1, 2, 3, 4, 5, 6, 7, 8, 9 or 10, more preferably 1, 2, 3, 4 or 5, even more preferably 1, 2 or 3, still more preferably 1 or 2, most preferably 1 amino acid substitution(s), addition(s) and/or deletion(s).

Furthermore, the variant may consist of the supportive peptide or the functionally active variant thereof, preferably the variant of a) and/or b), and at least one amino acid residue heterologous to the supportive peptide or variant thereof, such as a marker protein. The feature "heterologous amino acid" or "amino acid hctcrologous to the supportive peptide or variant thereof" refers to any amino acid which is different from that amino acid located adjacent to the supportive protein in any naturally occurring protein of *S. pneumoniae,* especially the sequence made reference to above. The one or more additional amino acids may be C-terminally, N-terminally or C- and N-terminally to the supportive peptide or variant thereof.

In a preferred embodiment the composition of the invention is defined in that two or more proteins of the at least two proteins are combined into one fusion protein. Accordingly, the two or more proteins may be combined into a fusion protein. The resulting fusion protein may encompass two or more of the proteins of subgroups i), ii) and iii) as defined above.

Examples of such fusion proteins include fusion proteins of the following two components (from N- to C-terminus):
- Protein of SEQ ID NO:1 - Protein of SEQ ID NO:2;
- Protein of SEQ ID NO:1 - Variant of protein of SEQ ID NO:2;
- Protein of SEQ ID NO:1 - Protein of SEQ ID NO:3;
- Protein of SEQ ID NO:1 - Variant of protein of SEQ ID NO:3;
- Protein of SEQ ID NO:2 - Protein of SEQ ID NO:1;
- Protein of SEQ ID NO:2 - Variant of protein of SEQ ID NO:1;
- Protein of SEQ ID NO:2 - Protein of SEQ ID NO:3;
- Protein of SEQ ID NO:2 - Variant of protein of SEQ ID NO:3;
- Protein of SEQ ID NO:3 - Protein of SEQ ID NO:1;
- Protein of SEQ ID NO:3 - Variant of protein of SEQ ID NO:1;
- Protein of SEQ ID NO:3 - Protein of SEQ ID NO:2;
- Protein of SEQ ID NO:3 - Variant of protein of SEQ ID NO:2;
- Variant of protein of SEQ ID NO:1 - Protein of SEQ ID NO:2;
- Variant of protein of SEQ ID NO:1 - Variant of protein of SEQ ID NO:2;
- Variant of protein of SEQ ID NO:1 - Protein of SEQ ID NO:3;
- Variant of protein of SEQ ID NO:1 - Variant of protein of SEQ ID NO:3;
- Variant of protein of SEQ ID NO:2 - Protein of SEQ ID NO:1;
- Variant of protein of SEQ ID NO:2 - Variant of protein of SEQ ID NO:1;
- Variant of protein of SEQ ID NO:2 - Protein of SEQ ID NO:3;
- Variant of protein of SEQ ID NO:2 - Variant of protein of SEQ ID NO:3;
- Variant of protein of SEQ ID NO:3 - Protein of SEQ ID NO:1;
- Variant of protein of SEQ ID NO:3 - Variant of protein of SEQ ID NO:1;
- Variant of protein of SEQ ID NO:3 - Protein of SEQ ID NO:2; or
- Variant of protein of SEQ ID NO:3 - Variant of protein of SEQ ID NO:2.

Examples of fusion proteins include fusion proteins of the following three components (from N- to C-terminus):
- Protein of SEQ ID NO:1 - Protein of SEQ ID NO:2 - Protein of SEQ ID NO:3;
- Protein of SEQ ID NO:1 - Protein of SEQ ID NO:2 - Variant of protein of SEQ ID NO:3;
- Protein of SEQ ID NO:1 - Variant of protein of SEQ ID NO:2 - Protein of SEQ ID NO:3;
- Protein of SEQ ID NO:1 - Variant of protein of SEQ ID NO:2 - Variant of protein of SEQ ID NO:3;
- Variant of protein of SEQ ID NO:1 - Protein of SEQ ID NO:2 - Protein of SEQ ID NO:3;
- Variant of protein of SEQ ID NO:1 - Protein of SEQ ID NO:2 - Variant of protein of SEQ ID NO:3;
- Variant of protein of SEQ ID NO:1 - Variant of protein of SEQ ID NO:2 - Protein of SEQ ID NO:3; or
- Variant of protein of SEQ ID NO:1 - Variant of protein of SEQ ID NO:2 - Variant of protein of SEQ ID NO:3.

In the above fusion proteins composed of three components a protein/variant of SEQ ID NO:1 is in the first position, a protein/variant of SEQ ID NO:2 is in the second position and a protein/variant of SEQ ID NO:3 is in third position of the fusion protein (from N- to C-terminus). However, similar fusion proteins can be prepared according to the following principle:

| 1^{st} position | 2^{nd} position | 3^{rd} position |
|---|---|---|
| 1 | 3 | 2 |
| 2 | 1 | 3 |
| 2 | 3 | 1 |
| 3 | 1 | 2 |
| 3 | 2 | 1 |

wherein
1 denotes a protein or variant of SEQ ID NO:1,
2 denotes a protein or variant of SEQ ID NO:2, and
3 denotes a protein or variant of SEQ ID NO:3.

The fusion protein may comprise or consist of two or more proteins as defined above. Additionally, the fusion protein may encompass a linker, such as a protein linker, to connect the two or more proteins or additional C- or N-terminal sequences, such as a tag in order to purify the fusion protein. Additional sequences may also result from genetic engineering and the use of suitable restriction sites when preparing the nucleic acid sequences underlying the fusion protein.

A protein of SEQ ID NO:1, 2 or 3 is intended to relate to a protective/supportive protein comprising or consisting of a peptide of SEQ ID NO:1, 2 or 3, respectively, as defined above. A variant of a protein of SEQ ID NO:1, 2 or 3 is intended to relate to a protective/supportive protein comprising or consisting of a functionally active variant of SEQ ID NO:1, 2 or 3, respectively, as defined above. However, the above-mentioned fusion proteins of three proteins/variants are preferred.

Preferred sequences of these fusion proteins are shown in the Table 1, i.e. the amino acid sequences of SEQ ID NO:4, SEQ ID NO:5, SEQ ID NO:6 and SEQ ID NO:7.

The proteins of subgroup i), ii) and/or iii) combined in a fusion protein may be directly joined to each other or may be combined over a linker. The linker may be e.g. a short amino acid sequence. The linker may result from the genetic engineering of a suitable fusion protein or may be introduced in order to allow the single proteins to operate effectively.

In another embodiment of the invention the composition may comprise at least one further protein of subgroup i), ii) and/or iii) in addition to the fusion protein as detailed above.

In another preferred embodiment ofthc composition of the invention comprises
- at least one protein as defined in i) and at least one protein as defined in ii); or
- at least one protein as defined in i) and at least one protein as defined in iii); or
- at least one protein as defined in ii) and at least one protein as defined in iii); or
- at least one protein as defined in i) and at least one protein as defined in ii) and at least one protein as defined in iii).

Examples of compositions include of the following proteins:
- Protein of SEQ ID NO:1 and Protein of SEQ ID NO:2;
- Protein of SEQ ID NO:1 and Protein of SEQ ID NO:3;
- Protein of SEQ ID NO:2 and Protein of SEQ ID NO:3;
- Protein of SEQ ID NO:1 and Protein of SEQ ID NO:2 and Protein of SEQ ID NO:3;
- Protein of SEQ ID NO: and Protein of SEQ ID NO:2 and Variant of protein of SEQ ID NO:3;
- Variant of protein of SEQ ID NO:1 and Variant of protein of SEQ ID NO:2;
- Variant of protein of SEQ ID NO:1 and Variant of protein of SEQ ID NO:3;
- Variant of protein of SEQ ID NO:2 and Variant of protein of SEQ ID NO:3;
- Variant of protein of SEQ ID NO:1 and Variant of protein of SEQ ID NO:2 and Variant of protein of SEQ ID NO:3;
- Variant of protein of SEQ ID NO:1 and Variant of protein of SEQ ID NO:2 and Protein of SEQ ID NO:3;
- Protein of SEQ ID NO:1 and Variant of protein of SEQ ID NO:2;
- Protein of SEQ ID NO:1 and Variant of protein of SEQ ID NO:3;
- Protein of SEQ ID NO:2 and Variant of protein of SEQ ID NO:3;
- Protein of SEQ ID NO:1 and Variant of protein of SEQ ID NO:2 and Protein of SEQ ID NO:3;
- Protein of SEQ ID NO:1 and Variant of protein of SEQ ID NO:2 and Variant of protein of SEQ ID NO:3;
- Variant of protein of SEQ ID NO:1 and Protein of SEQ ID NO:2;
- Variant of protein of SEQ ID NO:1 and Protein of SEQ ID NO:3;
- Variant of protein of SEQ ID NO:2 and Protein of SEQ ID NO:3;
- Variant of protein of SEQ ID NO:1 and Protein of SEQ ID NO:2 and Protein of SEQ ID NO:3; or
- Variant of protein of SEQ ID NO:1 and Protein of SEQ ID NO:2 and Variant of protein of SEQ ID NO:3.

A protein of SEQ ID NO:1, 2 or 3 is intended to relate to a protective/supportive protein comprising or consisting of a peptide of SEQ ID NO: 1, 2 or 3, respectively, as defined above. A variant of a protein of SEQ ID NO:1, 2 or 3 is intended to relate to a protective/supportive protein comprising or consisting of a functionally active variant of SEQ ID NO:1, 2 or 3, respectively, as defined above. However, the above-mentioned combinations of three proteins/variants are preferred.

More preferably, the composition of the invention as defined in any of the above embodiments comprises
- at least one protein comprising or consisting of the amino acid sequence of SEQ ID NO:1 and at least one protein comprising or consisting of the amino acid sequence of SEQ ID NO:2, or
- at least one protein comprising or consisting of the amino acid sequence of (SEQ ID NO:1 and at least one protein comprising or consisting of the amino acid sequence of SEQ ID NO:3; or
- at least one protein comprising or consisting of the amino acid sequence of SEQ ID NO:2 and at least one protein comprising or consisting of the amino acid sequence of SEQ ID NO:3; or
- at least one protein comprising or consisting of the amino acid sequence of SEQ ID NO: and at least one protein comprising or consisting of the amino acid sequence of SEQ ID NO:2 and at least one protein comprising or consisting of the amino acid sequence of SEQ ID NO:3.

The last combination is the most preferred one of these combinations.

Examples of compositions include the following proteins:
- Protein of SEQ ID NO:1 and Protein of SEQ ID NO:2;
- Protein of SEQ ID NO:1 and Protein of SEQ ID NO:3;
- Protein of SEQ ID NO:2 and Protein of SEQ ID NO:3; and
- Protein of (SEQ ID NO:1 and Protein of SEQ ID NO:2 and Protein of (SEQ ID NO:3.

A protein of SEQ ID NO:1, 2 or 3 is intended to relate to a protective/supportive protein comprising or consisting of a peptide of SEQ ID NO:1, 2 or 3, respectively, as defined above. However, the last combination is the preferred one.

Still another subject of the invention relates to one or more nucleic acid(s) encoding any of the protective peptides of the invention as defined above or any functionally active variant thereof as defined above or the at least two proteins comprised in the composition of the invention as defined above.

Nucleic acid molecules of the present invention may be in the form of RNA, such as mRNA or cRNA, or in the form of DNA, including, for instance, cDNA and genomic DNA e.g. obtained by cloning or produced by chemical synthetic techniques or by a combination thereof The DNA may be triple-stranded, double-stranded or single-stranded. Single-stranded DNA may be the coding strand, also known as the sense strand, or it may be the non-coding strand, also referred to as the anti-sense strand. Nucleic acid molecule as used herein also refers to, among other, single- and double-stranded DNA, DNA that is a mixture of single- and double-stranded RNA, and RNA that is a mixture of single- and double-stranded regions, hybrid molecules comprising DNA and RNA that may be single-stranded or, more typically, doublc-strandcd, or triple-stranded, or a mixture of single- and double-stranded regions. In addition, nucleic acid molecule as used herein refers to triple-stranded regions comprising RNA or DNA or both RNA and DNA.

The nucleic acid may be a fragment of a nucleic acid occurring naturally in *S. pneumoniae,* especially in *S. pneumoniae* serotype R6, T4, 1, 2, 3, 4, 5, 6A, 6B, 7F, 8, 9N, 9V, 10A, 11A, 12F, 14, 15B, 17F, 18C, 19 such as 19A or 19F, 20, 22F, 23F or 33F; especially the serotype is T4, 6B, 14, 19 or 23F.

The nucleic acid also includes sequences that are a result of the degeneration of the genetic codc. There arc 20 natural amino acids, most of which arc spccificd by morc than one codon. Therefore, all nucleotide sequences are included in the invention encoding the peptide as defined above.

Preferably, the one or more nucleic acid(s) comprise(s) or consist(s) of at least one nucleic acid sequence selected from the group consisting of SEQ ID NO:8, SEQ ID NO:9, SEQ ID NO:10, SEQ ID NO:11, SEQ ID NO:12, SEQ ID NO:13 and SEQ ID NO:14.

Additionally, the nucleic acid may contain one or more modified bases. Such nucleic acids may also contain modifications e.g. in the ribose-phosphate backbone to increase stability and half life of such molecules in physiological environments. Thus, DNAs or RNAs with backbones modified for stability or for other reasons are "nucleic acid molecule" as that feature is intended herein. Moreover, DNAs or RNAs comprising unusual bases, such as inosine, or modified bases, such as tritylatcd bases, to name just two examples, are nucleic acid molecule within the context of the present invention. It will be appreciated that a great variety of modifications have been made to DNA and RNA that serve many useful purposes known to those of skill in the art. The term nucleic acid molecule as it is employed herein embraces such chemically, enzymatically or metabolically modified forms of nucleic acid molecule, as well as the chemical forms of DNA and RNA characteristic of viruses and cells, including simple and complex cells, *inter alia.* For example, nucleotide substitutions can be made which do not affect the peptide or protein or composition of the invention encoded by the nucleic acid, and thus any nucleic acid molecule which encodes an antigenic pcptidc or functionally active variant thereof or a composition of the invention as defined above is encompassed by the present invention.

Furthermore, any of the nucleic acid molecules encoding an antigenic peptide or composition of the invention can be functionally linked, using standard techniques such as standard cloning techniques, to any desired regulatory sequences, whether a *S. pneumoniae* regulatory sequence or a heterologous regulatory sequence, heterologous leader sequence, heterologous marker sequence or a heterologous coding sequence to create a fusion protein.

The nucleic acid of the invention may be originally formed *in vitro* or in a cell in culture, in general, by the manipulation of nucleic acids by endonucleases and/or exonucleases and/or polymerases and/or ligases and/or recombinases or other methods known to the skilled practitioner to produce the nucleic acids.

In one embodiment of the invention, the one or more nucleic acid(s) of invention is/are located in a vector or a cell other than *S*. *pneumonia.*

A vector may additionally include nucleic acid sequences that permit it to replicate in the host cell, such as an origin of replication, one or more desired genes and/or selectable marker genes and other genetic elements known in the art such as regulatory elements directing transcription, translation and/or secretion of the encoded peptide or protein. The vector may be used to transduce, transform or infect a cell, thereby causing the cell to express inserted nucleic acids and/or proteins other than those native to the cell. The vector optionally includes materials to aid in achieving entry of the nucleic acid into the cell, such as a viral particle, liposome, protein coating or the like. Numerous types of appropriate expression vectors arc known in the art for protein expression, by standard molecular biology techniques. Such vectors are selected from among conventional vector types including insects, e.g., baculovirus expression, or yeast, fungal, bacterial or viral expression systems. Other appropriate expression vectors, of which numerous types are known in the art, can also be used for this purpose. Methods for obtaining such expression vectors are well-known (see, e.g. Sambrook et al, Molecular Cloning. A Laboratory Manual, 2nd edition, Cold Spring Harbor Laboratory, New York (1989)). In one embodiment, the vector is a viral vector. Viral vectors include, but arc not limited to, rctroviral and adcnoviral vectors.

Suitable host cells or cell lines for transfection by this method include bacterial cells. For example, the various strains of *E*. *coli* are well-known as host cells in the field of biotechnology. Various strains of *B. subtilis, Pseudomonas, Streptomyces,* and other bacilli and the like may also be employed in this method. Many strains of yeast cells known to those skilled in the art are also available as host cells for expression of the peptides of the present invention. Other fungal cells or insect cells such as *Spodoptera frugipedera* (Sf9) cells may also be employed as expression systems. Alternatively, mammalian cells, such as human 293 cells, Chinese hamster ovary cells (CHO), the monkey COS-1 cell line or murinc 3T3 cells derived from Swiss, BALB/c or NIH mice may be used. Still other suitable host cells, as well as methods for transfection, culture, amplification, screening, production, and purification are known in the art.

An antigenic peptide or composition of the invention or component thereof may be produced by expressing a nucleic acid of the invention in a suitable host cell. The host cells can be transfected, e.g. by conventional means such as electroporation with at least one expression vector containing a nucleic acid of the invention under the control of a transcriptional regulatory sequence. The transfected or transformed host cell is then cultured under conditions that allow expression of the protein. The expressed protein is recovered, isolated, and optionally purified from the cell (or from the culture medium, if expressed extracellularly) by appropriate means known to one of skill in the art. For example, the proteins are isolated in soluble form following cell lysis, or extracted using known techniques, e.g. in guanidine chloride. If desired, the peptides or fragments of the invention are produced as a fusion protein. Such fusion proteins are those described above. Alternatively, for example, it may be desirable to produce fusion proteins to enhance expression of the protein in a selected host cell or to improve purification. The molecules comprising the peptides and compositions of this invention may be further purified using any of a variety of conventional methods including, but not limited to: liquid chromatography such as normal or reversed phase, using HPLC, FPLC and the like; affinity chromatography (such as with inorganic ligands or monoclonal antibodies); size exclusion chromatography; immobilized metal chelate chromatography; gel electrophoresis; and the like. One of skill in the art may select the most appropriate isolation and purification techniques without departing from the scope of this invention. Such purification provides the peptide/protein/composition in a form substantially free from other proteinaceous and non-proteinaceous materials of the microorganism.

A further subject of the invention relates to a pharmaceutical composition, especially a vaccine, comprising at least one protective peptide or functionally active variant thereof according to the invention or the composition according to the invention, and optionally a pharmaceutically acceptable carrier or excipient.

An antigenic peptide or composition of the invention may be used for methods for immunizing or treating humans and/or animals with the disease caused by infection with *S. pneumoniae.* Therefore, the antigenic peptide or composition may be used within a pharmaceutical composition. The pharmaceutical composition of the present invention may further encompass pharmaceutically acceptable carriers and/or excipients. The pharmaceutically acceptable carriers and/or excipients useful in this invention are conventional and may include buffers, stabilizers, diluents, preservatives, and solubilizers. Remington's Pharmaceutical Sciences, by E. W. Martin, Mack Publishing Co., Easton, PA, 15th Edition (1975), describes compositions and formulations suitable for pharmaceutical delivery of the (poly)peptides/proteins herein disclosed.

If the pharmaceutical composition comprises the components of the invention, the proteins of subgroup i), ii) and/or iii) may be formulated into one or more pharmaceutical composition(s). Additionally, the two or more pharmaceutical compositions may be administered together, simultaneously or consecutively.

In general, the nature of the carrier or excipients will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions (e. g. powder, pill, tablet, or capsule forms), conventional non-toxic solid carriers can include, for example, pharmaceutical grades of mannitol, lactose, starch, or magnesium stearate. In addition to biologically neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

In a preferred embodiment the pharmaceutical composition further comprises an immunostimulatory substance such as an adjuvant. The adjuvant can be selected based on the method of administration and may include mineral oil-based adjuvants such as Freund's complete and incomplete adjuvant, Montanide incomplete Seppic adjuvant such as ISA, oil in water emulsion adjuvants such as the Ribi adjuvant system, syntax adjuvant formulation containing muramyl dipeptide, or aluminum salt adjuvants. Preferably, the adjuvant is a mineral oil-based adjuvant, most preferably 1SA206 (SEPPIC, Paris, France).

In a more preferred embodiment the immunostimulatory substance is selected from the group comprising polycationic polymers, especially polycationic peptides such as polyarginine, immunostimulatory deoxynucleotides (ODNs), especially Oligo(dIdC)₁₃, peptides containing at least two LysLeuLys motifs, especially KLKLLLLLKLK, neuroactive compounds, especially human growth hormone, alumn, adjuvants and combinations thereof. Preferably the combination is either a polycationic polymer and immunostimulatory deoxynucleotides or a peptide containing at least two LysLeuLys motifs and immunostimulatory deoxynucleotides. In a still more preferred embodiment the polycationic polymer is a polycationic peptide.

The term "Oligo(dIdC)₁₃" as used in the present invention means a phosphodiester backboned single-stranded DNA molecule containing 13 deoxy (inosine-cytosine) motifs, also defined by the term [oligo-d(IC)₁₃]. The exact sequence is 5'-dIdCdIdCdIdCdIdCdIdCdIdCdIdCdIdCdIdCdIdCdIdCdIdCdIdC-3'. Oligo(dIdC)₁₃ can also be defined by the terms (oligo-dIC₂₆); oligo-dIC₂₆₋ₘₑᵣ; oligo-dcoxy IC, 26-mcr; or oligo-dIC, 26-mer, as specified for example in WO 01/93903 and WO 01/93905.

In an even more preferred embodiment of the invention the immunostimulatory substance is at least one immunostimulatory nucleic acid. Immunostimulatory nucleic acids are e.g. neutral or artificial CpG containing nucleic acids, short stretches of nucleic acids derived from non-vertebrates or in form of short oligonucleotides (ODNs) containing non-methylated cytosine-guanine dinucleotides (CpG) in a defined base context (e.g. as described in WO 96/02555). Alternatively, also nucleic acids based on inosine and cytidine as e.g. described in WO 01/93903, or deoxynucleic acids containing deoxy-inosine and/or deoxyuridine residues (described in WO 01; 93905 and WO 02/095027) may preferably be used as immunostimulatory nucleic acids in the present invention. Preferably, mixtures of different immunostimulatory nucleic acids are used in the present invention. Additionally, the aforementioned polycationic compounds may be combined with any of the immunostimulatory nucleic acids as aforementioned. Preferably, such combinations are according to the ones described in WO 01/93905, WO 02/32451, WO 01/54720, WO 01/93903, WO 02/13857 and WO 02/095027 and WO 03/047602.

In addition or alternatively, such vaccine composition may comprise a neuroactive compound. Preferably, the neuroactive compound is human growth factor, e.g. described in WO 01/24822. Also preferably, the neuroactive compound is combined with any of the polycationic compounds and/or immunostimulatory nucleic acids as defined above.

In a highly preferred embodiment of the invention, the adjuvants are those used in the Example, e.g. Complete Freund's adjuvant, aluminum hydroxide or IC31® (Intercell; a synthetic adjuvant comprising the peptide motif KLK [WO 02/32451] and an oligonucleotide [WO 01/93905]).

The composition may be used e.g. for immunization or treatment of a subject. The pharmaceutical composition encompasses at least one antigenic peptide or composition of the invention; however, it may also contain a cocktail (i.e., a simple mixture) containing different peptides (including fragments and variants) or proteins or compositions of the invention, optionally mixed with a supportive peptide or protein or different antigenic pcptidcs or proteins of other pathogens. Such mixtures of these pcptidcs, polypeptides, proteins or fragments or variants thereof are useful e.g. in the generation of desired antibodies to a wide spectrum of *S*. *pneumoniae* isolates. The (poly)peptide(s)/composition(s) of the present invention may also be used in the form of a pharmaceutically acceptable salt. Suitable acids and bases which are capable of forming salts with the peptides of the present invention are well known to those of skill in the art, and include inorganic and organic acids and bases.

Alternatively, the pharmaceutical composition comprises
(i) the one or more nucleic acid(s) of the invention or one or more nucleic acid(s) complementary thereto, and
(ii) optionally a pharmaceutically acceptable carrier or excipient.

The nucleic acid sequences, alone or in combination with other nucleic acid sequences encoding peptides/proteins/compositions or antibodies or directed to other pathogenic microorganisms, may further be used as components of a pharmaceutical composition. The composition may be used for immunizing or treating humans and/or animals with the disease caused by infection with *S. pneumoniae.*

The pharmaceutically acceptable carrier or excipient may be as defined above.

In another embodiment, the nucleic acid sequences of this invention, alone or in combination with nucleic acid sequences encoding other antigens or antibodies from other pathogenic microorganisms, may further be used in compositions directed to actively induce a protective immune response in a subject to the pathogen. These components of the present invention are useful in methods for inducing a protective immune response in humans and/or animals against infection with *S. pneumoniae.*

For use in the preparation of the therapeutic or vaccine compositions, nucleic acid delivery compositions and methods are useful, which are known to those of skill in the art. The nucleic acid of the present invention or one or more nucleic acid(s) complementary thereto may be employed in the methods of this invention or in the compositions described herein as DNA sequences, either administered as naked DNA, or associated with a pharmaceutically acceptable carrier and provide for *in vivo* expression of the antigen, peptide or polypeptide. So-called "naked. DNA" may be used to express the antigenic peptide or composition of the invention *in vivo* in a patient. (See, e.g., J. Cohen, Science, 259:1691-1692, which describes similar uses of "naked DNA"). For example, "naked DNA" associated with regulatory sequences may be administered therapeutically or as part of the vaccine composition e.g., by injection.

Alternatively, a nucleic acid encoding the antigenic pcptidcs or compositions of the invention or a nucleic acid complementary thereto may be used within a pharmaceutical composition, e.g. in order to express the antigenic peptide or composition of the invention *in vivo,* e.g., to induce antibodies.

A preferred embodiment of the invention relates to a pharmaceutical composition, wherein the nucleic acid is comprised in a vector and/or a cell other than *S. pneumoniae.* Vectors and cells suitable in the context of the present invention arc described above. Vectors arc particularly employed for a DNA vaccine. An appropriate vector for delivery may be readily selected by one of skill in the art. Exemplary vectors for *in vivo* gene delivery are readily available from a variety of academic and commercial sources, and include, e.g., adeno-associated virus (International patent application No. PCT/US91/03440), adenovirus vectors (M. Kay et al, Proc. Natl. Acad. Sci. USA, 91:2353 (1994); S. Ishibashi et al, J. Clin. Invest., 92:883 (1993)), or other viral vectors, e.g., various poxviruses, vaccinia, etc.. Recombinant viral vectors, such as retroviruses or adenoviruses, are preferred for integrating the exogenous DNA into the chromosome of the cell.

Also included in the scope of the invention is the production of antibodies against an antigenic peptide or composition according to the invention. This includes, for example, monoclonal and polyclonal antibodies, chimeric, single chain, and humanized antibodies, as well as Fab fragments, or the product of a Fab expression library, which are able to specifically bind to the antigenic peptide or composition according to the invention.

In a preferred embodiment the antibody is a monoclonal, polyclonal, chimeric or humanized antibody or functionally active fragment thereof. In another preferred embodiment the functionally active fragment comprises a Fab fragment.

Antibodies generated against the antigenic peptide or composition according to the invention can be obtained by direct injection of the antigenic peptide or composition according to the invention into an animal or administering of the antigenic peptide or composition according to the invention to an animal, preferably a non-human. The antibody so obtained will then bind the antigenic peptide or composition according to the invention. Such antibodies can then be used to isolate reactive antigens, peptide or proteins from tissue expressing those.

For preparation of monoclonal antibodies, any technique known in the art, which provides antibodies produced by continuous cell line cultures, e.g. a hybridoma cell line, can be used.

Techniques described for the production of single chain antibodies (U. S. Patent No. 4,946,778) can be adapted to produce single chain antibodies to the antigenic pcptidcs or compositions according to the invention. Also, transgenic mice or other organisms such as other mammals may be used to express humanized antibodies to of the antigenic peptides or compositions according to the invention.

Antibodies may be also produced using a hybridoma cell line. Hybridoma cell lines expressing desirable monoclonal antibodies are generated by well-known conventional techniques. The hybridoma cell can be generated by fusing a normal-activated, antibody-producing B cell with a myeloma cell. In the context of the present invention the hybridoma cell is able to produce an antibody specifically binding to the antigenic peptide or composition according to the invention.

Similarly, desirable high titre antibodies are generated by applying known recombinant techniques to the monoclonal or polyclonal antibodies developed to these peptides/proteins/compositions (see, e.g., PCT Patent Application No. PCT/GB85/00392; British Patent Application Publication No. GB2188638A; Amit et al., Science, 233:747-753 (1986); Queen ct al., Proc. Natl. Acad. Sci. USA, 86:10029-10033 (1989); PCT Patent Application No. WO90/07861; Riechmann et al., Nature, 332:323-327 (1988); Huse et al., Science, 246:1275-1281 (1988)).

Accordingly, another subject of the invention is a method for producing an antibody, characterized by the following steps:
(a) administering an effective amount of at least one protective peptide or functionally active variant thereof of the invention as defined above and/or a composition of the invention as defined above to an animal; and
(b) isolating the antibody produced by the animal in response to the administration of step (a) from the animal.

An alternative method of the invention for producing an antibody is characterized by the following steps:
(a) contacting a B cell with an effective amount of at least one protective peptide or functionally active variant thereof of the invention as defined above and/or a composition of the invention as defined above;
(b) fusing the B cell of step (a) with a mycloma cell to obtain a hybridoma cell; and
(c) isolating the antibody produced by the cultivated hybridoma cell.

Particularly, the antibody may bc produced by initiating an immune response in a non-human animal by administrating an antigenic peptide or composition of the invention to an animal, removing an antibody containing body fluid from said animal, and producing the antibody by subjecting said antibody containing body fluid to further purification steps. Alternatively, the antibody may be produced by initiating an immune response in a non-human animal by administrating an antigenic peptide or composition, as defined in the present invention, to said animal, removing the spleen or spleen cells from said animal and/or producing hybridoma cells of said spleen or spleen cells, selecting and cloning hybridoma cells specific for the antigenic peptide or composition according to the invention and producing the antibody by cultivation of said cloned hybridoma cells. In a preferred embodiment the antibody produced according to a method of the invention is additionally purified. Methods of purification are known to the skilled artisan.

The antibody may be used in methods for preventing or treating an infection. Accordingly, still another subject of the invention relates to a pharmaceutical composition, especially a vaccine, comprising an antibody produced according to the invention. The pharmaceutical composition may encompass further components as detailed above. The composition may further encompass substances increasing their capacity to stimulate T cells. These include T helper cell epitopes, lipids or liposomes or preferred modifications as described in WO01/78767. Another way to increase the T cell stimulating capacity of epitopes is their formulation with immune stimulating substances for instance cytokines or chemokines like interleukin-2, -7, -12, -18, class I and II interferons (IFN), especially IFN-gamma, GM-CSF, TNF-alpha, flt3-ligand and others.

A further subject of the invention relates to the use of a protective peptide or functionally active variant thereof of the invention as defined above and/or a composition of the invention as defined above and/or the nucleic acid of the invention as defined above for the manufacture of a medicament for the immunization or treatment of a subject, preferably against *S. pneumoniae,* more preferably against pneumonia, bacteremia, otitis media, meningitis, sinusitis, peritonitis and/or arthritis caused by *S. pneumoniae.*

The peptides, proteins, compositions or the nucleic acids of the invention are generally useful for inducing an immune response in a subject. The vaccine used for immunization may be administcrcd to a subject susceptible to infection by *S. pneumoniae,* preferably mammals, and still more preferably humans, in any conventional manner, including oral, topical, intranasal, intramuscular, intra-lymph node, intradermal, intraperitoneal, subcutaneous, and combinations thereof, but most preferably through intramuscular injection. The volume of the dose for intramuscular administration is preferably up to about 5 ml, still more preferably between 0.5 ml and 3 ml, and most preferably about 1 to 2 ml. The volume of the dose when subcutaneous injection is the selected administration route is preferably up to about 5 ml, still more preferably between 0.5 ml and 3 ml, and most preferably about 1 to 2 ml. The amount of substance in each dose should be enough to confer effective immunity against and decrease the risk of developing clinical signs resulting from *S. pneumoniae* infection to a subject receiving a vaccination therewith. Preferably, the unit dose of protein should be up to about 5 µg protein/kg body weight, more preferably between about 0.2 to 3 µg, still more preferably between about 0.3 to 1.5 µg, more preferably between about 0.4 to 0.8 µg, and still more preferably about 0.6 µg. Alternative preferred unit doses of protein could be up to about 6 µg protein/kg body weight, more preferably between about 0.05 to 5 µg, still more preferably between about 0.1 to 4 µg. The dose is preferably administered 1 to 3 times, e.g. with an interval of 1 to 4 weeks. Preferred amounts of protein per dose are from approximately 1 µg to approximately 1 mg, more preferably from approximately 5 µg to approximately 500 µg, still more preferably from approximately 10 µg to approximately 250 µg and most preferably from approximately 25 µg to approximately 100 µg.

In still another aspect of the invention the antibody produced according to the invention or functional fragment thereof is used for the manufacture of a medicament for the treatment of an infection, preferably a *S. pneumoniae* infection. The treatment involves administering an effective amount of the antibody to a subject, preferably a mammal, more preferably a human. Thus, antibodies against the protective peptides or variants thereof or the composition of the present invention may be employed to inhibit and/or treat infections, particularly bacterial infections and especially infections arising from *S*. *pneumonia.*

An "effective amount" of peptides, proteins, compositions or the nucleic acids of the invention or an antibody produced according to the invention may be calculated as that amount capable of exhibiting an *in vivo* effect, e.g. preventing or ameliorating a sign or symptom of infection, particularly *S*. *pneumoniae* infection. Such amounts may be determined by one of skill in the art. Such a substance may be administered in any conventional manner, including oral, topical, intranasal, intramuscular, intra-lymph node, intradermal, intraperitoneal, subcutaneous, and combinations thereof, but preferably intramuscularly or subcutaneously. However, it may also be formulated to be administered by any other suitable route, including orally or topically. The selection of the route of delivery and dosage of such therapeutic compositions is within the skill of the art.

Treatment in the context of the present invention refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented.

Still a further subject of the invention relates to a method of diagnosing a *S. pneumoniae* infection comprising the steps of:
(a) contacting a sample obtained from a subject with a protective peptide or functionally active variant thereof of the invention as defined above and/or a composition of the invention as defined above; and
(b) detecting the presence of an antibody against the protective peptide functionally active variant and/or the composition in the sample,
wherein the presence of the antibody is indicative for the *S*. *pneumonia* infection.

The antigenic pcptidcs or compositions of the invention may be used for the detection of the *S*. *pneumoniae.* Preferably such detection is for diagnosis, more preferably for the diagnosis of a disease, most preferably for the diagnosis of a *S*. *pneumomiae* infection. The antigenic peptides or compositions may be used to detect the presence of a *S*. *pneumoniae-*specific antibody or fragment thereof e.g. in a sample obtained from a subject. The sample may be e.g. a blood sample. Alternatively, the presence of a *S*. *pneumoniae-specific* protective peptide can be detected using an antibody prepared according to the method of the invention.

The present invention also relates to diagnostic assays such as quantitative and diagnostic assays for detecting levels of the peptides, proteins or antibodies of the present invention in cells and tissues or body fluids, including determination of normal and abnormal levels. Assay techniques that can be used to determine levels of a peptide, a composition or an antibody, in a sample derived from a host are well known to those of skill in the art. Such assay methods include radioimmunoassays, competitive-binding assays, Western Blot analysis and ELISA assays. Among these, ELISAs frequently are preferred. An ELISA assay initially comprises preparing an antibody specific to the peptide or composition, particularly the protective peptide, preferably a monoclonal antibody. In addition, a reporter antibody generally is prepared which binds to the monoclonal antibody. The reporter antibody is attached to a detectable reagent such as radioactive, fluorescent or enzymatic reagent, such as horseradish peroxidase enzyme.

The antigenic peptides or compositions of the present invention may also be used for the purpose of or in connection with an array. More particularly, at least one of the antigenic peptides or compositions of the present invention may be immobilized on a support. Said support typically comprises a variety of peptides/proteins whereby the variety may be created by using one or several of the peptides or compositions of the present invention. The characterizing feature of such array as well as of any array in general is the fact that at a distinct or predefined region or position on said support or a surface thereof, a distinct polypeptide is immobilized. Because of this any activity at a distinct position or region of an array can be correlated with a specific polypeptide. The number of different peptides or antibodies of the present invention immobilized on a support may range from as little as 10 to several 1000 different pcptidcs or compositions of the present invention. Alternatively, antibodies produced according to the present invention may be used to detect antigenic peptides or compositions of the invention.

The manufacture of such arrays is known to the one skilled in the art and, for example, described in US patent 5,744,309. The array preferably comprises a planar, porous or nonporous solid support having at least a first surface. Preferred support materials are, among others, glass or cellulose. It is also within the present invention that the array is used for any of the diagnostic applications described herein. Apart from the peptides or antibodies of the present invention also the nucleic acid molecules according to the present invention may be used for the generation of an array as described above.

An alternative method for diagnosing an infection with *S*. *pneumoniae* comprises the steps of:
a) contacting a sample obtained from a subject with a primer and/or a probe specific for the one or more nucleic acid(s) of the invention; and
b) detecting the presence of one or more nucleic acid(s) of the invention in the sample, wherein the presence of the one or more nucleic acid(s) is indicative for the *S*. *pneumoniae* infection.

A series of methods for detecting nucleic acids in probes by using specific primers and/or probes is known in the art. In general, these methods are based on the specific binding of a primer or probe to the nucleic acid in question. The methods may involve amplification of the nucleic acid, e.g. RNA or DNA, before the actual detection step. Therefore, primers may be used to specifically induce transcription and/or amplification of RNA or DNA in order to generate a detectable amount of nucleic acid. Suitable well known techniques may be PCR and RT-PCR. Suitable primers and probes for the method of the invention may be produced based on sequence information provided in the present application. Guidelines and computer-assisted programs (e.g. Primer Express^{®}, Applied Biosystems, Foster City, CA, USA) for designing primers and probes to a specific nucleic acid are known to the person skilled in the art.

After the amplification step the amplified nucleic acid, in general DNA, may be detected e.g. by its size (e.g. involving agarose gel electrophoresis) or using labeled probes which specifically bind to the amplified nucleic acid. The probes may be labeled with a dye, radioactive marker, a fluorescent marker, an enzyme-linked marker or any other marker.

For example, FRET (Förster resonance energy transfer) may be used for the detection of the nucleic acid of the invention. In FRET, a donor fluorophore molecule absorbs excitation energy and delivers this via dipole-dipole interaction to a nearby acceptor fluorophorc molecule. This process only occurs when the donor and acceptor molecules arc sufficiently close to one another. Several different strategies for determining the optimal physical arrangement of the donor and acceptor moieties are known to the skilled practitioner. For this, a fluorescent donor is excited at its specific fluorescence excitation wavelength. By a long-range dipole-dipole coupling mechanism, this excited state is then nonradiatively transferred to a second molecule, the acceptor. The donor returns to the electronic ground state. The described energy transfer mechanism is termed "Förster resonance energy transfer" (FRET). The process involves measuring fluorescence as FRET donor and acceptor moieties are brought together as a result of DNA hybridization. For examples two probes each labeled with a suitable marker hybridize to the nucleic acid of the invention within a distance which allows FRET to occur. Suitable markers include Cyan 500, Cy5, Cy3, SYBR Green I, fluorescein, HEX, Red 610 and Red 640, wherein the two marker involved have to be selected based on there excitation and emission spectrums as known by the skilled person. A suitable system for the detection of nucleic acids is the LightCycler^{®} (Roche Diagnostics).

A further subject of the invention relates to a method for identifying a ligand capable of binding to a protective peptide or variant thereof according to the invention or the composition according to the invention:
(a) providing a test system comprising the peptide and/or composition,
(b) contacting the test system with a test compound, and
(c) detecting a signal generated in response to the binding of the test compound to the peptide and/or composition.

More particularly, the method may be carried out by contacting an isolated or immobilized antigenic peptide or composition according to the invention with a candidate ligand under conditions to permit binding of the candidate ligand to the pcptidc, wherein the test system comprises a component capable of providing a detectable signal in response to the binding of the candidate ligand to said peptide; and detecting the presence or absence of a signal generated in response to the binding of the ligand to the peptide. The ligand may be an agonist or an antagonist.

Test systems for detection binding of a ligand are known to the skilled artisan and include e.g. binding assays with labeled ligand such as radioligands, fluorescence-labeled ligands or enzyme-labeled ligands.

The test compound can be any test compound cithcr naturally occurring or chemically synthesized. Naturally occurring test compounds include in particular antibodies, preferably those showing similarity to the antibodies of the invention. In one preferred embodiment of the invention the test compound is provided in the form of a chemical compound library. Chemical compound libraries include a plurality of chemical compounds and have been assembled from any of multiple sources, including chemically synthesized molecules and natural products, or have been generated by combinatorial chemistry techniques. They are especially suitable for high throughput screening. They may be comprised of chemical compounds of a particular structure or compounds of a particular creature such as a plant.

A further subject of the invention relates to the use of the protective peptide or variant thereof according to the invention or the composition according to the invention for the isolation and/or purification and/or identification of an interaction partner of the antigenic peptide and/or composition. The isolation and/or purification and/or identification of the ligand may be carried out as detailed above or as known to the person skilled in the art. In a preferred embodiment of the invention an affinity device may be used. The affinity device may comprise as least a support material and any antigenic peptide or composition according to the present invention, which is attached to the support material. Because of the specificity of the antigenic peptides and/or compositions according to the present invention for their target cells or target molecules or their interaction partners, the antigenic peptides and/or compositions allow a selective removal of their interaction partner(s) from any kind of sample applied to the support material provided that the conditions for binding arc met. The sample may be a biological or medical sample, including but not limited to, fermentation broth, cell debris, cell preparation, tissue preparation, organ preparation, blood, urine, lymph liquid, liquor and the like. The peptide or composition may be attached to the matrix in a covalent or non-covalent manner. Suitable support material is known to the one skilled in the art and can be selected from the group comprising cellulose, silicon, glass, aluminum, paramagnetic beads, starch and dextrane.

The present invention is further illustrated by the following Figures, Examples and the Sequence Listing, from which further features, embodiments and advantages may be taken. It is to be understood that the present examples are given by way of illustration only and not by way of limitation of the disclosure.

### FIGURES

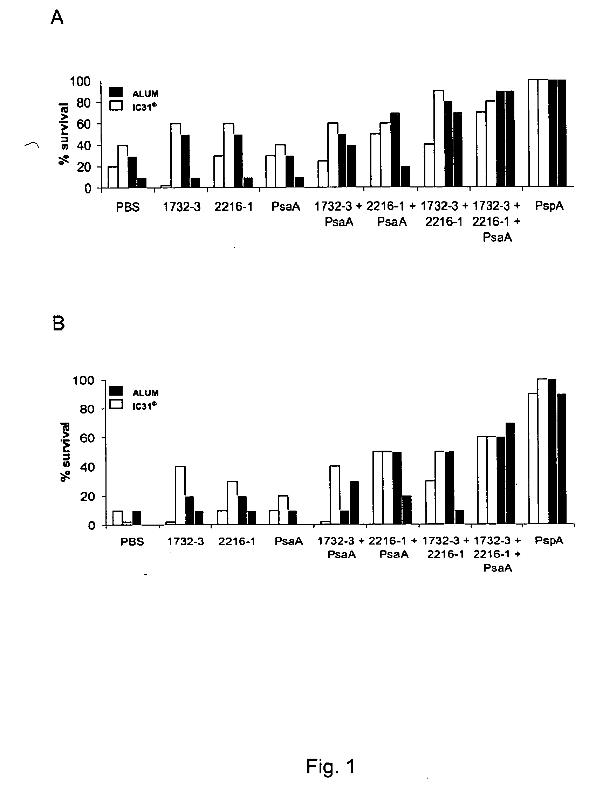

**Figure 1** shows the protection achieved by active immunization with selected *S. pneumoniae* antigens in a mouse lethality model. C3H/HeN mice (10 mice per group) were immunized with recombinant antigens cloned from a serotype 4 or 6B *S. pneumoniae* strain and challenged with a serotype 6B strain. Survival was monitored for 14 days post-challenge. Mice were immunized subcutaneously with 50 µg SP2216-1, SP1732-3, PsaA or combinations of these antigens adjuvanted with either aluminum hydroxide (ALUM) or IC31^{®} (100 nmol KLKLLLLLKLK; 4 nmol ODN1a ([dIdC]₁₃)). Mice were then challenged intraperitoneally with 10⁴ cfu *S. pneumoniae* 6B. Adjuvant control mice were used as negative controls, while PspA (SP0117) served as positive control. Percentage of survival is depicted either on day 7 **(A)** or on day 14 **(B).** Two independent experiments using aluminum hydroxide (black bars) or two independent experiments using IC31^{®} (white bars) as adjuvant are depicted.

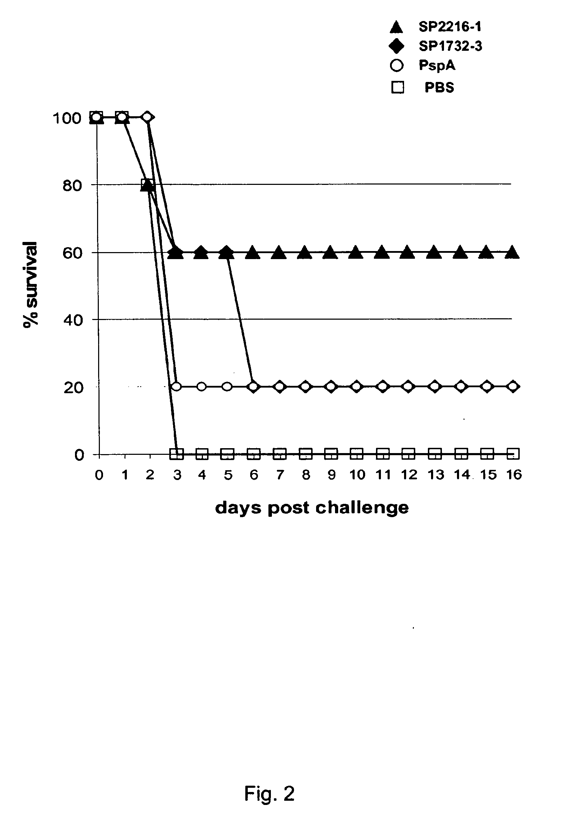

**Figure 2** shows the protection achieved by active immunization with SP1732-3 and SP2216-1 in an intranasal mouse sepsis model. NMRI mice (10 mice per group) were subcutaneously immunized with 50 µg protein antigen adjuvanted with CFA/IFA. Mice were intranasally challenged with 5 x 10⁶ cfu *S*. *pneumoniae* 6301. Adjuvant control mice were used as negative controls, while PspA (SP0117) served as positive control. Survival was monitored for 16 days post challenge and was depicted as percentage of total animals.

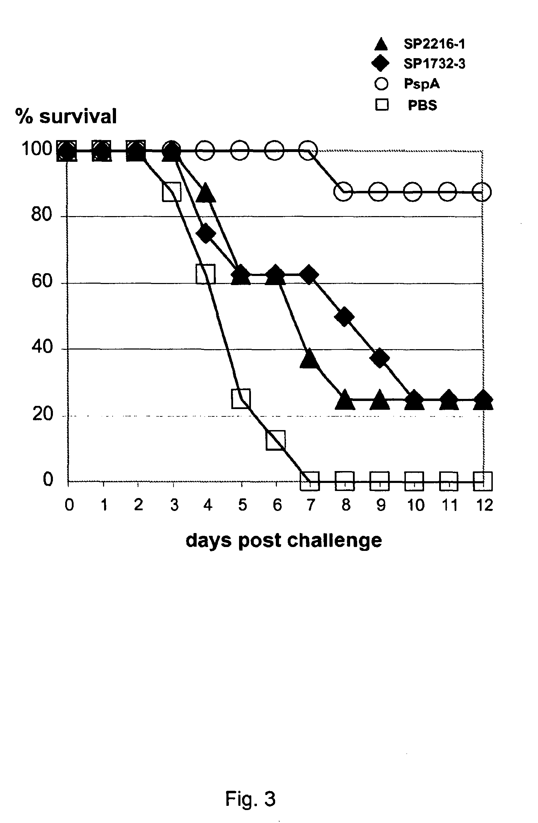

**Figure 3** shows the protection achieved by active immunization with SP1732-3 and SP2216-1 in an intravenous mouse sepsis model. CBA/N mice (10 mice per group) were subcutaneously immunized with 50 µg protein antigen adjuvanted with CFA/IFA. Mice were intravenously challenged with 5 x 10⁴ cfu *S*. *pneumoniae* TIGR4. Adjuvant control mice were used as negative controls, while PspA (SP0117) served as positive control. Survival was monitored for 12 days post challenge and was depicted as percentage of total animals.

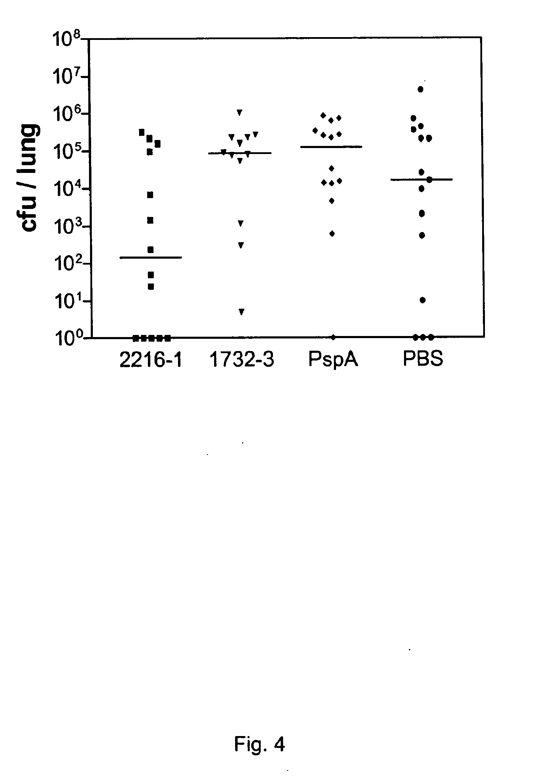

**Figure 4** shows the protection achieved by active immunization with SP1732-3 and SP2216-1 in a pneumonia model. CBA/N mice (10 mice per group) were subcutaneously immunized with 50 µg protein antigen adjuvanted with CFA/IFA. Mice were intravenously challenged with 10⁷ cfu *S*. *pneumoniae* EF3030. Adjuvant control mice were used as negative controls, while PspA (SP0117) served as positive control. As read-out for pneumonia, lungs were removed at day 6 after challenge under sterile conditions, homogenized and cultures of lung homogenates were quantitatively plated on blood agar plates. Cfus per organ were determined for each individual mouse (5 - 10 mice/group).
**Figure 5** shows a sequence alignment of fragment SP2216-1 (SEQ ID NO:1) and the respective fragment derived from six other prevalent serotypes.

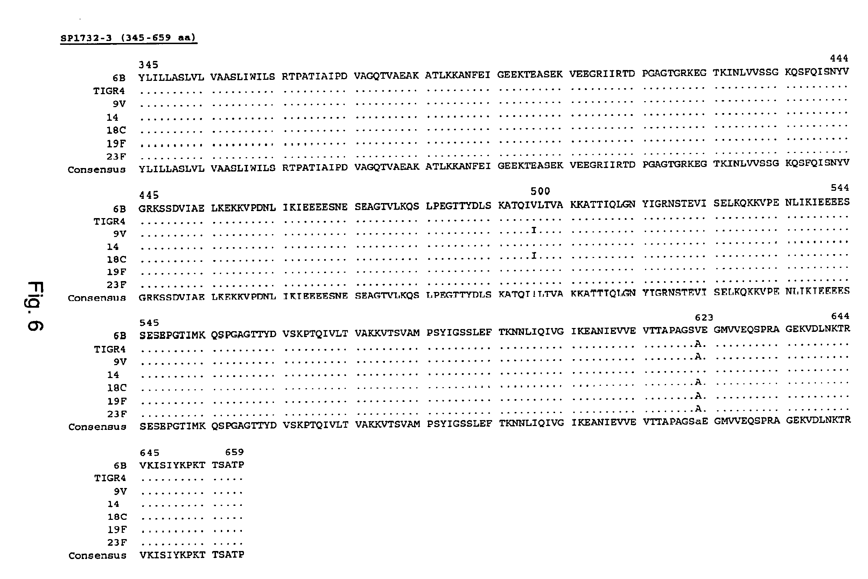

**Figure 6** shows a sequence alignment of fragment SP1732-3 (SEQ ID NO:2) and the respective fragment derived from six other serotypes. The alignment revealed a low level of variability and only two single positions (500 and 623) for variation.

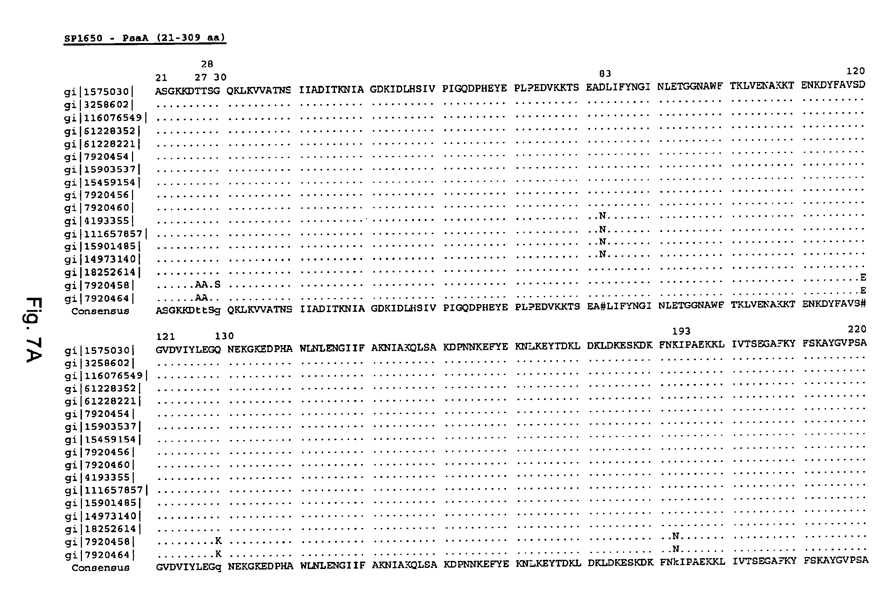

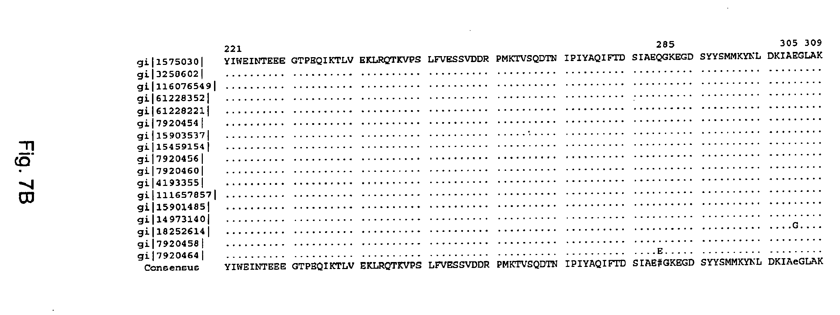

**Figures 7A** and **B** show a sequence alignment of a fragment of PsaA (SEQ ID NO:3) and the respective fragments derived from 16 other serotypes. The sequences on which the alignment is based were available to public, for example originated from a common database, called "non redundant protein database" (freely available from NCBI, Bethesda, MD) and the codes refer to the identifiers of this database. The alignment identifies nine positions displaying variation: 27, 28, 30, 83, 120, 130, 193, 285 and 305.

Multiple sequence alignments of Fig. 5 to 7 were done by the MultAlin tool (http://bioinfo.genopole-toulouse.prd.fr/multalin/multalin.html).

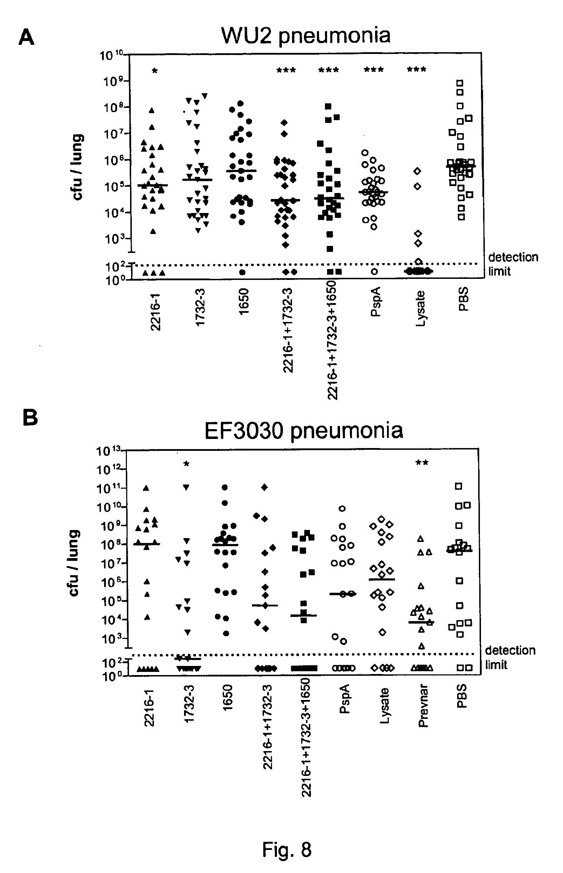

**Figure 8** shows the protection level achieved by active immunization with SP1732-3, SP2216-1 and SP1650 (PsaA) as well as combinations of SP1732-3, SP2216-1 (and SP1650) in a pneumonia model. CD-1 mice (10 mice per group) were subcutaneously immunized with 50 µg protein antigen adjuvanted with aluminum hydroxide (ALUM). Mice were intranasally challenged with **(A)** 10⁵ cfu *S. pneumoniae* WU2 or **(B)** 5 x 10⁷ cfu *S. pneumoniae* EF3030. Adjuvant control mice were used as negative controls, while PspA (SP0117), Prevnar or lysate served as positive control. As read-out for pneumonia, lungs were removed at day 3 after challenge under sterile conditions, homogenized and cultures of lung homogenates were quantitatively plated on blood agar plates. Cfus per organ were determined for each individual mouse. A summary of 2 or 3 experiments is shown (10 mice / group / experiment). Statistical significance (Mann-Whitney two sample rank test) is indicated with star(s).

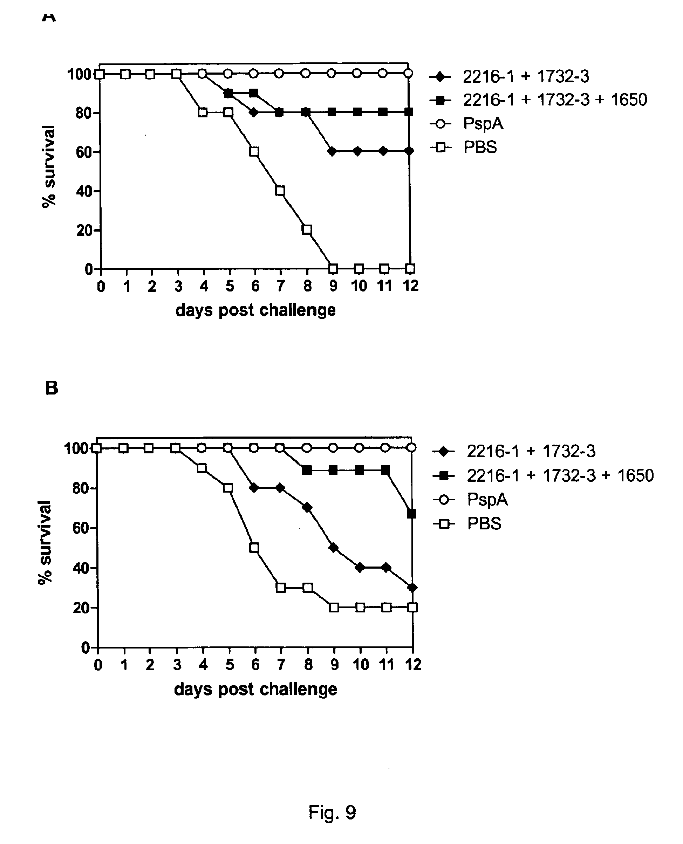

**Figure 9** shows the protection level achieved by active immunization with the combination of SP1732-3 and SP2216-1 or with combinations of SP1732-3, SP2216-1 and SP1650 (PsaA) in a mouse lethality model. C3H/HeN mice (10 mice per group) were immunized **(A)** subcutaneously or **(B)** intramuscularly with the respective combination of recombinant antigens adjuvanted with ALUM. Mice were intraperitoneally challenged with 10⁴ cfu *S*. *pneunoniae* 6B. Adjuvant control mice were used as negative controls, while PspA (SP0117) served as positive control. Survival was monitored for 12 days post challenge and was depicted as percentage of total animals.

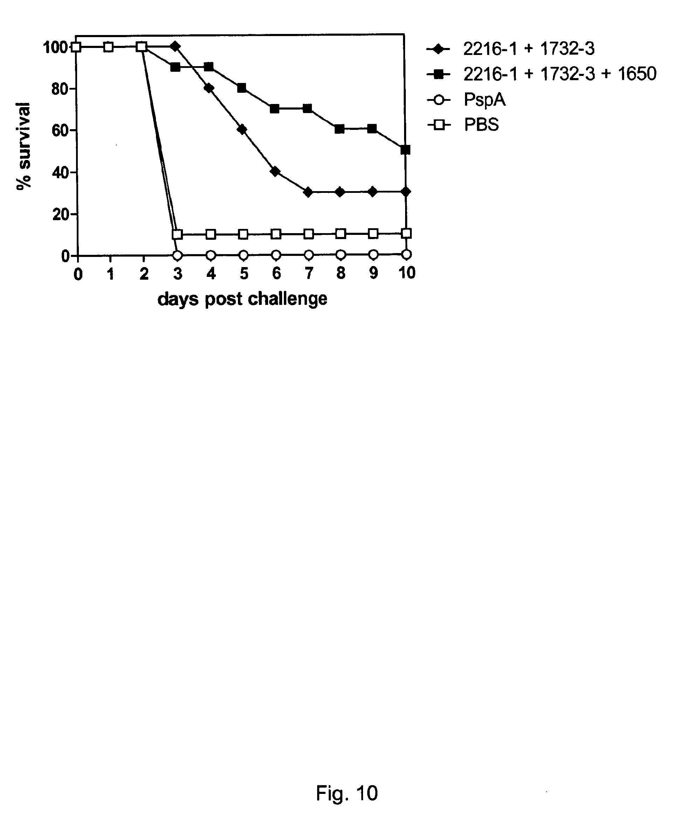

**Figure 10** shows the protection level achieved by active immunization with the combination of SP1732-3 and SP2216-1 or with combinations of SP1732-3, SP2216-1 and SP1650 (PsaA) in an intranasal mouse sepsis model. NMRI mice (10 mice per group) were subcutaneously immunized with 50 µg protein antigen adjuvanted with ALUM. Mice were intranasally challenged with 5x 10⁶ cfu *S. pneumoniae* 6301. Adjuvant control mice were used as negative controls, while PspA (SP0117) served as positive control. Survival was monitored for 10 days post challenge and was depicted as percentage of total animals.

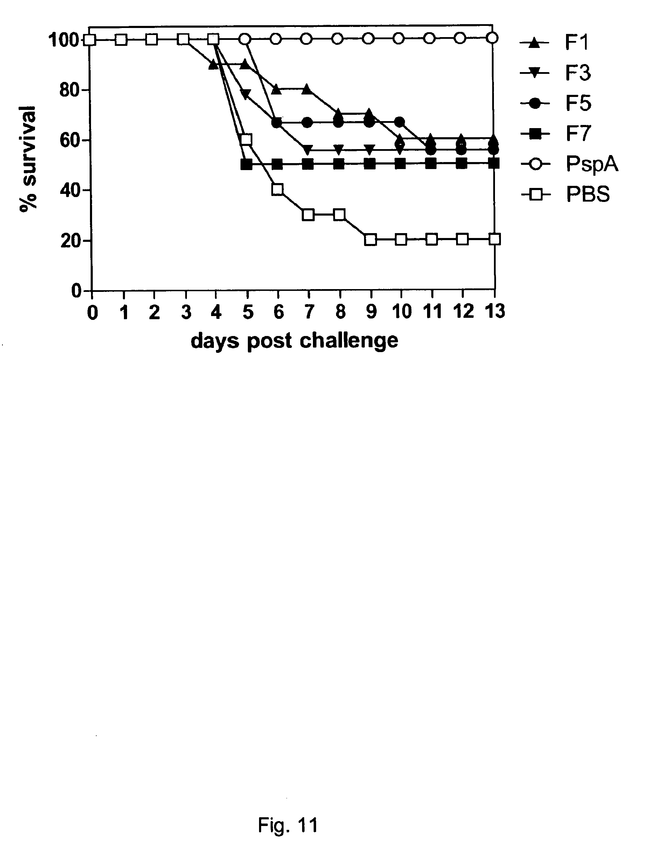

**Figure 11** shows the protection achieved by active immunization with selected *S. pneumoniae* fusion antigens in a mouse lethality model. C3H/HeN mice (10 mice per group) were immunized with recombinant antigens cloned from a serotype 4 or 60 *S. pneumoniae* strain (Table 1) and challenged with a serotype 6B strain. Survival was monitored for 13 days post-challenge. Mice were immunized subcutaneously with 50 µg of the fusion proteins adjuvanted with ALUM. Mice were then challenged intraperitoneally with 10⁴ cfu *S. pneumoniae* 6B. Adjuvant control mice were used as negative controls, while PspA (SP0117) served as positive control. Survival was monitored for 13 days post challenge and was depicted as percentage of total animals.

### EXAMPLE 1

### Combinations of protective antigens induce superior protective immune responses against lethal sepsis and pneumonia induced by S. pneumoniae

### Experimental procedures

### Expression and purification of recombinant pneumococcal proteins

*Cloning of genes* / *DNA fragments:* The gene/DNA fragment of interest was amplified from genomic DNA of *S*. *pneumoniae* (scrotype 4 or 6B) by PCR using gene specific primers. Apart from the gene specific part, the primers had restriction sites that aided in a directional cloning of the amplified PCR product. The gene annealing (specific) part of the primer ranged between 15-30 bases in length. The PCR products obtained were digested with the appropriate restriction enzymes and cloned into the pET28b (+) vector (Novagen) for His-tagged proteins or into pGEX-4T-1 (Pharmacia) for GST fusion proteins. The constructs including encoded chimera proteins of SP2216-1 and/or SP1732 fragment SP1732-3 or SP1732-PASTA are listed in Table 1. SP1732-PASTA is a C-terminal portion of SP1732-3 comprising a PASTA domain (for penicillin-binding protein and serine/threonine kinase associated domain). Once the recombinant plasmid was confirmed to contain the gene of interest, *E*. *coli* BL21 star^{®} cells (Invitrogen) that served as expression host were transformed.

**Table 1: List of genes, gene fragments or gene fusions selected for expression. The nomenclature of the genes is derived from the genome of S. pneumoniae TIGR4. The restriction sites (RE) used for cloning and the position (start/stop) of the amplicon are indicated for each construct.**

| **Construct** | **Gene** | **Serotype** | **Vestor** | **RE** | **start/stop** | |
|---|---|---|---|---|---|---|
| 1 | SP2216-1 | T4 | pET28b | NcoI/NotI | nt: | 82-834 |
| | | | | | aa: | 28-278 |
| 2 | SP1732-3 | 6B | pET28b | NcoI/XhoI | nt: | 1033-1977 |
| | | | | | aa: | 345-659 |
| 3 | SP1650 (PsaA) | 6B | pET28b | NcoI/XhoI | nt: | 61-927 |
| | | | | | aa: | 21-309 |
| 4 | F1 | T4¹, 6B² | pET28b | Ncol/SalI, | nt: | SP2216-1: 82-834 |
| | SP2216-1¹ + | | | SalI/NotI | | SP1732-3: 1033-1977 |
| | SPI 732-3² | | | | aa: | SP2216-1: 28-278 |
| | | | | | | SP1732-3: 345-659 |
| 5 | SP2216-1¹ + | T4¹, 662 | pGEX-4T | BamHI/SalI, | nt: | SP2216-1: 82-834 |
| | SP1732-3² | | | Sall/Notl | | SP1732-3: 1033-1977 |
| | | | | | aa: | SP2216-1: 28-278 |
| | | | | | | SP1732-3: 345-659 |
| 6 | F3 | 6B¹, T4² | pET28b | Ncol/SalI, | nt: | SP1732-3: 1033-1977 |
| | SP1732-3¹ + | | | SalI/Notl | | SP2216-1: 82-834 |
| | SP2216-1² | | | | aa: | SP1732-3: 345-659 |
| | | | | | | SP2218-1: 28-278 |
| 7 | SP1732-3¹ + | 6B¹, T4² | pGEX-4T | BamHI/SalI, | nt: | SP1732-3: 10331977 |
| | SP2216-1² | | | SalI/Notl | | SP2216-1: 82-834 |
| | | | | | aa: | SP1732-3: 345-659 |
| | | | | | | SP2216-1: 28-278 |
| 8 | F5 | T4¹, 6B² | pET28b | Ncol/SmaI, | nt: | SP2216-1: 82-834 |
| | SP2216-1¹ + | | | SmaI/Notl | | SP1732-PASTA: 1735-1977 |
| | SP1732- | | | | aa: | SP2216-1: 28-278 |
| | PASTA² | | | | | SP1732-PASTA: 579-659 |
| 9 | SP2216-1¹ + | T4¹, 6B² | pGEX-4T | BamHI/SmaI, | nt: | SP2216-1: 82-834 |
| | SP1732- | | | SmaI/NotI | | SP1732-PASTA: 1735-1977 |
| | PASTA² | | | | aa: | SP2216-1: 28-278 |
| | | | | | | SP1732-PASTA: 579-659 |
| 10 | F7 | 6B¹, T4² | pET28b | NcoI/SmaI, | nt: | SP1732-PASTA: 1735-1977 |
| | SP1732- | | | Smal/Notl | | SP2216-1:82-834 |
| | PASTA¹ + | | | | aa: | SP1732-PASTA: 579-659 |
| | SP2216-1² | | | | | SP2216-1: 28-278 |
| 11 | SP1732- | 6B¹, T4² | pGEX-4T | BamHI/SmaI, | nt: | SP1732-PASTA: 1735-1977 |
| | PASTA¹ + | | | Smal/Notl | | SP2216-1: 82-834 |
| | SP2216-1² | | | | aa: | SP1732-PASTA: 579-659 |
| | | | | | | SP2216-1: 28-278 |

| | | | | | | |
|---|---|---|---|---|---|---|
| and serotype of the gene used for fusion proteins | | | | | | |

### Amino Acid Sequences:

Construct 1: SP2216-1; SEQ ID NO:1
ETTDDKIAAQDNKISNLTAQQQEAQKQVDQIQEQVSAIQAEQSNLQAENDRLQAESKKLEG
EITELSKNIVSRNQSLEKQARSAQTNGAVTSYINTIVNSKSITEAISRVAAMSEIVSANNK
MLEQQKADKKAISEKQVANNDAINTVIANQQKLADDAQALTTKQAELKAAELSLAAEKATA
EGEKASLLEQKAAAEAEARAAAVAEAAYKEKRASQQQSVLASANTNLTAQVQAVSESAAAP
VRAKVRP

Construct 2: SP1732-3; SEQ ID NO:2
YLILLASLVLVAASLIWILSRTPATIAIPDVAGQTVAEAKATLKKANFEIGEEKTEASEKV
EEGRIIRTDPGAGTGRKEGTKINLVVSSGKQSFQISNYVGRKSSDVIAELKEKKVPDNLIK
IEEEESNESEAGTVLKQSLPEGTTYDLSKATQIVLTVAKKATTIQLGNYIGRNSTEVISEL
KQKKVPENLIKIEEEESSESEPGTIMKQSPGAGTTYDVSKPTQIVLTVAKKVTSVAMPSYI
GSSLEFTKNNLIQIVGIKEANIEVVEVTTAPAGSVEGMVVEQSPRAGEKVDLNKTRVKISI
YKPKTTSATP

Construct 3: SP1650 (PsaA); SEQ ID NO:3
ASGKKDTTSGQKLKVVATNSIIADITKNIAGDKIDLHSIVPIGQDPHEYEPLPEDVKKTSE
ADLIFYNGINLETGGNAWFTKLVENAKKTENKDYFAVSDGVDVIYLEGQNEKGKEDPHAWL
NLENGIIFAKNIAKQLSAKDPNNKEFYEKNLKEYTDKLDKLDKESKDKFNKIPAEKKLIVT
SEGAFKYFSKAYGVPSAYIWEINTEEEGTPEQIKTLVEKLRQTKVPSLFVESSVDDRPMKT
VSQDTNIPIYAQIFTDSIAEQGKEGDSYYSMMKYNLDKIAEGLAK

Construct 4 & 5: SP2216-1 + SP1732-3; SEQ ID NO:4
ETTDDKIAAQDNKISNLTAQQQEAQKQVDQIQEQVSAIQAEQSNLQAENDRLQAESKKLEG
EITELSKNIVSRNQSLEKQARSAQTNGAVTSYINTIVNSKSITEAISRVAAMSEIVSANNK
MLEQQKADKKAISEKQVANNDAINTVIANQQKLADDAQALTTKQAELKAAELSLAAEKATA
EGEKASLLEQKAAAEAEARAAAVAEAAYKEKRASQQQSVLASANTNLTAQVQAVSESAAAP
VRAKVRPGPVDPGYLILLASLVLVAASLIWILSRTPATIAIPDVAGQTVAEAKATLKKANF
EIGEEKTEASEKVEEGRIIRTDPGAGTGRKEGTKINLVVSSGKQSFQISNYVGRKSSDVIA
ELKEKKVPDNLIKIEEEESNESEAGTVLKQSLPEGTTYDLSKATQIVLTVAKKATTIQLGN
YIGRNSTEVISELKQKKVPENLIKIEEEESSESEPGTIMKQSPGAGTTYDVSKPTQIVLTV
AKKVTSVAMPSYIGSSLEFTKNNLIQIVGIKEANIEVVEVTTAPAGSVEGMVVEQSPRAGE
KVDLNKTRVKISIYKPKTTSATP
_ indicates the linker between SP2216-1 and SP1732-3

Construct 6 & 7: SP1732-3 + SP2216-1; SEQ ID NO:5
YLILLASLVLVAASLIWILSRTPATIAIPDVAGQTVAEAKATLKKANFEIGEEKTEASEKV
EEGRIIRTDPGAGTGRKEGTKINLVVSSGKQSFQISNYVGRKSSDVIAELKEKKVPDNLIK
IEEEESNESEAGTVLKQSLPEGTTYDLSKATQIVLTVAKKATTIQLGNYIGRNSTEVISEL
KQKKVPENLIKIEEEESSESEPGTIMKQSPGAGTTYDVSKPTQIVLTVAKKVTSVAMPSYI
GSSLEFTKNNLIQIVGIKEANIEVVEVTTAPAGSVEGMVVEQSPRAGEKVDLNKTRVKISI
YKPKTTSATPGPVDPGETTDDKIAAQDNKISNLTAQQQEAQKQVDQIQEQVSAIQAEQSNL
QAENDRLQAESKKLEGEITELSKNIVSRNQSLEKQARSAQTNGAVTSYINTIVNSKSITEA
ISRVAAMSEIVSANNKMLEQQKADKKAISEKQVANNDAINTVIANQQKLADDAQALTTKQA
ELKAAELSLAAEKATAEGEKASLLEQKAAAEAEARAAAVAEAAYKEKRASQQQSVLASANT
NLTAQVQAVSESAAAPVRAKVRP
_ indicates the linker between SP1732-3 and SP2216-1

Construct 8 & 9: SP2216-1 + SP1732-PASTA; SEQ ID NO:6
ETTDDKIAAQDNKISNLTAQQQEAQKQVDQIQEQVSAIQAEQSNLQAENDRLQAESKKLEG
EITELSKNIVSRNQSLEKQARSAQTNGAVTSYINTIVNSKSITEAISRVAAMSEIVSANNK
MLEQQKADKKAISEKQVANNDAINTVIANQQKLADDAQALTTKQAELKAAELSLAAEKATA
EGEKASLLEQKAAAEAEARAAAVAEAAYKEKRASQQQSVLASANTNLTAQVQAVSESAAAP
VRAKVRPGGPGVTSVAMPSYIGSSLEFTKNNLIQIVGIKEANIEVVEVTTAPAGSVEGMVV
EQSPRAGEKVDLNKTRVKISIYKPKTTSATP
_ indicates the linker between SP2216-1 and SP1732-PASTA

Construct 10 & 11: SP1732-PASTA + SP2216-1; SEQ ID NO:7
VTSVAMPSYIGSSLEFTKNNLIQIVGIKEANIEVVEVTTAPAGSVEGMVVEQSPRAGEKVD
LNKTRVKISIYKPKTTSATPGGPGETTDDKIAAQDNKISNLTAQQQEAQKQVDQIQEQVSA
IQAEQSNLQAENDRLQAESKKLEGEITELSKNIVSRNQSLEKQARSAQTNGAVTSYINTIV
NSKSITEAISRVAAMSEIVSANNKMLEQQKADKKAISEKQVANNDAINTVIANQQKLADDA
QALTTKQAELKAAELSLAAEKATAEGEKASLLEQKAAAEAEARAAAVAEAAYKEKRASQQQ
SVLASANTNLTAQVQAVSESAAAPVRAKVRP
_ indicates the linker between SP1732-PASTA and SP2216-1

### DNA Sequences:

Construct 1: SP2216-1; SEQ ID NO:8
GAAACGACTGATGACAAAATTGCTGCTCAAGATAATAAAATTAGTAACTTAACAGCACAAC
AACAAGAAGCCCAAAAACAAGTTGACCAAATTCAGGAGCAAGTATCAGCTATTCAAGCTGA
GCAGTCTAACTTGCAAGCTGAAAATGATAGATTACAAGCAGAATCTAAGAAACTCGAGGGT
GAGATTACAGAACTTTCTAAAAACATTGTTTCTCGTAACCAATCGTTGGAAAAACAAGCTC
GTAGTGCTCAAACAAATGGAGCCGTAACTAGCTATATCAATACCATTGTAAACTCAAAATC
AATTACAGAAGCTATTTCACGTGTTGCTGCAATGAGTGAAATCGTATCTGCAAACAACAAA
ATGTTAGAACAACAAAAGGCAGATAAAAAAGCTATTTCTGAAAAACAAGTAGCAAATAATG
ATGCTATCAATACTGTAATTGCTAATCAACAAAAATTGGCTGATGATGCTCAAGCATTGAC
TACGAAACAGGCAGAACTAAAAGCTGCTGAATTAAGTCTTGCTGCTGAGAAAGCGACAGCT
GAAGGGGAAAAAGCAAGTCTATTAGAGCAAAAAGCAGCAGCTGAGGCAGAGGCTCGTGCAG
CTGCGGTAGCAGAAGCAGCTTATAAAGAAAAACGAGCTAGCCAACAACAATCAGTACTTGC
TTCAGCAAACACTAACTTAACAGCTCAAGTGCAAGCAGTATCTGAATCTGCAGCAGCACCT
GTCCGTGCAAAAGTTCGTCCA

Construct 2: SP1732-3; SEQ ID NO:9
TACCTGATTTTGTTGGCCAGCCTTGTATTGGTGGCAGCTTCTCTTATTTGGATACTATCCA
GAACTCCTGCAACCATTGCCATTCCAGATGTGGCAGGTCAGACAGTTGCAGAGGCCAAGGC
AACGCTCAAAAAGCCAATTTTGAGATTGGTGAGGAGAAGACAGAGGCTAGTGAAAAGGTG
GAAGAAGGGCGGATTATCCGTACAGATCCTGGCGCTGGAACTGGTCGAAAAGAAGGAACGA
AAATCAATTTGGTTGTCTCATCAGGCAAACAATCCTTCCAAATTAGTAATTATGTCGGCCG
GAAATCTTCTGATGTTATCGCGGAATTAAAAGAGAAAAAAGTTCCAGATAATTTGATTAAA
ATTGAGGAAGAAGAGTCGAATGAGAGTGAGGCTGGAACGGTCCTGAAGCAAAGTCTACCAG
AAGGTACGACCTATGACTTGAGCAAGGCAACTCAAATTGTTTTGACAGTAGCTAAAAAAGC
TACGACGATTCAATTAGGGAACTATATTGGACGGAACTCTACAGAAGTAATCTCAGAACTC
AAGCAGAAGAAGGTTCCTGAGAATTTGATTAAGATAGAGGAAGAAGAGTCCAGCGAAAGCG
AACCAGGAACGATTATGAAACAAAGTCCAGGTGCCGGAACGACTTATGATGTGAGTAAACC
TACTCAAATTGTCTTGACAGTAGCTAAAAAAGTTACAAGTGTTGCCATGCCGAGTTACATT
GGTTCCAGCTTGGAGTTTACTAAGAACAATTTGATTCAAATTGTTGGGATTAAGGAAGCTA
ATATAGAAGTTGTAGAAGTGACGACAGCGCCTGCAGGTAGTGTAGAAGGCATGGTTGTTGA
ACAAAGTCCTAGAGCAGGTGAAAAGGTAGACCTAAATAAGACTAGAGTCAAGATTTCAATC
TACAAACCTAAAACAACTTCAGCTACTCCT

Construct 3: SP1650 (PsaA); SEQ ID NO:10
GCTAGCGGAAAAAAGATACAACTTCTGGTCAAAAACTAAAAGTTGTTGCTACAAACTCAA
TCATCGCTGATATTACTAAAAATATTGCTGGTGACAAAATTGACCTTCATAGTATCGTTCC
GATTGGGCAAGACCCACACGAATACGAACCACTTCCTGAAGACGTTAAGAAAACTTCTGAG
GCTGATTTGATTTTCTATAACGGTATCAACCTTGAAACAGGTGGCAATGCTTGGTTTACAA
AATTGGTAGAAAATGCCAAGAAAACTGAAAACAAAGACTACTTCGCAGTCAGCGACGGCGT
TGATGTTATCTACCTTGAAGGTCAAAATGAAAAAGGAAAAGAAGACCCACACGCTTGGCTT
AACCTTGAAAACGGTATTATTTTTGCTAAAAATATCGCCAAACAATTGAGCGCCAAAGACC
CTAACAATAAAGAATTCTATGAAAAAAATCTCAAAGAATATACTGATAAGTTAGACAAACT
TGATAAAGAAAGTAAGGATAAATTTAATAAGATCCCTGCTGAAAAGAAACTCATTGTAACC
AGCGAAGGAGCATTCAAATACTTCTCTAAAGCCTATGGTGTCCCAAGTGCCTACATCTGGG
AAATCAATACTGAAGAAGAAGGAACTCCTGAACAAATCAAGACCTTGGTTGAAAAACTTCG
CCAAACAAAAGTTCCATCACTCTTTGTAGAATCAAGTGTGGATGACCGTCCAATGAAAACT
GTTTCTCAAGACACAAACATCCCAATCTACGCACAAATCTTTACTGACTCTATCGCAGAAC
AAGGTAAAGAAGGCGACAGCTACTACAGCATGATGAAATACAACCTTGACAAGATTGCTGA
AGGATTGGCAAAA

Construct 4 & 5: SP2216-1 + SP1732-3; SEQ ID NO:11
GAAACGACTGATGACAAAATTGCTGCTCAAGATAATAAAATTAGTAACTTAACAGCACAAC
AACAAGAAGCCCAAAAACAAGTTGACCAAATTCAGGAGCAAGTATCAGCTATTCAAGCTGA
GCAGTCTAACTTGCAAGCTGAAAATGATAGATTACAAGCAGAATCTAAGAAACTCGAGGGT
GAGATTACAGAACTTTCTAAAAACATTGTTTCTCGTAACCAATCGTTGGAAAAACAAGCTC
GTAGTGCTCAAACAAATGGAGCCGTAACTAGCTATATCAATACCATTGTAAACTCAAAATC
AATTACAGAAGCTATTTCACGTGTTGCTGCAATGAGTGAAATCGTATCTGCAAACAACAAA
ATGTTAGAACAACAAAAGGCAGATAAAAAAGCTATTTCTGAAAAACAAGTAGCAAATAATG
ATGCTATCAATACTGTAATTGCTAATCAACAAAAATTGGCTGATGATGCTCAAGCATTGAC
TACGAAACAGGCAGAACTAAAAGCTGCTGAATTAAGTCTTGCTGCTGAGAAAGCGACAGCT
GAAGGGGAAAAAGCAAGTCTATTAGAGCAAAAAGCAGCAGCTGAGGCAGAGGCTCGTGCAG
CTGCGGTAGCAGAAGCAGCTTATAAAGAAAAACGAGCTAGCCAACAACAATCAGTACTTGC
TTCAGCAAACACTAACTTAACAGCTCAAGTGCAAGCAGTATCTGAATCTGCAGCAGCACCT
GTCCGTGCAAAAGTTCGTCCAGGACCAGTCGACCCTGGTTACCTGATTTTGTTGGCCAGCC
TTGTATTGGTGGCAGCTTCTCTTATTTGGATACTATCCAGAACTCCTGCAACCATTGCCAT
TCCAGATGTGGCAGGTCAGACAGTTGCAGAGGCCAAGGCAACGCTCAAAAAAGCCAATTTT
GAGATTGGTGAGGAGAAGACAGAGGCTAGTGAAAAGGTGGAAGAAGGGCGGATTATCCGTA
CAGATCCTGGCGCTGGAACTGGTCGAAAAGAAGGAACGAAAATCAATTTGGTTGTCTCATC
AGGCAAACAATCCTTCCAAATTAGTAATTATGTCGGCCGGAAATCTTCTGATGTTATCGCG
GAATTAAAAGAGAAAAAAGTTCCAGATAATTTGATTAAAATTGAGGAAGAAGAGTCGAATG
AGAGTGAGGCTGGAACGGTCCTGAAGCAAAGTCTACCAGAAGGTACGACCTATGACTTGAG
CAAGGCAACTCAAATTGTTTTGACAGTAGCTAAAAAAGCTACGACGATTCAATTAGGGAAC
TATATTGGACGGAACTCTACAGAAGTAATCTCAGAACTCAAGCAGAAGAAGGTTCCTGAGA
ATTTGATTAAGATAGAGGAAGAAGAGTCCAGCGAAAGCGAACCAGGAACGATTATGAAACA
AAGTCCAGGTGCCGGAACGACTTATGATGTGAGTAAACCTACTCAAATTGTCTTGACAGTA
GCTAAAAAAGTTACAAGTGTTGCCATGCCGAGTTACATTGGTTCCAGCTTGGAGTTTACTA
AGAACAATTTGATTCAAATTGTTGGGATTAAGGAAGCTAATATAGAAGTTGTAGAAGTGAC
GACAGCGCCTGCAGGTAGTGTAGAAGGCATGGTTGTTGAACAAAGTCCTAGAGCAGGTGAA
AAGGTAGACCTAAATAAGACTAGAGTCAAGATTTCAATCTACAAACCTAAAACAACTTCAG
CTACTCCT

Construct 6 & 7: SP1732-3 + SP2216-1; SEQ ID NO:12
TACCTGATTTTGTTGGCCAGCCTTGTATTGGTGGCAGCTTCTCTTATTTGGATACTATCCA
GAACTCCTGCAACCATTGCCATTCCAGATGTGGCAGGTCAGACAGTTGCAGAGGCCAAGGC
AACGCTCAAAAAAGCCAATTTTGAGATTGGTGAGGAGAAGACAGAGGCTAGTGAAAAGGTG
GAAGAAGGGCGGATTATCCGTACAGATCCTGGCGCTGGAACTGGTCGAAAAGAAGGAACGA
AAATCAATTTGGTTGTCTCATCAGGCAAACAATCCTTCCAAATTAGTAATTATGTCGGCCG
GAAATCTTCTGATGTTATCGCGGAATTAAAAGAGAAAAAAGTTCCAGATAATTTGATTAAA
ATTGAGGAAGAAGAGTCGAATGAGAGTGAGGCTGGAACGGTCCTGAAGCAAAGTCTACCAG
AAGGTACGACCTATGACTTGAGCAAGGCAACTCAAATTGTTTTGACAGTAGCTAAAAAAGC
TACGACGATTCAATTAGGGAACTATATTGGACGGAACTCTACAGAAGTAATCTCAGAACTC
AAGCAGAAGAAGGTTCCTGAGAATTTGATTAAGATAGAGGAAGAAGAGTCCAGCGAAAGCG
AACCAGGAACGATTATGAAACAAAGTCCAGGTGCCGGAACGACTTATGATGTGAGTAAACC
TACTCAAATTGTCTTGACAGTAGCTAAAAAAGTTACAAGTGTTGCCATGCCGAGTTACATT
GGTTCCAGCTTGGAGTTTACTAAGAACAATTTGATTCAAATTGTTGGGATTAAGGAAGCTA
ATATAGAAGTTGTAGAAGTGACGACAGCGCCTGCAGGTAGTGTAGAAGGCATGGTTGTTGA
ACAAAGTCCTAGAGCAGGTGAAAAGGTAGACCTAAATAAGACTAGAGTCAAGATTTCAATC
TACAAACCTAAAACAACTTCAGCTACTCCTGGACCAGTCGACCCTGGTGAAACGACTGATG
ACAAAATTGCTGCTCAAGATAATAAAATTAGTAACTTAACAGCACAACAACAAGAAGCCCA

AAAACAAGTTGACCAAATTCAGGAGCAAGTATCAGCTATTCAAGCTGAGCAGTCTAACTTG
CAAGCTGAAAATGATAGATTACAAGCAGAATCTAAGAAACTCGAGGGTGAGATTACAGAAC
TTTCTAAAAACATTGTTTCTCGTAACCAATCGTTGGAAAAACAAGCTCGTAGTGCTCAAAC
AAATGGAGCCGTAACTAGCTATATCAATACCATTGTAAACTCAAAATCAATTACAGAAGCT
ATTTCACGTGTTGCTGCAATGAGTGAAATCGTATCTGCAAACAACAAAATGTTAGAACAAC
AAAAGGCAGATAAAAAAGCTATTTCTGAAAAACAAGTAGCAAATAATGATGCTATCAATAC
TGTAATTGCTAATCAACAAAAATTGGCTGATGATGCTCAAGCATTGACTACGAAACAGGCA
GAACTAAAAGCTGCTGAATTAAGTCTTGCTGCTGAGAAAGCGACAGCTGAAGGGGAAAAAG
CAAGTCTATTAGAGCAAAAAGCAGCAGCTGAGGCAGAGGCTCGTGCAGCTGCGGTAGCAGA
AGCAGCTTATAAAGAAAAACGAGCTAGCCAACAACAATCAGTACTTGCTTCAGCAAACACT
AACTTAACAGCTCAAGTGCAAGCAGTATCTGAATCTGCAGCAGCACCTGTCCGTGCAAAAG
TTCGTCCA

Construct 8 & 9: SP2216-1 + SP1732-PASTA; SEQ ID NO:13
GAAACGACTGATGACAAAATTGCTGCTCAAGATAATAAAATTAGTAACTTAACAGCACAAC
AACAAGAAGCCCAAAAACAAGTTGACCAAATTCAGGAGCAAGTATCAGCTATTCAAGCTGA
GCAGTCTAACTTGCAAGCTGAAAATGATAGATTACAAGCAGAATCTAAGAAACTCGAGGGT
GAGATTACAGAACTTTCTAAAAACATTGTTTCTCGTAACCAATCGTTGGAAAAACAAGCTC
GTAGTGCTCAAACAAATGGAGCCGTAACTAGCTATATCAATACCATTGTAAACTCAAAATC
AATTACAGAAGCTATTTCACGTGTTGCTGCAATGAGTGAAATCGTATCTGCAAACAACAAA
ATGTTAGAACAACAAAAGGCAGATAAAAAAGCTATTTCTGAAAAACAAGTAGCAAATAATG
ATGCTATCAATACTGTAATTGCTAATCAACAAAAATTGGCTGATGATGCTCAAGCATTGAC
TACGAAACAGGCAGAACTAAAAGCTGCTGAATTAAGTCTTGCTGCTGAGAAAGCGACAGCT
GAAGGGGAAAAAGCAAGTCTATTAGAGCAAAAAGCAGCAGCTGAGGCAGAGGCTCGTGCAG
CTGCGGTAGCAGAAGCAGCTTATAAAGAAAAACGAGCTAGCCAACAACAATCAGTACTTGC
TTCAGCAAACACTAACTTAACAGCTCAAGTGCAAGCAGTATCTGAATCTGCAGCAGCACCT
GTCCGTGCAAAAGTTCGTCCAGGCGGACCCGGGGTTACAAGTGTTGCCATGCCGAGTTACA
TTGGTTCCAGCTTGGAGTTTACTAAGAACAATTTGATTCAAATTGTTGGGATTAAGGAAGC
TAATATAGAAGTTGTAGAAGTGACGACAGCGCCTGCAGGTAGTGTAGAAGGCATGGTTGTT
GAACAAAGTCCTAGAGCAGGTGAAAAGGTAGACCTAAATAAGACTAGAGTCAAGATTTCAA
TCTACAAACCTAAAACAACTTCAGCTACTCCT

Construct 10 & 11: SP1732-PASTA + SP2216-1; SEQ ID NO:14
GTTACAAGTGTTGCCATGCCGAGTTACATTGGTTCCAGCTTGGAGTTTACTAAGAACAATT
TGATTCAAATTGTTGGGATTAAGGAAGCTAATATAGAAGTTGTAGAAGTGACGACAGCGCC
TGCAGGTAGTGTAGAAGGCATGGTTGTTGAACAAAGTCCTAGAGCAGGTGAAAAGGTAGAC
CTAAATAAGACTAGAGTCAAGATTTCAATCTACAAACCTAAAACAACTTCAGCTACTCCTG
GCGGACCCGGGGAAACGACTGATGACAAAATTGCTGCTCAAGATAATAAAATTAGTAACTT
AACAGCACAACAACAAGAAGCCCAAAAACAAGTTGACCAAATTCAGGAGCAAGTATCAGCT
ATTCAAGCTGAGCAGTCTAACTTGCAAGCTGAAAATGATAGATTACAAGCAGAATCTAAGA
AACTCGAGGGTGAGATTACAGAACTTTCTAAAAACATTGTTTCTCGTAACCAATCGTTGGA
AAAACAAGCTCGTAGTGCTCAAACAAATGGAGCCGTAACTAGCTATATCAATACCATTGTA
AACTCAAAATCAATTACAGAAGCTATTTCACGTGTTGCTGCAATGAGTGAAATCGTATCTG
CAAACAACAAAATGTTAGAACAACAAAAGGCAGATAAAAAAGCTATTTCTGAAAAACAAGT
AGCAAATAATGATGCTATCAATACTGTAATTGCTAATCAACAAAAATTGGCTGATGATGCT
CAAGCATTGACTACGAAACAGGCAGAACTAAAAGCTGCTGAATTAAGTCTTGCTGCTGAGA
AAGCGACAGCTGAAGGGGAAAAAGCAAGTCTATTAGAGCAAAAAGCAGCAGCTGAGGCAGA
GGCTCGTGCAGCTGCGGTAGCAGAAGCAGCTTATAAAGAAAAACGAGCTAGCCAACAACAA
TCAGTACTTGCTTCAGCAAACACTAACTTAACAGCTCAAGTGCAAGCAGTATCTGAATCTG
CAGCAGCACCTGTCCGTGCAAAAGTTCGTCCA

### Expression and purification of proteins:

*E. coli* BL21 star^{®} cells harboring the recombinant plasmid were grown into log phase in the required culture volume. Once an OD₆₀₀ₙₘ of 0.6 was reached the culture was induced with 0.5 mM IPTG for 3 hours at 37°C. The cells were harvested by centrifugation, lysed by a combination of the freeze-thaw method followed by disruption of cells with 'Bug-buster^{®,} (Novagen). The lysate was separated by centrifugation into soluble (supernatant) and insoluble (pellet) fractions. Depending on the location of the protein different purification strategies were applied.
A) If the His-tagged protein was in the soluble fraction, protein purification was done by binding the supernatant to Ni-Sepharose beads (Ni-Sepharose^{™} 6 Fast Flow, GE Healthcare). Due to the presence of the hexa Histidine (6x HIS) at the C-terminus of the expressed protein, it bound to the Ni-Sepharose while the other contaminating proteins were washed from the column by wash buffer. The protein was cluted by 500 mM Imidazole in 20 mM NaH₂PO₄, 0.5 mM NaCl buffer at pH 7.4. The eluate was concentrated, assayed by Bradford for protein concentration and checked by SDS-PAGE and Western blot.
B) If the protein was present in the insoluble fraction the pellet was solubilized in suitable buffer containing 8 M urea and applied onto the Ni-NTA column under denaturing conditions (in buffer containing 8 M urea) using the same materials and procedure as mentioned above. Contaminating proteins were washed from the column by wash buffer without urea. Refolding of the His-taggcd protein was performed while the protein was immobilized on the Ni-NTA matrix. After renaturation, proteins were eluted by the addition of 500 mM Imidazole. The eluate was dialyzed to remove traces of urea and concentrated if the volume was large, checked by SDS-PAGE and measured by the Bradford method.
C) For purification of GST fusion proteins the soluble fraction was bound to Glutathione Sepharose^{™} 4B (Amersham). After removing unbound proteins by washing with PBS, the GST fusion protein was digested with thrombin over night at room temperature and the cleaved protein was collected in the flow through.

### Animal protection studies

Animals: C3H/HeN, CBA/N and NMRI female mice were used.

Active immunization: 50 µg of recombinant proteins were injected subcutaneously, adjuvanted with Complete Freund's adjuvant (CFA), aluminum hydroxide or IC31^{®} Animals were boosted twice with the same amount of protein and adjuvant, (except for CFA where Incomplete Freund's adjuvant (IFA) was used) at days 14 and 28. The published protective antigen PspA (SP0117) was used as a positive control, while mice immunized with adjuvant only served as negative controls. Antibody titres were measured at day 35 by ELISA using the respective recombinant proteins, in order to confirm that the immunization had induced a proper immune response.

Bacterial challenge: A frozen glycerol stock of *S. pneumoniae* serotype 6B, EF3030 or TIGR4 was prepared and used for all experiments including these serotypes. For other *S. pneumoniae* serotypes (e.g. 6301) freshly grown bacteria were used. In order to determine the viable cell numbers present in the bacterial inoculum, cfus were determined via plating on blood agar plates. 10² - 10⁸ cfu were applied intraperitoneally, intravenously or intranasally as challenge into individual mice. For intranasal applications, mice were anesthetized before the treatment. Protection by immunization was measured for both, a sepsis and pneumonia model. In the sepsis model, survival rates were followed for approximately 2 weeks post-challenge and survival was expressed in percentage of total number of animals (10 mice / group). For the pneumonia model, lungs were removed at day 3 post challenge under sterile conditions, homogenized and cultures of lung homogenates were quantitatively plated on blood agar plates. Cfus per organ were determined for each individual mouse (5 - 10 mice/group).

### Results

Combinations of different pneumococcal antigens were identified showing a larger level of protection in a mouse sepsis/lethality model than the individual proteins alone (Figure 1). The best levels of protection were achieved by immunization with a combination of three recombinant proteins SP2216-1, SP1732-3 and PsaA, while lower levels of protection were observed with combinations of only two proteins (SP2216-1 + SP1732-3 superior to SP2216-1 + PsaA superior to SP1732-3 + PsaA). PsaA alone did not show a significant level of protection, but in combination with the other two proteins increased the protection level significantly. No negative influence on the protection level could be observed for any of the proteins tested. The increase in protection levels was independent of the adjuvant used, since no difference in protection was seen by using either Aluminum hydroxide or IC31^{®} adjuvant (Intercell AG, Vienna, Austria).

SP2216-1 and SP1732-3 were not only been shown to be protective against *S. pneumoniae* 6B (Figure 1), but it could be also demonstrated that they provided protection against different serotypes of *S. pneumoniae.* Protection could be shown against sepsis for *S. pneumoniae* strain 6301 (serotype 1) after intranasal application, which represents the more physiological way of challenge in contrast to the intraperitoneal route. SP2216-1 demonstrated a significant level of protection against sepsis in this model, which was higher than the protection capacity of PspA, the positive control protein. SP1732-3 showed a significant level of protection comparable to PspA, and well above the negative control (Figure 2).

CBA/N mice have a spontaneous mutation in the *xid* gene and therefore lack the ability to produce antibodies to polysaccharides and lack serum antibodies to the phosphocholine determinant of pneumococcal tcichoic acid. Immunization with SP2216-1 and SP1732-3 provided protection against challenge with the *S. pneumoniae* TIGR4 strain (serotype 4) in CBA/N mice. The protective effect was therefore clearly due to antibodies directed against the immunized proteins, but not against the bacterial polysaccharide. Although it is well known that the TIGR4 strain is a highly virulent serotype of *S. pneumoniae,* mice were partially protected after vaccination with both proteins. The protection levels were well above the negative control, although not as high as those for PspA (Figure 3).

Significant protection was also observed in additional sepsis models with a variety of serotypes of *S. pneumoniae* after immunization with both proteins, SP2216-1 and SP1732-3 (data not shown).

Beside sepsis, pneumonia is a major cause of pneumococcal death in patients. Therefore, it is relevant to show that protection or reduction of bacterial colonization can be induced by the vaccine candidates SP1732-3 and SP2216-1. Here we demonstrate the protective effect of a pneumococcal antigen against pneumonia. Mice immunized with SP2216-1 showed a reduced level of colonization when lung homogenates were tested for colonization 6 days after challenge with *S. pneumoniae* strain EF3030 (serotype 19F) (Figure 4). SP2216-1 vaccinated animals clearly promoted a reduced bacterial load (2 to 3 log reduced) in the lung.

### EXAMPLE 2

### Identification of pneumococcal antigens and combinations inducing protective immune responses against lethal sepsis and pneumonia induced by S. pneumoniae

### Experimental procedures

### Expression and purification of recombinant pneumococcal proteins

### Cloning of genes /DNA fragments: as described in Example 1.

### Expression and purification of proteins: as described in Example 1.

### Animal protection studies

### Animals: C3H/HcN, CD-1 and NMRI female mice were used.

Active immunization: 50 µg of recombinant proteins were injected subcutancously or intramuscularly, adjuvanted with aluminum hydroxide (ALUM). Animals were boosted twice with the same amount of protein and adjuvant at days 14 and 28. The published protective antigen PspA (SP0117), Prevnar or the respective lysate was used as a positive control, while mice immunized with adjuvant only served as negative controls. Antibody titres were measured at day 35 by ELISA using the respective recombinant proteins.

Bacterial challenge: A frozen glycerol stock of *S. pneumoniae* serotype 6B, WU2 (serotype 3) or EF3030 (serotype 19F) was prepared and used for all experiments including these serotypes. For other *S. pneumoniae* serotypes (e.g. *S. pneumoniae* 6301 - serotype 1) freshly grown bacteria were used for all experiments. In order to determine the viable cell numbers present in the bacterial inoculum, cfus were determined via plating on blood agar plates. 10⁴ - 10⁸ cfu were applied intraperitoneally or intranasally, as challenge into individual mice. For intranasal applications, mice were anesthetized before the treatment. Protection by immunization was measured for both, a sepsis and pneumonia model. In the sepsis model, survival rates were followed for 2 weeks post-challenge and survival was expressed in percentage of total number of animals (10 mice / group). For the pneumonia model, lungs were removed at day 3 post challenge under sterile conditions, homogenized and cultures of lung homogenates were quantitatively plated on blood agar plates. Cfus per organ were determined for each individual mouse (10 mice/group).

### Results

Combinations of different pneumococcal antigens were identified showing a similar or larger level of protection in mouse pneumonia models than the individual proteins alone (Figure 8). Upon immunization with ALUM, the highest reduction in colonization with WU2 (serotype 3) was achieved, besides the lysate control, by immunization with a combination of two or three recombinant proteins, SP2216-1, SP1732-3 (and SP1650 (PsaA)), while lower levels of protection were observed with the single proteins as well as with the second positive control, PspA (Figure 8A). SP1650 (PsaA) alone did not show a significant level of protection, but had no negative influence on the protection using combinations including SP2216-1 and SP1732-3.

Immunization and challenge experiments using the EF3030 pneumonia model (serotype 19F) in combination with ALUM adjuvant showed that particularly SP1732-3, SP2216-1 + SP1732-3, SP2216-1 + SP1732-3 + SP1650 as well as Prevnar lowered lung colonization by day 3 substantially (by 3 to 5 logs) (Figure 8B). PspA had also a beneficial effect on the reduction of colonization (1 to 4 logs). SP1650 did not reduce lung colonization at all. In combination with SP1732-3 and SP2216-1, SP1650 in general did not change the outcome substantially.

So far, all experiments have been performed using the subcutaneous immunization route. To define whether that is the most efficient route in mice, experiments were performed to compare the subcutaneous versus the intramuscular route in the 6B sepsis/lethality model. Combinations including all three proteins (SP2216-1, SP1732-3 and SP1650) were compared to the combination of SP2216-1 and SP1732-3. As seen in Figure 9, no significant difference for the combination of all three proteins could be observed. For the combination with two proteins, the subcutaneous route showed slightly better protection.

Combinations of the proteins SP2216-1, SP1732-3 and SP1650 (PsaA) were not only shown to be protective against *S. pneumoniae* 6B, WU2 and EF3030 (Figure 8, 9), but it could be also demonstrated that they provided protection against a further serotype of *S. pneumoniae.* Protection could be obtained against sepsis for *S. pneumoniae* strain 6301 (serotype 1) after intranasal application, which represents the more physiological way of challenge in contrast to the intraperitoneal route. The best protection was seen for the combination including all three proteins or the combination of only SP1732-3 and SP2216-1 (Figure 10).

In order to assess the possibility to combine two vaccine candidates in one recombinant protein, 4 different fusion proteins (F1, F3, F5 and F7, for details see Table 1) of SP1732 and SP2216 were generated. The experiments employing said fusion proteins show in addition to the combination experiments that a high level of protection can be obtained in a mouse sepsis/lethality model with the indicated recombinant fusion proteins (Figure 11). All generated constructs, independently of the way the proteins were fused, showed a good level of protection in a similar range as the combination of both single proteins.

The following pages 68 to 73 contain specific embodiments.
1. A protective peptide consisting of the amino acid sequence of the SEQ ID NO:1, or a functionally active variant of the protective peptide.
2. The functionally active variant of the protective peptide of 1, wherein the functionally active variant
   a) is a functionally active fragment of the protective peptide, the functionally active fragment comprising at least 75% of the sequence of the protective peptide, preferably at least 80%, more preferably at least 85%, still more preferably at least 90%, even more preferably at least 95% and most preferably at least 97%, 98% or 99%;
   b) is derived from the protective peptide by at least one amino acid substitution and/or deletion, wherein the functionally active variant has a sequence identity to the protective peptide or to the functionally active fragment as defined in a) of at least 75%, preferably at least 80%, more preferably at least 85%, still more preferably at least 90%, even more preferably at least 95% and most preferably at least 97%, 98% or 99%; and/or
   c) consists of the protective pcptidc or a functionally active variant thereof and additionally at least one amino acid heterologous to the protective peptide.
3. A protective peptide consisting of the amino acid sequence of the SEQ ID NO:2, or a functionally active variant of the protective peptide.
4. The functionally active variant of the protective peptide of 3, wherein the functionally active variant
   a) is a functionally active fragment of the protective peptide, the functionally active fragment comprising at least 75% of the sequence of the protective peptide, preferably at least 80%, more preferably at least 85%, still more preferably at least 90%, even more preferably at least 95% and most preferably at least 97%, 98% or 99%;
   b) is derived from the protective peptide by at least one amino acid substitution, addition and/or deletion, wherein the functionally active variant has a sequence identity to the protective peptide or to the functionally active fragment as defined in a) of at least 75%, preferably at least 80%, more preferably at least 85%, still more preferably at least 90%, even more preferably at least 95% and most preferably at least 97%, 98% or 99%; and/or
   c) consists of the protective peptide or a functionally active variant thereof and additionally at least one amino acid heterologous to the protective peptide.
5. A composition comprising at least two proteins selected from the group consisting of
   i) a protective protein comprising or consisting of the protective peptide or functionally active variant thereof of 1 or 2;
   ii) a protective protein comprising or consisting of the protective peptide or functionally active variant thereof of 3 or 4; and
   iii) a supportive protein comprising or consisting of the supportive peptide of the SEQ ID NO:3 or a functionally active variant of the supportive peptide,
   wherein the at least two proteins are selected from at least two of the subgroups i), ii) and iii).
6. The composition of 5, wherein two or more proteins of the at least two proteins arc combined into one fusion protein, preferably a fusion protein comprising or consisting of the amino acid sequence of SEQ ID NO:4, SEQ ID NO:5, SEQ ID NO:6 or SEQ ID NO:7.
7. The composition of 5 or 6 comprising
   - at least one protein as defined in i) and at least one protein as defined in ii); or
   - at least one protein as defined in i) and at least one protein as defined in iii); or
   - at least one protein as defined in ii) and at least one protein as defined in iii); or
   - at least one protein as defined in i) and at least one protein as defined in ii) and at least one protein as defined in iii).
8. The composition of any of. 5 to 7 comprising
   - at least one protein comprising or consisting of the amino acid sequence of SEQ ID NO:1 and at least one protein comprising or consisting of the amino acid sequence of SEQ ID NO:2; or
   - at least one protein comprising or consisting of the amino acid sequence of SEQ lD NO:1 and at least one protein comprising or consisting of the amino acid sequence of SEQ ID NO:3; or
   - at least one protein comprising or consisting of the amino acid sequence of (SEQ ID NO:2 and at least one protein comprising or consisting of the amino acid sequence of SEQ ID NO:3; or
   - at least one protein comprising or consisting of the amino acid sequence of SEQ ID NO:1 and at least one protein comprising or consisting of the amino acid sequence of SEQ ID NO:2 and at least one protein comprising or consisting of the amino acid sequence of SEQ ID NO:3.
9. The composition of any of 5 to 8, wherein the functionally active variant of the supportive peptide
   a) is a functionally active fragment of the supportive peptide, the functionally active fragment comprising at least 60% of the sequence of the supportive peptide, preferably at least 70%, more preferably at least 80%, still more preferably at least 90%, even more preferably at least 95% and most preferably at least 97%, 98% or 99%;
   b) is derived from the supportive peptide by at least one amino acid substitution, addition and/or deletion and has a sequence identity to the supportive pcptidc or to the functionally active fragment as defined in a) of at least 60%, preferably at least 70%, more preferably at least 80%, still more preferably at least 90%, even more preferably at least 95% and most preferably at least 97%, 98% or 99%; and/or
   c) consists of the supportive peptide or a functionally active variant thereof and at least one amino acid heterologous to the supportive peptide.
10. One or more nucleic acid(s) encoding any of the protective peptides or functionally active variants thereof according to any of 1 to 4 or the at least two proteins comprised in the composition according to any of 5 to 9.
11. The one or more nucleic acid(s) of 10, comprising or consisting of at least one nucleic acid sequence selected from the group consisting of SEQ ID NO:8, SEQ ID NO:9, SEQ ID NO:10, SEQ ID NO:11, SEQ ID NO:12, SEQ ID NO:13 and SEQ ID NO:14.
12. The one or more nucleic acid(s) of any of 9 to 11, wherein the nucleic acid(s) is/are located in a vector or a cell other than *S. pneumoniae.*
13. A pharmaceutical composition, especially a vaccine, comprising at least one protective peptide or functionally active variant thereof according to any of claims 1 to 4 or the composition according to any of 5 to 9, and optionally a pharmaceutically acceptable carrier or cxcipicnt.
14. A pharmaceutical composition comprising
   (i) the one or more nucleic acid(s) according to any of 10 to 12 or one or more nucleic acid(s) complementary thereto, and
   (ii) optionally a pharmaceutically acceptable carrier or excipient.
15. A method for producing an antibody, characterized by the following steps:
   (a) administering an effective amount of at least one protective peptide or functionally active variant thereof according to any of 1 to 4 and/or a composition according to any of 5 to 9 to an animal; and
   (b) isolating the antibody produced by the animal in response to the administration of step (a) from the animal.
16. A method for producing an antibody, characterized by the following steps:
   (a) contacting a B cell with an effective amount of at least one protective peptide or functionally active variant thereof according to any of claims 1 to 4 and/or a composition according to any of 5 to 9;
   (b) fusing the B cell of step (a) with a myeloma cell to obtain a hybridoma cell; and
   (c) isolating the antibody produced by the cultivated hybridoma cell.
17. The method of 15 or 16, wherein the isolated antibody is additionally purified.
18. Use of the protective peptide according to any of claims 1 to 4 and/or a composition according to any of 5 to 9 and/or one ore more the nucleic acid(s) according to any of 10 to 12 for the manufacture of a medicament for the immunization or treatment of a subject, preferably against *S. pneumoniae,* more preferably against pneumonia, bacteremia, otitis media, meningitis, sinusitis, peritonitis and/or arthritis caused by *S. pneumoniae.*
19. A method of diagnosing a *S. pneumoniae* infection comprising the steps of:
   (a) contacting a sample obtained from a subject with a protective peptide according to any of 1 to 4 and/or a composition according to any of 5 to 9; and
   (b) detecting the presence of an antibody against the protective peptide, the functionally active variant and/or the composition in the sample,
   wherein the presence of the antibody is indicative for the *S. pneumoniae* infection.
20. A method for diagnosing an infection with *S. pneumoniae* comprising the steps of:
   a) contacting a sample obtained from a subject with the with a primer and/or a probe specific for the one or more nucleic acid(s) of any of 10 to 12; and
   b) detecting the presence of one or more nucleic acid(s) of any of 10 to 12 in the sample,
   wherein the presence of the one or more nucleic acid(s) is indicative for the *S. pneumoniae* infection.
21. A method for identifying a ligand capable of binding to a protective peptide or variant thereof according to any of 1 to 4 and/or a composition according to any of 5 to 9 comprising:
   (a) providing a test system comprising the peptide and/or composition,
   (b) contacting the test system with a test compound, and
   (c) detecting a signal generated in response to the binding of the test compound to the peptide and/or composition.
22. Use of a protective peptide or variant thereof according to any of I to 4 and/or a composition according to any of 5 to 9 for the isolation and/or purification and/or identification of an interaction partner of the peptide.

## Claims

1. A protective peptide consisting of the amino acid sequence of the Seq ID No 2, or a functionally active variant of the protective peptide.

2. The functionally active variant of the protective peptide of claim 1, wherein the functionally active variant
a) is a functionally active fragment of the protective peptide, the functionally active fragment comprising at least 75% of the sequence of the protective peptide, preferably at least 80%, more preferably at least 85%, still more preferably at least 90%, even more preferably at least 95% and most preferably at least 97%, 98% or 99%;
b) is derived from the protective peptide by at least one amino acid substitution and/or deletion, wherein the functionally active variant has a sequence identity to the protective peptide or to the functionally active fragment as defined in a) of at least 75%, preferably at least 80%, more preferably at least 85%, still more preferably at least 90%, even more preferably at least 95% and most preferably at least 97%, 98% or 99%; and/or
c) consists of the protective peptide or a functionally active variant thereof and additionally at least one amino acid heterologous to the protective peptide.

3. A composition comprising at least two proteins selected from the group consisting of
i) a protective protein comprising or consisting of the protective peptide or functionally active variant thereof of claim 1 or 2; and
ii) a protective protein comprising or consisting of the amino acid sequence of the Seq ID No 1, or a functionally active variant of the protective peptide, wherein the functional active variant is preferably as defined in claim 2; and/or
iii) a supportive protein comprising or consisting of the supportive peptide of the SEQ ID NO:3 or a functionally active variant of the supportive peptide,
wherein the at least two proteins are selected from at least two of the subgroups i), ii) and iii).

4. The composition of claim 3,
a) wherein two or more proteins of the at least two proteins are combined into one fusion protein, preferably a fusion protein comprising or consisting of the amino acid sequence of SEQ ID NO:4, SEQ ID NO:5, SEQ ID NO:6 or SEQ ID NO:7;
b) wherein the composition comprises
- at least one protein as defined in i) and at least one protein as defined in ii); or
- at least one protein as defined in i) and at least one protein as defined in iii); or
- at least one protein as defined in i) and at least one protein as defined in ii) and at least one protein as defined in iii);
c) wherein the composition comprises
- at least one protein comprising or consisting of the amino acid sequence of Seq ID No 2 and at least one protein comprising or consisting of the amino acid sequence of SEQ ID NO:3; or
- at least one protein comprising or consisting of the amino acid sequence of Seq ID No 2 and at least one protein comprising or consisting of the amino acid sequence of SEQ ID NO:1; or
- at least one protein comprising or consisting of the amino acid sequence of Seq ID No 2 and at least one protein comprising or consisting of the amino acid sequence of 1 and at least one protein comprising or consisting of the amino acid sequence of SEQ ID NO:3; and/or
d) wherein the functionally active variant of the supportive peptide
i) is a functionally active fragment of the supportive peptide, the functionally active fragment comprising at least 60% of the sequence of the supportive peptide, preferably at least 70%, more preferably at least 80%, still more preferably at least 90%, even more preferably at least 95% and most preferably at least 97%, 98% or 99%;
ii) is derived from the supportive peptide by at least one amino acid substitution, addition and/or deletion and has a sequence identity to the supportive peptide or to the functionally active fragment as defined in i) of at least 60%, preferably at least 70%, more preferably at least 80%, still more preferably at least 90%, even more preferably at least 95% and most preferably at least 97%, 98% or 99%; and/or
iii)consists of the supportive peptide or a functionally active variant thereof and at least one amino acid heterologous to the supportive peptide.

5. One or more nucleic acid(s) encoding any of the protective peptides or functionally active variants thereof according to any of claims 1 to 2 or the at least two proteins comprised in the composition according to claim 3 or 4.

6. The one or more nucleic acid(s) of claim 5, comprising or consisting of at least one nucleic acid sequence of Seq ID No 9.

7. The one or more nucleic acid(s) of any of claims 5 to 6, wherein the nucleic acid(s) is/are located in a vector or a cell other than *S. pneumoniae.*

8. A pharmaceutical composition, especially a vaccine, comprising at least one protective peptide or functionally active variant thereof according to any of claims 1 to 2 or the composition according to any of claims 3 or 4, and optionally a pharmaceutically acceptable carrier or excipient.

9. A pharmaceutical composition comprising
(i) the one or more nucleic acid(s) according to any of claims 5 to 7 or one or more nucleic acid(s) complementary thereto, and
(ii) optionally a pharmaceutically acceptable carrier or excipient.

10. The pharmaceutical composition of claim 8 or 9 for use as a medicament.

11. The pharmaceutical composition of claim 8 or 9 for use in a method of immunizing or treating a subject, preferably against *S. pneumoniae,* more preferably against pneumonia, bacteremia, otitis media, meningitis, sinusitis, peritonitis and/or arthritis caused by *S. pneumoniae.*

12. A method for producing an antibody, **characterized by** the following steps:
(a) administering an effective amount of at least one protective peptide or functionally active variant thereof according to any of claims 1 to 2 and/or a composition according to any of claims 3 or 4 to an animal; and
(b) isolating the antibody produced by the animal in response to the administration of step (a) from the animal.

13. A method for producing an antibody, **characterized by** the following steps:
(a) contacting a B cell with an effective amount of at least one protective peptide or functionally active variant thereof according to any of claims 1 to 2 and/or a composition according to any of claims 3 or 4;
(b) fusing the B cell of step (a) with a myeloma cell to obtain a hybridoma cell;
(c) isolating the antibody produced by the cultivated hybridoma cell; and
(d) optionally purifying the isolated antibody of step c).

14. Use of the protective peptide according to any of claims 1 to 2 and/or a composition according to any of claims 3 or 4 and/or one ore more the nucleic acid(s) according to any of claims 5 to 7 for the manufacture of a medicament for the immunization or treatment of a subject, preferably against *S. pneumoniae,* more preferably against pneumonia, bacteremia, otitis media, meningitis, sinusitis, peritonitis and/or arthritis caused by *S. pneumoniae.*
